(19) Europäisches Patentamt
European Patent Office
Office européen des brevets

(11) EP 3 770 180 A1

(12) **EUROPEAN PATENT APPLICATION**
published in accordance with Art. 153(4) EPC

(43) Date of publication:
**27.01.2021 Bulletin 2021/04**

(21) Application number: **19772452.9**

(22) Date of filing: **20.03.2019**

(51) Int Cl.:
***C08B 37/04** (2006.01)*
***A61K 9/08** (2006.01)*
***A61K 31/196** (2006.01)*
***A61K 45/00** (2006.01)*
***A61K 47/36** (2006.01)*
***A61K 47/54** (2017.01)*
***A61K 47/61** (2017.01)*
***A61P 19/02** (2006.01)*
***A61P 29/00** (2006.01)*

(86) International application number:
**PCT/JP2019/011715**

(87) International publication number:
**WO 2019/182015 (26.09.2019 Gazette 2019/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.03.2018 JP 2018053767**

(71) Applicant: **Mochida Pharmaceutical Co., Ltd.
Tokyo 160-8515 (JP)**

(72) Inventors:
- **FURUSAKO, Shoji
Tokyo 160-8515 (JP)**
- **SAKURADA, Isao
Tokyo 160-8515 (JP)**

(74) Representative: **Hoffmann Eitle
Patent- und Rechtsanwälte PartmbB
Arabellastraße 30
81925 München (DE)**

# (54) ALGINIC ACID DERIVATIVE BONDED TO NONSTEROIDAL ANTI-INFLAMMATORY COMPOUND

(57) Provided is water-soluble compound that can be used in a sustained-release preparation and is capable of stably releasing a fixed amount of an active ingredient in vivo by using the novel potential base material option of alginic acid as the base material. The present invention relates to an alginic acid derivative having a structure which is obtained by covalently bonding a nonsteroidal anti-inflammatory compound and alginic acid or a salt thereof via a linker, and preferably relates to an alginic acid derivative represented by formula (1) (in the formula: (A) represents one residue derived from alginic acid or a salt thereof and having the C(=O)- group from either L-guluronic acid or D-mannuronic acid, the monosaccharides that constitute alginic acid; (D) represents one residue from a nonsteroidal anti-inflammatory compound; and -L- represents a linker having a functional group which is capable of bonding to (A) by means of an amide bond and having a functional group which is capable of bonding to (D) by means of an ester bond).

(D)-L-(A)     (1)

EP 3 770 180 A1

**Description**

Technical Field

The present invention relates to a water-soluble alginic acid derivative in which alginic acid and a nonsteroidal anti-inflammatory compound are covalently bonded through a linker, and a sustained-release pharmaceutical composition containing the same.

Background Art

Alginic acid is a polymeric polysaccharide extracted from brown algae composed of β-D-mannuronic acid and α-L-guluronic acid, which is non-toxic, is not easily degraded due to the lack of specific degradative enzymes *in vivo,* is biocompatible, and is non-immunogenic. Moreover, it has the property of forming a gel by being cross-linked with a divalent metal ion such as calcium. Utilizing such a property of alginic acid, it is used for industrial use, food use, and further as a pharmaceutical additive. In recent years, wound coating applications (Japanese Patent Application Publication No. 2007-75425 (Patent Literature 1)), cartilage disease treatment applications (International Publication No. WO2008/102855 (Patent Literature 2)), rheumatoid arthritis treatment applications (International Publication No. WO2009/54181 (Patent Literature 3)), and intervertebral disc treatment applications (International Publication No. WO2017/163603 (Patent Literature 4)) have been proposed as the main agents of medicines.

On the other hand, nonsteroidal anti-inflammatory drugs (hereinafter, also referred to as NSAIDs) are widely used as a suppressive agent and a palliative agent for pain caused by arthropathy. Generally, when NSAIDs are used as suppressive agents and palliative agents for these pains, they are used as an oral dosage form or a transdermal absorption type formulation. However, in oral dosage forms containing NSAIDs, it may be necessary to take a large amount in order to allow an effective amount of NSAIDs to reach the diseased site, resulting in a problem that more side effects than expected may be caused on the digestive system and the like. In addition, transdermal absorption type dosage forms have problems that the effects may not be stable because the amount of NSAIDs absorbed from the start of contact to the end of contact with the diseased site (joint) is not constant, and also that side effects may be caused such as contact dermatitis more than expected in the case of using a transdermal absorption type preparation containing a high concentration of NSAIDs.

Therefore, in light of the above problems, for example, International Publication No. WO2005/66214 (Patent Literature 5) or BMC Musculoskelet Disord. 2018; 19: 157. (Non-Patent Literature 3) discloses the providing of a drug which can greatly contribute to the alleviation and suppression of pain associated with arthropathy and to the fundamental treatment of arthropathy by preparing a novel derivative in which NSAIDs and antirheumatic drugs (DMARDs) are chemically introduced into sodium hyaluronate which is a therapeutic agent for arthropathy, and injecting this into the diseased site, and a drug having sustained effects by controlling the release of NSAIDs and DMARDs. In addition, International Publication No. WO2015/5458 (Patent Literature 6) describes the providing of a glycosaminoglycan derivative and a method of producing the same which can control the drug release rate without depending largely on the structure of the drug, and which is introduced with the drug to be released at an appropriate rate according to the disease to which it is applied. In addition, International Publication No. WO2007/4675 (Patent Literature 7) discloses a hyaluronic acid derivative into which a drug such as NSAIDs or DMARDs and a photoreactive group are introduced, and a photocrosslinked hyaluronic acid derivative gel, and describes the providing of a formulation having enhanced sustained release of a drug. In addition, Journal of Young Pharmacists (2009), 1(4), 301-304 (Non-Patent Literature 1) and Pharmaceutica Acta Helvetiae (1997), 72(3), 159-164 (Non-Patent Literature 2) disclose beads that is a cross-linked mixture of alginic acid and diclofenac with calcium ions, and observed to exhibit sustained release of diclofenac at neutral for several hours. In addition, although it is not a disclosure of NSAIDs or DMARDs, Japanese Patent Application Publication No. Hei 08-24325 (Patent Literature 8) describes the providing of a medical purpose polymer gel capable of releasing a therapeutically effective amount of a drug only at a lesion site where an enzyme is produced, and the providing of a water-swellable polymer gel which is highly transparent, excellent in biocompatibility, heat resistance, and stability, and useful as a constituent component of various medical purpose materials such as wound coating materials, biological tissue adhesives, and adhesion prevention materials.

In addition, Japanese Translation of PCT International Application Publication No. Hei 08-502053 (Patent Literature 9) discloses an alginate-bioactive agent combination connected through an acid-labile biodegradable spacer bond. It is described that this combination is effective for delivering the bioactive agent to a target present in a low pH environment, at the target surface, or within the target, and alginates covalently bonded to bioactive substances (including drugs and prodrugs) can be used to control the rate of release of the substances.

Citation List

Patent Literatures

Patent Literature 1: Japanese Patent Application Publication No. 2007-75425
Patent Literature 2: International Publication No. WO2008/102855
Patent Literature 3: International Publication No. WO2009/54181
Patent Literature 4: International Publication No. WO2017/163603
Patent Literature 5: International Publication No. WO2005/66214
Patent Literature 6: International Publication No. WO2015/5458
Patent Literature 7: International Publication No. WO2007/4675
Patent Literature 8: Japanese Patent Application Publication No. Hei 08-24325
Patent Literature 9: Japanese Translation of PCT International Application Publication No. Hei 08-502053

Non-Patent Literatures

Non-Patent Literature 1: Journal of Young Pharmacists (2009), 1(4), 301-304
Non-Patent Literature 2: Pharmaceutica Acta Helvetiae (1997), 72(3), 159-164
Non-Patent Literature 3: BMC Musculoskelet Disord. 2018; 19: 157.

Summary of Invention

Problems to be solved by the invention

However, conventional sustained release formulations containing NSAIDs have not reached wide practical application. For example, a sustained release formulation containing NSAIDs using hyaluronic acid as a base material has been proposed, but hyaluronic acid is degraded by an enzyme (hyaluronidase) existing *in vivo,* which may affect the release of NSAIDs. In addition, a sustained release formulation containing NSAIDs using a base material derived from plants and brown algae, which can be used as a new option for a base material, has not yet achieved sufficient sustained release itself.

In view of such problems, an object of the present invention is to provide a water-soluble compound capable of use in a sustained release formulation, which can stably release a certain active ingredient *in vivo* by using alginic acid as a base material that can be a new option for a base material.

Means for solution of the problems

The present inventors have made diligent studies to solve the above problems, and have found as a result that an alginic acid derivative having a structure in which alginic acid or a salt thereof and a nonsteroidal anti-inflammatory compound are covalently bonded with a specific linker is water-soluble, and by using this as a sustained release formulation, it is possible to deliver the nonsteroidal anti-inflammatory compound to the diseased site in a stable manner for an unexpectedly long period of time. Thus, the present invention has been completed.

That is, the present invention is configured as follows:

Another aspect of the present invention may be as in [1] to [14] below.

[1] A water-soluble alginic acid derivative, comprising a structure in which alginic acid or a salt thereof and a nonsteroidal anti-inflammatory compound are covalently bonded through a linker.
[1a] The water-soluble alginic acid derivative according to [1] described above, wherein the linker is a divalent linker.
[2] The water-soluble alginic acid derivative according to [1] described above, which comprises a structure represented by the following formula (1):

$$(A)\text{-}L\text{-}(D) \qquad (1)$$

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound; and
L is a linker having a functional group capable of binding to (A) via an amide bond and having a functional group capable of binding to (D) via an ester bond.

[3] The water-soluble alginic acid derivative according to [1] described above, which comprises a structure represented by the following formula (2):

$$(A)\text{-}NH\text{-}(CH_2)_{n1}\text{-}[X^1]_{n2}\text{-}(CR^1R^2)_{n3}\text{-}[Y]_{n4}\text{-}(CH_2)_{n5}\text{-}(CR^3R^4)_{n6}\text{-}[X^2]_{n7}\text{-}(CH_2)_{n8}\text{-}[Z]\text{-}(D) \quad (2)$$

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound;
$X^1$ and $X^2$ represent hetero atoms;
$R^1$, $R^2$, $R^3$, and $R^4$ each independently represent hydrogen, a halogen atom, a $C_{1-10}$ alkyl group, a $C_{1-10}$ alkoxy group, or a $C_{1-10}$ alkoxycarbonyl group; or $R^1$ and $R^2$ or $R^3$ and $R^4$ together form =O;
Y represents a cycloalkane ring, an aromatic ring, or a heterocycle, wherein aforementioned the cycloalkane ring, the aromatic ring, or the heterocycle may be substituted with halogen atom(s) or $C_{1-10}$ alkyl group(s);
Z represents O or C(=O) for forming an ester bond with (D); and
n1 represents any integer of 0 to 10; and n2 to n8 independently represent any integer of 0 to 3, but not all of n1 to n8 are 0.

[3a] The water-soluble alginic acid derivative according to [1] described above, which is represented by the following formula (2a):

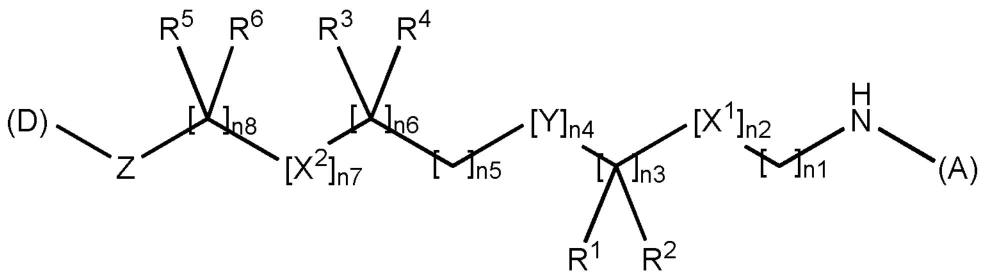
(2a)

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound;
$X^1$ and $X^2$ are hetero atoms;
$R^1$, $R^2$, $R^3$, $R^4$, $R^5$, and $R^6$ are each independently a group selected from a hydrogen atom, a halogen atom, a $C_{1-6}$ alkyl group, a $C_{1-6}$ alkoxy group, or a $C_{1-6}$ alkoxycarbonyl group ($R^1$ and $R^2$, $R^3$ and $R^4$, or $R^5$ and $R^6$ can together form an oxo group (=O));
Y is a $C_{3-8}$ cycloalkyl ring, a $C_{6-10}$ aryl ring, or a heterocycle, wherein aforementioned the $C_{3-8}$ cycloalkyl ring, the $C_{6-10}$ aryl ring, or the heterocycle may be substituted with 1 to 3 halogen atoms or $C_{1-6}$ alkyl groups;
Z is an oxygen atom or a carbonyl group;
n1 or n8 is any integer of 0 to 10;
n3, n5, or n6 are independently any integer of 0, 1, 2, or 3;
n2, n4, or n7 are independently any integer of 0 or 1; and not all of n1 to n8 are 0.

[4] The water-soluble alginic acid derivative according to any one of [1] to [3] described above, wherein the nonsteroidal anti-inflammatory compound has a carboxyl group, and the carboxyl group is bonded to a linker.
[4a] The alginic acid derivative according to any one of [1a], [2], and [3a] described above, wherein the nonsteroidal anti-inflammatory compound has a carboxyl group, and the carboxyl group is bonded to a linker, wherein the linker

is represented by the following formula (LKA-1) [excluding a left side of a broken line in the formula]:

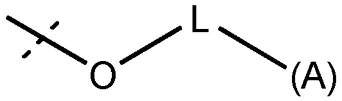

(LKA-1)

wherein the definitions of -L- and (A) are the same as those defined in [2a] described above; or represented by a formula (LKA-2) [excluding a left side of a broken line in the formula]:

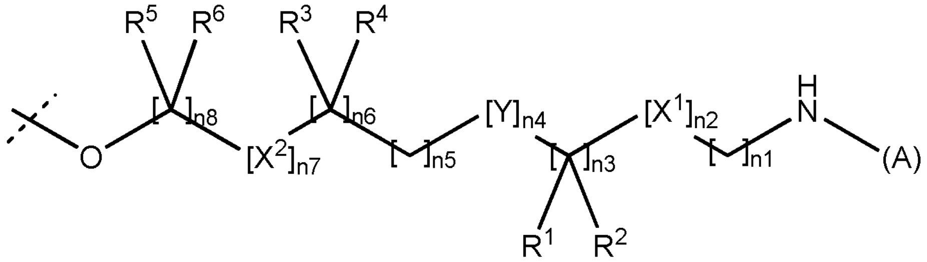

(LKA-2)

wherein the definitions of (A), $R^1$, $R^2$, $R^3$, $R^4$, $R^5$, $R^6$, $X^1$, $X^2$, Y, n1, n2, n3, n4, n5, n6, n7, and n8 are the same as those defined in [3] described above.

[5] The water-soluble alginic acid derivative according to any one of [1] to [4] described above, wherein the nonsteroidal anti-inflammatory compound is a salicylic acid-based, propionic acid-based, or acetic acid-based nonsteroidal anti-inflammatory drug (NSAID), and a carboxyl group of the NSAID is bonded to a linker.

[5a] The alginic acid derivative according to any one of [1a], [2], [3a], and [4a] described above, wherein the nonsteroidal anti-inflammatory compound is a salicylic acid-based, propionic acid-based, or phenylacetic acid-based nonsteroidal anti-inflammatory drug (NSAID), and a carboxyl group of the NSAID is bonded to the linker represented by formula (LKA-1) or formula (LKA-2) according to [4a] described above.

[6] The water-soluble alginic acid derivative according to [5] described above, wherein the nonsteroidal anti-inflammatory compound is an acetic acid-based nonsteroidal anti-inflammatory drug (NSAID).

[6a] The alginic acid derivative according to [5a] described above, wherein the nonsteroidal anti-inflammatory compound is a phenylacetic acid-based nonsteroidal anti-inflammatory drug (NSAID).

[6a-1] The alginic acid derivative according to [5a] described above, wherein the nonsteroidal anti-inflammatory compound is a propionic acid-based nonsteroidal anti-inflammatory drug (NSAID).

[7] The water-soluble alginic acid derivative according to [6] described above, wherein the nonsteroidal anti-inflammatory compound is diclofenac.

[7a] The water-soluble alginic acid derivative according to [6a] described above, wherein the nonsteroidal anti-inflammatory compound is diclofenac or felbinac.

[7a-1] The water-soluble alginic acid derivative according to [6a-1] described above, wherein the nonsteroidal anti-inflammatory compound is ketoprofen or naproxen.

[8] The water-soluble alginic acid derivative according to any one of [1] to [7] described above, wherein the introduction rate (mol%) of the nonsteroidal anti-inflammatory compound is at least 1.0 mol% or more.

[8a] The alginic acid derivative according to any one of [1a], [2], [3a], [4a], [5a], [6a], [6a-1], [7a], and [7a-1] described above, wherein the introduction rate (mol%) of the nonsteroidal anti-inflammatory compound is at least 1.0 mol% or more.

[9] An alginic acid derivative gel obtained by cross-linking the water-soluble alginic acid derivative according to any one of [1] to [8] described above.

[9a] An alginic acid derivative gel obtained by cross-linking the alginic acid derivative according to any one of [1a], [2], [3a], [4a], [5a], [6a], [6a-1], [7a], [7a-1], and [8a] described above.

[10] A sustained-release pharmaceutical composition, comprising the water-soluble alginic acid derivative according to any one of [1] to [8] described above or the alginic acid derivative gel according to [9] described above.

[10a] A sustained-release pharmaceutical composition, comprising the alginic acid derivative according to any one of [1a], [2], [3a], [4a], [5a], [6a], [6a-1], [7a], [7a-1], and [8a] or the alginic acid derivative gel according to [9a] described above.

[11] The sustained-release pharmaceutical composition according to [10] described above, as an arthritis therapeutic agent.

[11a] The sustained-release pharmaceutical composition according to [10a] described above, as an arthritis therapeutic agent.

[12] Use of the water-soluble alginic acid derivative according to any one of [1] to [8] described above or the alginic acid derivative gel according to [9] described above, for sustained release of a nonsteroidal anti-inflammatory compound.

[12a] Use of the alginic acid derivative according to any one of [1a], [2], [3a], [4a], [5a], [6a], [6a-1], [7a], [7a-1], and [8a] or the alginic acid derivative gel according to [9a] described above, for sustained release of a nonsteroidal anti-inflammatory compound.

[13] In the alginic acid derivative of the formula (2) of [3a] described above, a preferable one is selected from an alginic acid derivative listed below, wherein (A) is one residue derived from alginic acid or the salt thereof which has a C(=O)- group in a monosaccharide of either L-guluronic acid or D-mannuronic acid constituting alginic acid:

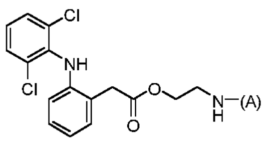
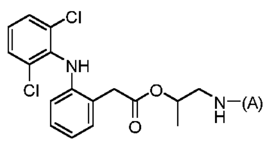
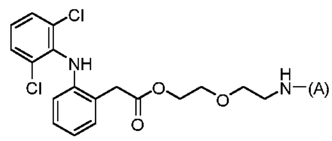
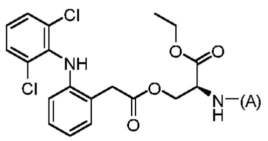
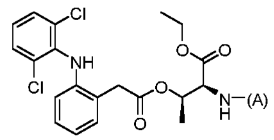
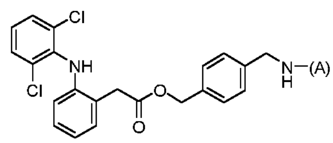
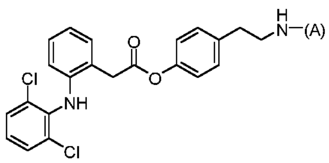
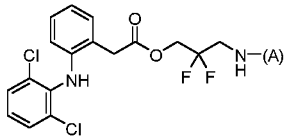
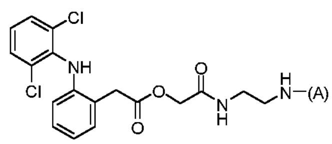
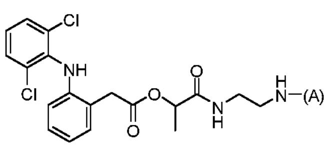
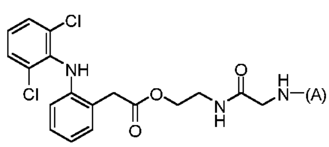
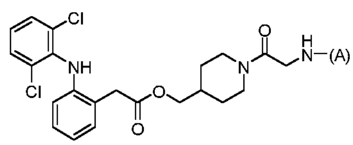
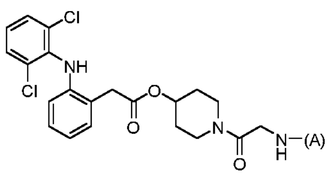
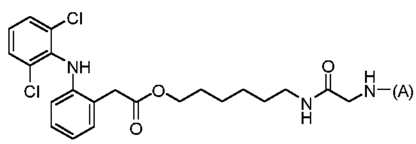
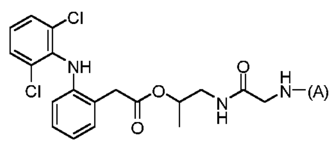
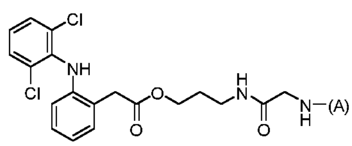
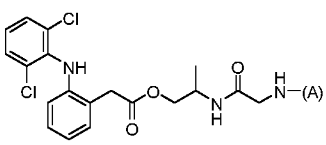
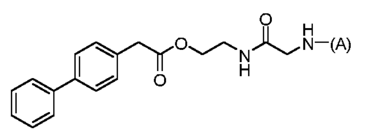
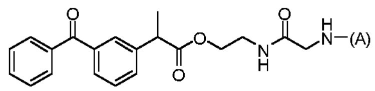
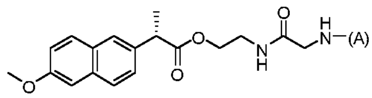

[14] An amino compound represented by the following formula (AM), a salt thereof, or a hydrate thereof:

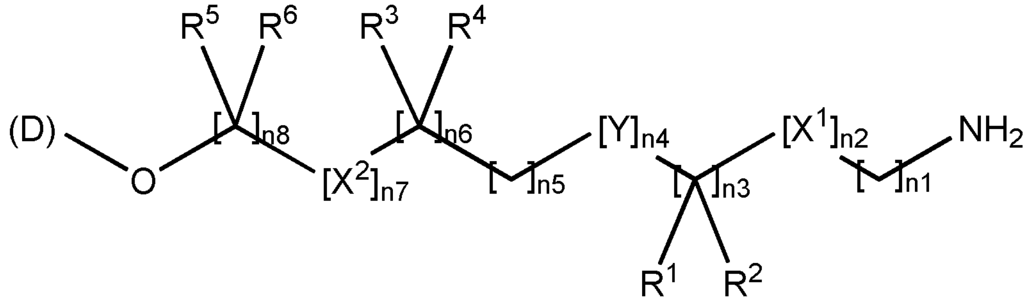

(AM)

wherein the definitions of (D), $X^1$, $X^2$, $R^1$, $R^2$, $R^3$, $R^4$, $R^5$, $R^6$, Y, Z, n1, n2, n3, n4, n5, n6, n7, and n8 are the same as those defined in [3a] described above. Advantageous Effects of Invention

The present invention makes it possible to provide a water-soluble compound capable of releasing a nonsteroidal anti-inflammatory compound at a stable rate and capable of use in a sustained release formulation. In addition, by gelling, it is possible to provide a water-soluble compound capable of further enhancing the sustained release of the compound.

Description of Embodiments

<Water-Soluble Alginic Acid Derivative>

The present invention relates to a water-soluble alginic acid derivative, comprising a structure in which alginic acid or a salt thereof and a nonsteroidal anti-inflammatory compound are covalently bonded through a linker. Preferably, the linker is covalently bonded to the carboxyl group of alginic acid or a salt thereof and the carboxyl group or hydroxyl group of the nonsteroidal anti-inflammatory compound. The binding mode is not particularly limited as long as the object of the present invention is achieved, but in the water-soluble alginic acid derivative, the bond between the alginic acid and the linker is preferably an amide bond, and the bond between the nonsteroidal anti-inflammatory compound and the linker is preferably an ester bond. The binding site to the linker in alginic acid or a salt thereof (functional group of alginic acid or a salt thereof) may be a hydroxyl group or a carboxyl group, but a carboxyl group capable of forming an amide bond is more preferable.

Therefore, as an aspect of the present invention, the water-soluble alginic acid derivative has a structure represented by the following formula (1):

(A)-L-(D) (1).

In the formula (1), (A) represents one residue derived from alginic acid or the salt thereof which has a C(=O)- group in a monosaccharide of either L-guluronic acid or D-mannuronic acid constituting alginic acid. In addition, in the formula (1), L is a linker having a functional group capable of binding to (A) via an amide bond and having a functional group capable of binding to (D) via an ester bond. In addition, in the formula (1), (D) represents one residue of the nonsteroidal anti-inflammatory compound, and the one residue of (D) may be a hydroxyl group or a carboxyl group, and is preferably a carboxyl group.

Also, as another aspect of the present invention, the water-soluble alginic acid derivative is an alginic acid derivative having a structure represented by the following formula (1):

(D)-L-(A) (1)

(in the formula (1), (A) represents one residue derived from alginic acid or the salt thereof which has a C(=O)- group of a monosaccharide of either L-guluronic acid or D-mannuronic acid constituting alginic acid;

(D) represents one residue of the nonsteroidal anti-inflammatory compound, and the one residue of (D) is a hydroxyl group or a carboxyl group, preferably a carboxyl group;

-L- is the following partial structural formula (LS-1) [excluding the outside of the broken line in the formula]:

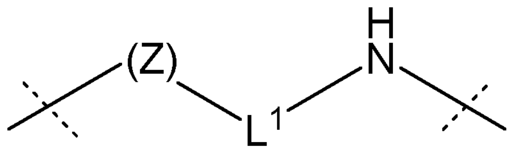

(LS-1)

(wherein $-L^1-$ is a linear group or a group in which a cyclic group is introduced into a part of a linear group, and the linear group is an alkylene group ($-(CH_2)_n-$, n = integer of 1 to 30) (each of the multiple (for example, 1 to 10, preferably 1 to 5) $-CH_2-$ may be substituted with group(s) selected from groups such as >C=O, -O-, -NH-, -S-, $-SO_2-$, $C_{3-8}$ cycloalkyl ring, $C_{6-10}$ aryl ring, and heterocycle (for example, selected from aromatic heterocycles such as pyridine ring, piperidine ring, and piperazine ring, non-aromatic heterocycles, and the like), and each of the multiple (for example, 1 to 10, preferably 1 to 5) hydrogen atoms of the $-CH_2-$ may be substituted with group(s) selected from groups such as oxo group (=O), $C_{1-6}$ alkyl group (for example, selected from methyl group, ethyl group, n-propyl group, iso-propyl group, and the like), $C_{1-6}$ alkoxy group (for example, selected from methoxy group, ethoxy group, propoxy group, and the like), $C_{1-6}$ alkoxycarbonyl group (for example, selected from methoxycarbonyl, ethoxycarbonyl, tert-butoxycarbonyl, and the like), $C_{7-16}$ aralkyl group (for example, selected from benzyl group, phenethyl group, and the like), halogen atom (for example, selected from fluorine atom, chlorine atom, bromine atom, iodine atom, and the like), and hydroxyl group (-OH)); and

Z is an oxygen atom or a carbonyl group, and preferably an oxygen atom)).

Furthermore, as an embodiment of the present invention, the water-soluble alginic acid derivative has a structure represented by the following formula (2):

$$(A)-NH-(CH_2)_{n1}-[X^1]_{n2}-(CR^1R^2)_{n3}-[Y]_{n4}-(CH_2)_{n5}-(CR^3R^4)_{n6}-[X^2]_{n7}-(CH_2)_{n8}-[Z]-(D) \quad (2).$$

In the formula (2), (A) represents one residue derived from alginic acid or the salt thereof which has a C(=O)- group in a monosaccharide of either L-guluronic acid or D-mannuronic acid constituting alginic acid, (D) represents one residue of the nonsteroidal anti-inflammatory compound, and wherein $X^1$ and $X^2$ represent hetero atoms, $R^1$, $R^2$, $R^3$, and $R^4$ each independently represent hydrogen, a halogen atom, a $C_{1-10}$ alkyl group, a $C_{1-10}$ alkoxy group, or a $C_{1-10}$ alkoxycarbonyl group, or $R^1$ and $R^2$ or $R^3$ and $R^4$ together form =O, Y represents a cycloalkane ring, an aromatic ring, or a heterocycle, wherein the cycloalkane ring, the aromatic ring, or the heterocycle may be substituted with a halogen atom or a $C_{1-10}$ alkyl group, Z represents O or C(=O) for forming an ester bond with (D), and n1 represents any integer of 0 to 10, and n2 to n8 independently represent any integer of 0 to 3, but not all of n1 to n8 are 0. Preferably, n2, n4, and n7 are independently 0 to 2, more preferably independently 0 or 1. In addition, n3, n5, n6, and n8 are preferably 1 to 12 in total and more preferably 2 to 10 in total.

In addition, as another aspect of the present invention, the water-soluble alginic acid derivative is one having a structure represented by the following formula (2a):

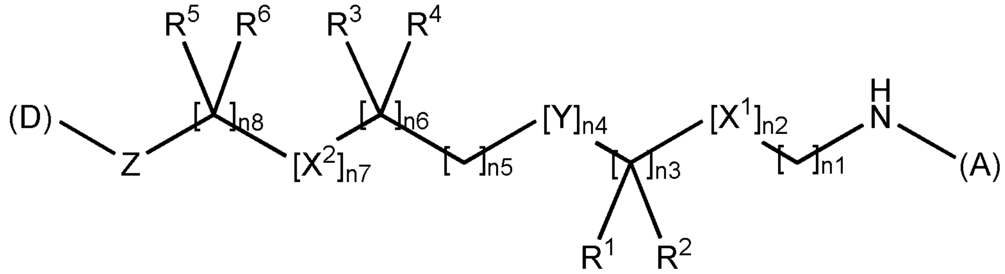

(2a)

(wherein, (A) represents one residue derived from alginic acid or the salt thereof which has a C(=O)- group in a monosaccharide of either L-guluronic acid or D-mannuronic acid constituting alginic acid;

(D) represents one residue of the nonsteroidal anti-inflammatory compound;

$X^1$ and $X^2$ are oxygen atoms or imino groups (NH);

$R^1$, $R^2$, $R^3$, $R^4$, $R^5$, and $R^6$ are each independently a group selected from a hydrogen atom, a halogen atom, a $C_{1-6}$ alkyl group, a $C_{1-6}$ alkoxy group, or a $C_{1-6}$ alkoxycarbonyl group ($R^1$ and $R^2$, $R^3$ and $R^4$, or $R^5$ and $R^6$ can together form an oxo group (=O)), and preferably a group selected from a hydrogen atom, a halogen atom, a $C_{1-3}$ alkyl group, a $C_{1-3}$ alkoxy group, and a $C_{1-3}$ alkoxycarbonyl group ($R^1$ and $R^2$, $R^3$ and $R^4$, or $R^5$ and $R^6$ can together form an oxo group (=O));

Y is a $C_{6-10}$ aryl ring or a heterocycle;
Z is an oxygen atom or a carbonyl group;
n1 is any integer of 0 to 5;
n8 is any integer of 0 to 10;
n3, n5, or n6 are independently any integer of 0, 1, 2, or 3;
n2, n4, or n7 are independently any integer of 0 or 1;
not all of n1 to n8 are 0; n3, n5, n6, and n8 are preferably 1 to 12 in total, and more preferably 2 to 10 in total).

Moreover, as another aspect of the present invention, the water-soluble alginic acid derivative is a water-soluble alginic acid derivative having a structure represented by the following formula (2a):

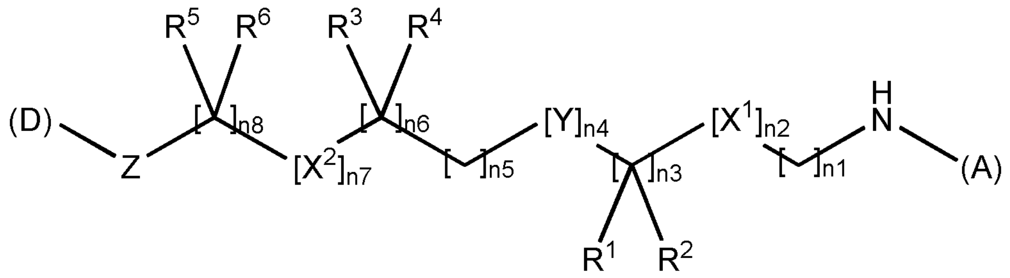

(2a)

(wherein

(A) represents one residue derived from alginic acid or the salt thereof which has a C(=O)- group in a monosaccharide of either L-guluronic acid or D-mannuronic acid constituting alginic acid;
(D) represents one residue of a nonsteroidal anti-inflammatory compound selected from diclofenac, ketoprofen, naproxen, and felbinac;
$X^1$ and $X^2$ are oxygen atoms or imino groups (NH);
$R^1$, $R^2$, $R^3$, $R^4$, $R^5$, and $R^6$ are each independently a group selected from a hydrogen atom, a fluorine atom, a methyl group, and an ethoxycarbonyl group (the $R^1$ and $R^2$, or $R^3$ and $R^4$ can together form an oxo group (=O));
Y is a benzene ring or a piperidine ring;
Z is an oxygen atom;
n1 is any integer of 0 to 2;
n8 is any integer of 0 to 6;
n3, n5, or n6 are independently any integer of 0 or 1;
n2, n4, or n7 are independently any integer of 0 or 1;
not all of n1 to n8 are 0; n3, n5, n6, and n8 are preferably 1 to 12 in total, and more preferably 2 to 10 in total).

In the formula (2), the preferable water-soluble alginic acid derivative has a structure represented by the following formulas (3) to (6):

$$(A)\text{-}NH\text{-}(CH_2)_{n1}\text{-}(CR^1R^2)_{n3}\text{-}(CH_2)_{n5}\text{-}[Z]\text{-}(D) \qquad (3)$$

(wherein

(A) represents one residue derived from alginic acid or the salt thereof which has a C(=O)- group in a monosaccharide of either L-guluronic acid or D-mannuronic acid constituting alginic acid,
(D) represents one residue of a nonsteroidal anti-inflammatory compound,
for $R^1$ and $R^2$, $R^1$ represents hydrogen or a halogen atom, $R^2$ represents hydrogen, a halogen atom, a methyl group, or an ethyl group, or $R^1$ and $R^2$ together represent =O.
Z represents O for forming an ester bond with (D), and
n1, n3, and n5 represent any integer of 1 to 4 in total).

$$(A)\text{-}NH\text{-}(CH_2)_{n1}\text{-}[X^1]\text{-}(CR^1R^2)_{n3}\text{-}(CR^3R^4)_{n6}\text{-}(CH_2)_{n8}\text{-}[Z]\text{-}(D) \qquad (4)$$

(wherein

(A) represents one residue derived from alginic acid or the salt thereof which has a C(=O)- group in a monosaccharide of either L-guluronic acid or D-mannuronic acid constituting alginic acid,
(D) represents one residue of a nonsteroidal anti-inflammatory compound,
$X^1$ represents O or NH,
for $R^1$ and $R^2$, $R^1$ represents hydrogen and $R^2$ represents hydrogen, a halogen atom, a methyl group, or an ethyl group, or $R^1$ and $R^2$ together represent =O.
when $X^1$ is O, $R^1$ is preferably hydrogen, and $R^2$ is preferably hydrogen, a methyl group, or an ethyl group,
when $X^1$ is NH, $R^1$ is preferably hydrogen, and $R^2$ is preferably hydrogen, a methyl group, or an ethyl group, or $R^1$ and $R^2$ together preferably represent =O.
for $R^3$ and $R^4$, $R^3$ represents hydrogen, and $R^4$ represents hydrogen, a halogen atom, a methyl group, or an ethyl group, or $R^3$ and $R^4$ together represent =O,
Z represents O for forming an ester bond with (D), and
n1 represents any integer of 1 to 3, and n3, n6, and n8 represent any integer of 1 to 3 in total).

$$\text{(A)-NH-(CH}_2)_{n1}\text{-[Y]-(CH}_2)_{n5}\text{-[Z]-(D)} \qquad (5)$$

(wherein

(A) represents one residue derived from alginic acid or the salt thereof which has a C(=O)- group in a monosaccharide of either L-guluronic acid or D-mannuronic acid constituting alginic acid,
(D) represents one residue of a nonsteroidal anti-inflammatory compound,
Y represents an aromatic ring,
Z represents O for forming an ester bond with (D), and
n1 and n5 represent any integer of 1 to 4 in total).

$$\text{(A)-NH-(CH}_2)_{n1}\text{-(CR}^1\text{R}^2)_{n3}\text{-(CH}_2)_{n5}\text{-(CR}^3\text{R}^4)_{n6}\text{-(CH}_2)_{n8}\text{-[Z]-(D)} \qquad (6)$$

(wherein

(A) represents one residue derived from alginic acid or the salt thereof which has a C(=O)- group in a monosaccharide of either L-guluronic acid or D-mannuronic acid constituting alginic acid,
(D) represents one residue of a nonsteroidal anti-inflammatory compound,
for $R^1$ and $R^2$, $R^1$ represents hydrogen, and $R^2$ represents hydrogen, a methoxy group, an ethoxy group, a methoxycarbonyl group, or an ethoxycarbonyl group, or $R^1$ and $R^2$ together represent =O.
for $R^3$ and $R^4$, $R^3$ represents hydrogen, and $R^4$ represents hydrogen, a methyl group, or an ethyl group, or $R^3$ and $R^4$ together represent =O,
Z represents O for forming an ester bond with (D), and
n1, n3, n5, n6, and n8 represent any integer of 1 to 4 in total).

In addition, in the formula (2a), a preferable water-soluble alginic acid derivative is one having a structure represented by the following formula (3a):

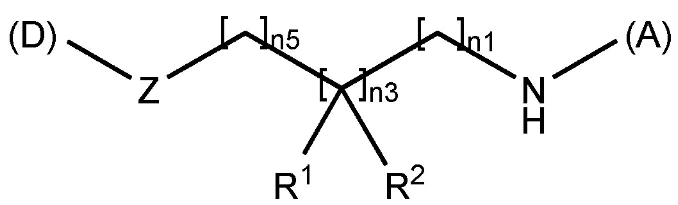

(3a)

(wherein

(A) represents one residue derived from alginic acid or the salt thereof which has a C(=O)- group in a monosaccharide of either L-guluronic acid or D-mannuronic acid constituting alginic acid;

(D) represents one residue of a nonsteroidal anti-inflammatory compound; preferably one residue of diclofenac;
$R^1$ and $R^2$ are each independently a group selected from a hydrogen atom, a halogen atom, a methyl group, and an ethoxycarbonyl group (aforementioned $R^1$ and $R^2$ can together form an oxo group (=O));
Z is an oxygen atom; and
n1, n3, and n5 represent any integer of 1 to 4 in total).

In addition, a specific example of the formula (3a) is a water-soluble alginic acid derivative selected from the following formulas:

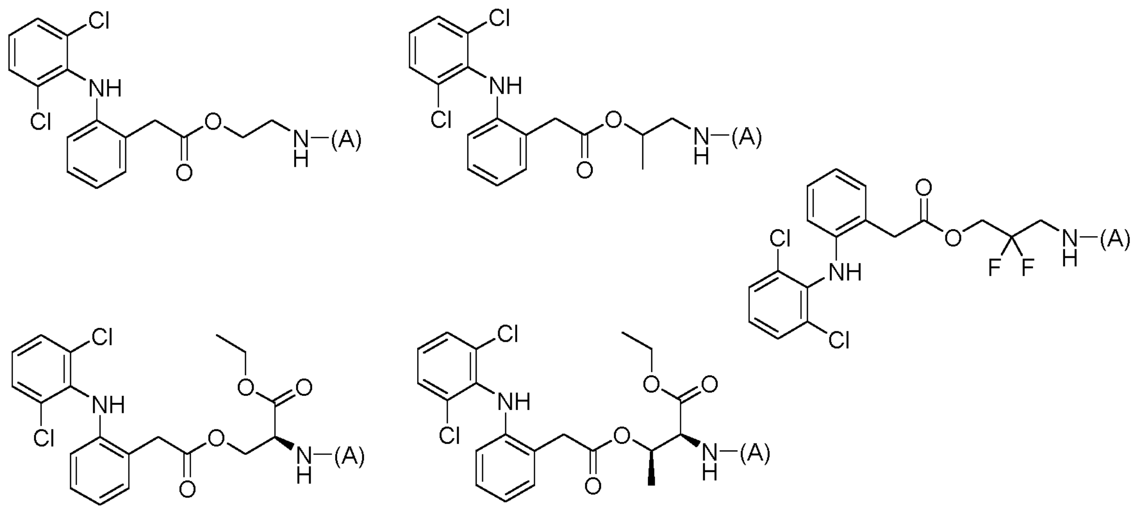

(in each formula, (A) represents one residue derived from alginic acid or the salt thereof which has a C(=O)- group in a monosaccharide of either L-guluronic acid or D-mannuronic acid constituting alginic acid).

In addition, in the formula (2a), a preferable water-soluble alginic acid derivative is one having a structure represented by the following formula (4a):

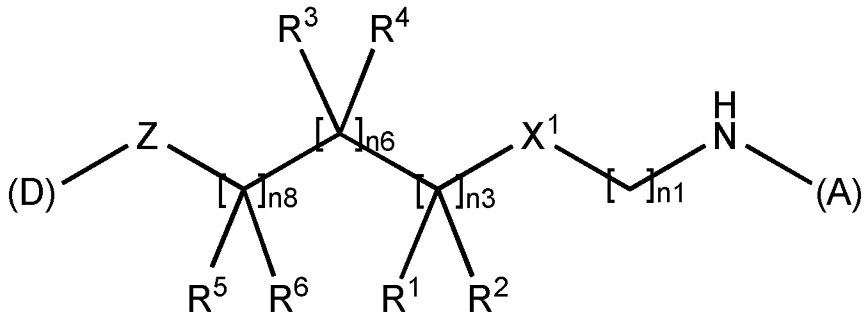 (4a)

(wherein

(A) represents one residue derived from alginic acid or the salt thereof which has a C(=O)- group in a monosaccharide of either L-guluronic acid or D-mannuronic acid constituting alginic acid;
(D) represents one residue of a nonsteroidal anti-inflammatory compound; preferably one residue of diclofenac;
$X^1$ is an oxygen atom or an imino group (NH);
$R^1$, $R^2$, $R^3$, $R^4$, $R^5$, and $R^6$ are each independently a group selected from a hydrogen atom, a halogen atom, a methyl group, an ethyl group, and an ethoxycarbonyl group (aforementioned $R^1$ and $R^2$, $R^3$ and $R^4$, or $R^5$ and $R^6$ can together form an oxo group (=O)) (when $X^1$ is O, $R^1$ is preferably a hydrogen atom, $R^2$ is preferably a hydrogen atom, a methyl group, or an ethyl group, $R^3$ is preferably a hydrogen atom, $R^4$ is preferably a hydrogen atom, a methyl group, or an ethyl group, $R^5$ is preferably a hydrogen atom, and $R^6$ is preferably a hydrogen atom, a methyl group, or an ethyl group; more preferably, $R^1$, $R^2$, $R^3$, $R^4$, $R^5$, and $R^6$ are hydrogen atoms; when $X^1$ is an imino group (NH), $R^1$ is preferably hydrogen, $R^2$ is preferably hydrogen, a methyl group, or an ethyl group, or $R^1$ and $R^2$ preferably together form =O, $R^3$ is preferably hydrogen, $R^4$ is preferably a hydrogen atom, a methyl group, or an

ethyl group, $R^5$ is preferably hydrogen, and $R^6$ is preferably a hydrogen atom, a methyl group, or an ethyl group; more preferably, $R^1$ and $R^2$ together are =O, $R^3$ is a hydrogen atom, $R^4$ is a hydrogen atom or a methyl group, $R^5$ is a hydrogen atom, and $R^6$ is a hydrogen atom or a methyl group);
Z represents an oxygen atom, and
n1 represents any integer of 1 to 3, and n3, n6, and n8 represent any integer of 1 to 3 in total).

In addition, a specific example of the formula (4a) is an alginic acid derivative selected from the following formulas:

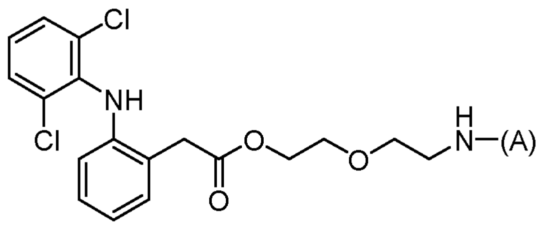

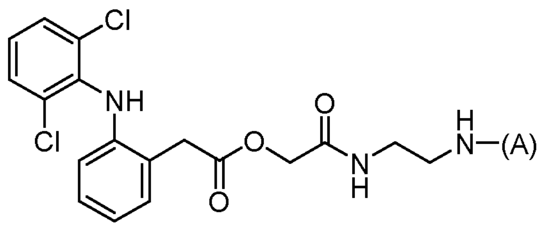

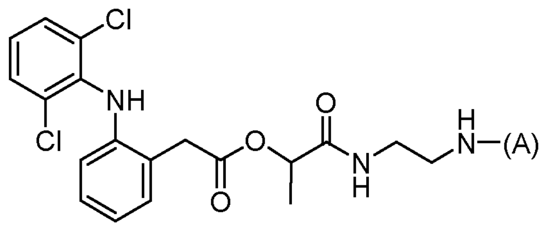

(in each formula, (A) represents one residue derived from alginic acid or the salt thereof which has a C(=O)- group in a monosaccharide of either L-guluronic acid or D-mannuronic acid constituting alginic acid).

In addition, in the formula (2a), a preferable water-soluble alginic acid derivative is one having a structure represented by the following formula (5a):

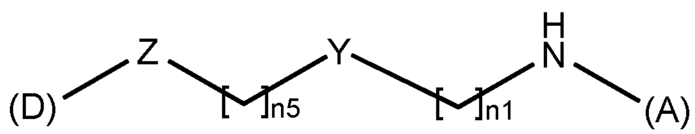

(5a)

(wherein

(A) represents one residue derived from alginic acid or the salt thereof which has a C(=O)- group in a monosaccharide of either L-guluronic acid or D-mannuronic acid constituting alginic acid;
(D) represents one residue of a nonsteroidal anti-inflammatory compound; preferably one residue of diclofenac;
Y is a $C_{6-10}$ aryl ring, and preferably a benzene ring;
Z is an oxygen atom; and
n1 and n5 represent any integer of 1 to 4 in total.

In addition, a specific example of the formula (5a) is a water-soluble alginic acid derivative selected from the following formulas:

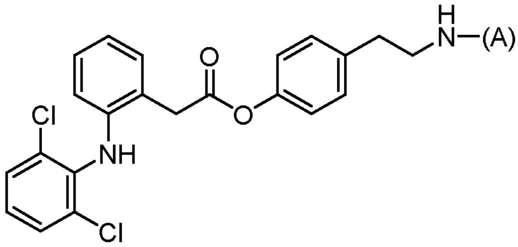

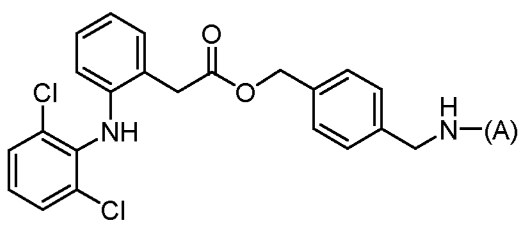

(in each formula, (A) represents one residue derived from alginic acid or the salt thereof which has a C(=O)- group in a monosaccharide of either L-guluronic acid or D-mannuronic acid constituting alginic acid).

In addition, in the formula (2a), a preferable water-soluble alginic acid derivative is one having a structure represented by the following formula (6a):

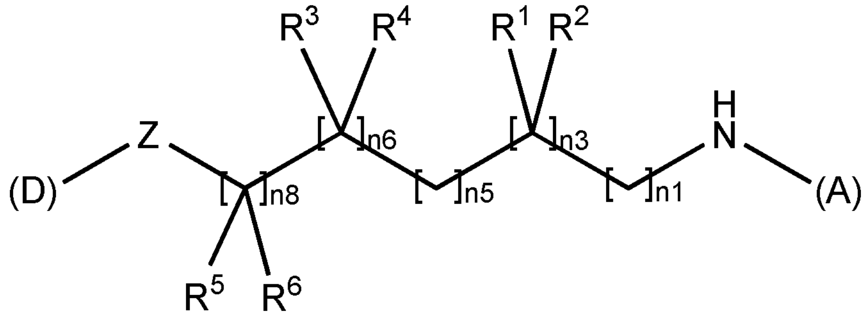

(6a)

(wherein,

(A) represents one residue derived from alginic acid or the salt thereof which has a C(=O)- group in a monosaccharide of either L-guluronic acid or D-mannuronic acid constituting alginic acid
(D) represents one residue of a nonsteroidal anti-inflammatory compound; preferably one residue of diclofenac;
$R^1$ and $R^2$ are each independently a group selected from a hydrogen atom, a halogen atom, a methyl group, an ethyl group, and an ethoxycarbonyl group (the $R^1$ and $R^2$ can together form an oxo group (=O));
$R^3$ and $R^4$ are each independently a group selected from a hydrogen atom, a halogen atom, a methyl group, an ethyl group, and an ethoxycarbonyl group (the $R^3$ and $R^4$ can together form an oxo group (=O));
$R^5$ and $R^6$ are each independently a group selected from a hydrogen atom, a halogen atom, a methyl group, an ethyl group, and an ethoxycarbonyl group (the $R^5$ and $R^6$ can together form an oxo group (=O));
Z is an oxygen atom; and
n1, n3, n5, n6, and n8 represent any integer of 1 to 4 in total).

In addition, a specific example of the formula (6a) is a water-soluble alginic acid derivative selected from the following formulas:

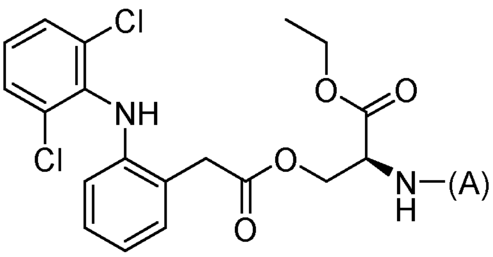

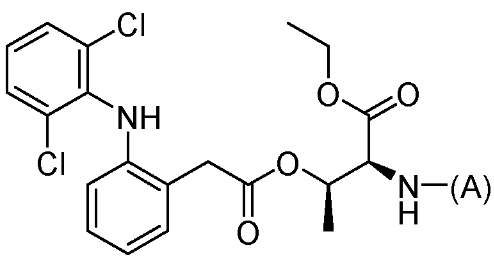

(in each formula, (A) represents one residue derived from alginic acid or the salt thereof which has a C(=O)- group in a monosaccharide of either L-guluronic acid or D-mannuronic acid constituting alginic acid).

In addition, in the formula (2a), a preferable water-soluble alginic acid derivative is an alginic acid derivative having a structure represented by the following formula (7a):

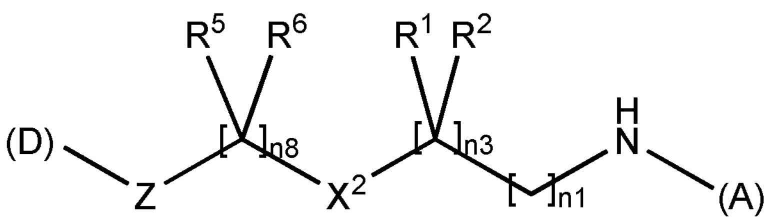

(7a)

(wherein

(A) represents one residue derived from alginic acid or the salt thereof which has a C(=O)- group in a monosaccharide of either L-guluronic acid or D-mannuronic acid constituting alginic acid;
(D) is a residue of a nonsteroidal anti-inflammatory compound; preferably a residue of a nonsteroidal anti-inflammatory compound selected from diclofenac, ketoprofen, naproxen, and felbinac;
$R^1$ and $R^2$ are each independently a group selected from a hydrogen atom, a halogen atom, a methyl group, and an ethyl group (the $R^1$ and $R^2$ can together form an oxo group (=O)); preferably, $R^1$ and $R^2$ together form an oxo group (=O);
$R^5$ and $R^6$ are each independently a group selected from a hydrogen atom, a halogen atom, a methyl group, and an ethyl group (the $R^5$ and $R^6$ can together form an oxo group (=O)); preferably, $R^5$ is a hydrogen atom, and $R^6$ is a hydrogen atom or a methyl group;
$X^2$ is an imino group (NH);
Z is an oxygen atom; and
n1, n3, and n8 represent any integer of 1 to 10 in total).

In addition, a specific example of the formula (7a) is, for example, a water-soluble alginic acid derivative selected from the following formulas:

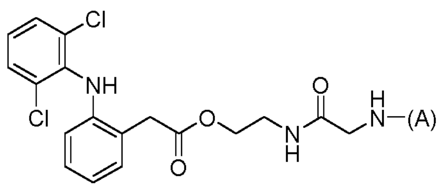
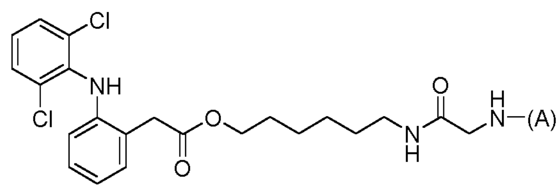
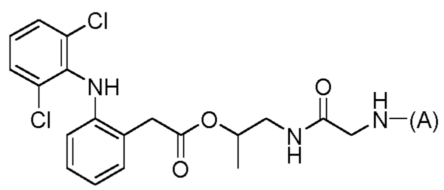
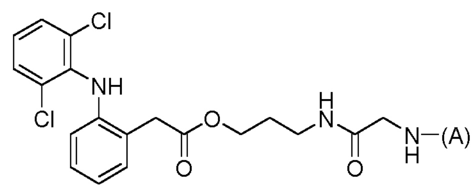
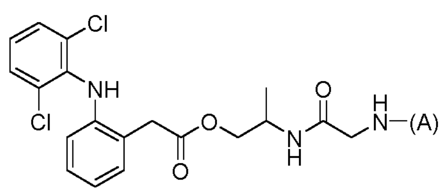
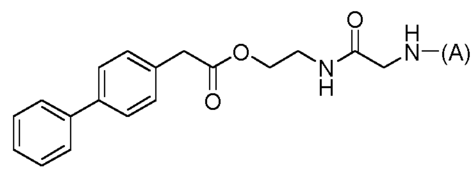
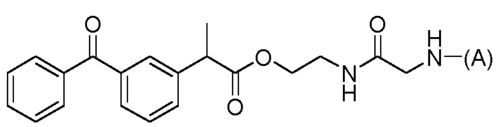
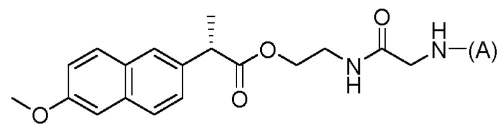

(wherein (A) represents one residue derived from alginic acid or the salt thereof which has a C(=O)- group in a monosaccharide of either L-guluronic acid or D-mannuronic acid constituting alginic acid).
In addition, in the formula (2a), a preferable water-soluble alginic acid derivative is an alginic acid derivative having a structure represented by the following formula (8a):

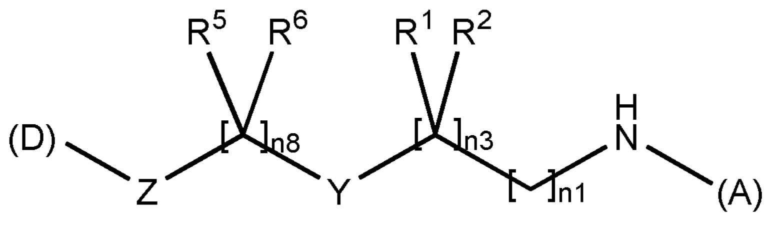

(8a)

(wherein

(A) represents one residue derived from alginic acid or the salt thereof which has a C(=O)- group in a monosaccharide of either L-guluronic acid or D-mannuronic acid constituting alginic acid;
(D) represents one residue of a nonsteroidal anti-inflammatory compound; preferably one residue of diclofenac;
$R^1$ and $R^2$ are each independently a group selected from a hydrogen atom, a halogen atom, a methyl group, and an ethyl group (the $R^1$ and $R^2$ can together form an oxo group (=O)); preferably, $R^1$ and $R^2$ together form an oxo group (=O);
$R^5$ and $R^6$ are each independently a group selected from a hydrogen atom, a halogen atom, a methyl group, and an ethyl group (the $R^5$ and $R^6$ can together form an oxo group (=O)); preferably, $R^5$ is a hydrogen atom, and $R^6$ is a hydrogen atom;
Y is a heterocycle; preferably a piperidine ring;
Z is an oxygen atom; and
n1, n3, and n8 represent any integer of 1 to 5 in total).

In addition, a specific example of the formula (8a) is a water-soluble alginic acid derivative selected from the following formulas:

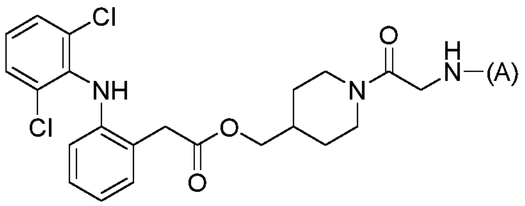

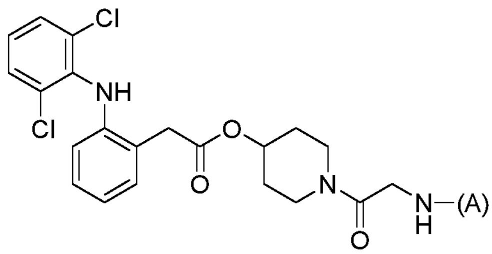

(wherein (A) represents one residue derived from alginic acid or the salt thereof which has a C(=O)- group in a monosaccharide of either L-guluronic acid or D-mannuronic acid constituting alginic acid). Note that the linker structure of the water-soluble alginic acid derivative of the present invention is described later in the section of «Linker».

In addition, in the water-soluble alginic acid derivative of the present invention, the introduction rate of a nonsteroidal anti-inflammatory compound is preferably such that side effects are unlikely to occur in the body, and the nonsteroidal anti-inflammatory compound can be appropriately released continuously, for example, at a concentration capable of alleviating or relieving arthritis. For example, the introduction rate (mol%) is preferably 1.0 mol% or more, more preferably 2.0 mol% or more, and further preferably 4.0% or more. Here, regarding the introduction rate (mol%) in the present invention, for example, consider the case of introducing a nonsteroidal anti-inflammatory compound into the carboxyl group of L-guluronic acid or D-mannuronic acid constituting alginic acid through a linker. The introduction rate of 10 mol% means such a ratio that 10 nonsteroidal anti-inflammatory compounds have been introduced into 100 monosaccharides, with the monosaccharide of L-guluronic acid or D-mannuronic acid constituting alginic acid being defined as 1 unit (count). Therefore, a nonsteroidal anti-inflammatory compound may be introduced into a carboxyl group of adjacent monosaccharides through a linker.

The type of the linker and the introduction rate of the nonsteroidal anti-inflammatory compound can be appropriately adjusted in consideration of, for example, the final dosage form (such as gel form, sol form, or microbeads form) of the pharmaceutical composition containing the compound described later, or the required amount or sustained release efficiency of the nonsteroidal anti-inflammatory compound in the diseased site when administered to the living body.

Here, the water-soluble alginic acid derivative of the present invention is a polymer compound containing a nonsteroidal anti-inflammatory compound, and is characterized by being water-soluble. That is, even when the introduction rate of the water-soluble alginic acid derivative of a nonsteroidal anti-inflammatory compound, which is generally known to be hydrophobic, is high, for example 10 mol% or more, it is soluble in water. For example, it is demonstrated that, when 0.1 parts by mass of the water-soluble alginic acid derivative are added to 100 parts by mass of water and shaken or stirred at room temperature (for example, 20°C), it does not become a gel form but dissolves within 24 hours. That is, it is demonstrated that the water-soluble alginic acid derivative of the present invention dissolves in an aqueous solvent at a concentration of 0.1% or more. Note that, in the present specification, the "room temperature" generally means a temperature of about 0°C to about 35°C unless otherwise specified.

In addition, the water solubility of the water-soluble alginic acid derivative in the present invention is equivalent to the water solubility of, for example, sodium alginate salt, and there is an advantage that the gelation or sol formation is easy to handle according to the use described later. Therefore, the solution of the water-soluble alginic acid derivative

of the present invention can be filtered with a filter, and dust removal, bacteria reduction, and bacteria elimination can be performed by filter filtration. That is, dust removal and bacteria reduction can be performed by passing through a filter of 5 μm to 0.45 μm, and furthermore, bacteria elimination can be performed by passing through a filter of, desirably, 0.22 μm.

Note that the water-soluble alginic acid derivative of the present invention can be dissolved in water, aqueous solvents such as a solution containing a pharmaceutically acceptable metal salt or a pH adjuster, and a buffer. Specifically, it can be dissolved in water for injection, phosphate buffered saline, physiological saline, and the like.

In addition, the water-soluble alginic acid derivative in the present invention does not bring about the anti-inflammatory effect possessed by the nonsteroidal anti-inflammatory compound by itself, but when, for example, it is administered *in vivo,* the nonsteroidal anti-inflammatory compound is appropriately cleaved from the linker according to the situation *in vivo,* and the nonsteroidal anti-inflammatory compound is released to exert its effect. The nonsteroidal anti-inflammatory compound continues to release only the amount necessary for suppressing inflammation in the diseased site and for analgesia, and as a result, it is possible to stably concentrate on the diseased site for a certain period of time to bring about an anti-inflammatory effect and an analgesic effect. The water-soluble alginic acid derivative, depending on the structure of the linker which is a constituent component thereof, can adjust the sustained release rate of the nonsteroidal anti-inflammatory compound to a desired mode. Thus, optimization of the combination of the introduction rate of the nonsteroidal anti-inflammatory compound and the type of the linker depending on the desired effect makes it possible to bring about a long-term sustainable analgesic action and anti-inflammatory action in the case of injection *in vivo,* for example, injection into the joint cavity, and especially injection into the knee joint cavity.

In addition, since alginic acid is not degraded by the enzyme *in vivo,* the release rate of the nonsteroidal anti-inflammatory compound is hardly affected by any factors except for the cleavage of the linker site, and the water-soluble alginic acid derivatives in the present invention can stably release a certain active ingredient.

In the water-soluble alginic acid derivative of the present invention, by changing the binding mode between alginic acid or a salt thereof and the linker and the binding mode between the nonsteroidal anti-inflammatory compound and the linker, the degradability and the order of degradation *in vivo* can be changed, and as a result, it becomes possible to control the release rate and release speed of the nonsteroidal anti-inflammatory compound. Specifically, it is known that ester bonds are more susceptible to hydrolysis than amide bonds *in vivo.* The order of degradation does not matter as long as the nonsteroidal anti-inflammatory compound is finally released, but in the water-soluble alginic acid derivative of the present invention, preferably, the bond between the nonsteroidal anti-inflammatory compound and the linker is first hydrolyzed to release the nonsteroidal anti-inflammatory compound. Specifically, alginic acid or a salt thereof and a linker are bound by an amide bond, and a nonsteroidal anti-inflammatory compound and a linker are bound by an ester bond, thereby the ester bond is first hydrolyzed, releasing the nonsteroidal anti-inflammatory compound first from the linker.

In addition, alginic acid does not adversely affect the living body to which it is applied, and a specific receptor that binds to alginic acid *in vivo* has not been identified, and thus alginic acid or a salt thereof after releasing the nonsteroidal anti-inflammatory compound is degraded in the body without causing toxicity.

Preferably, the water-soluble alginic acid derivative of the present invention is released very slowly in a situation where sustained release is expected for a long period of time under mildly acidic conditions. For example, when the water-soluble alginic acid derivative of the present invention is prepared in an aqueous solution having a concentration of 0.1% by mass and incubated at 37°C for 7 days, the nonsteroidal anti-inflammatory compound preferably exhibits the behavior of being released at a release rate of 1.0% or less at pH 5.3. In addition, in a situation where a short-term sustained release is expected under neutral conditions, slow release is preferable. For example, under the above condition (pH 7.0), the compound exhibits the behavior of being released at a release rate of preferably more than 0% and 50% or less, the behavior of being released at a release rate of more preferably 0.04 to 45%, the behavior of being released at a release rate of more preferably 1 to 40%, and the behavior of being released at a release rate of further preferably 1.5 to 30%.

As above, the water-soluble alginic acid derivative of the present invention can be used properly according to the release rate at various pH depending on the use environment. In addition, it is possible to further enhance the sustained release effect by gelling the water-soluble alginic acid derivative of the present invention with a cross-linking agent.

For example, when the water-soluble alginic acid derivative of the present invention is used as an arthritis therapeutic agent for intra-articular administration of the knee joint, and the pH of the inflamed diseased site exhibits weakly acidic behavior, it is expected that the sustained release of the nonsteroidal anti-inflammatory compound stably continues for 7 days or longer, preferably 15 days or longer, and more preferably 30 days or longer after administration to the diseased site by injection or the like. Here, the release rate indicates the ratio of the released amount of the nonsteroidal anti-inflammatory compound to the total amount of the nonsteroidal anti-inflammatory compound contained in the water-soluble alginic acid derivative.

When the water-soluble alginic acid derivative of the present invention is used, the effective amount of the drug

is effectively retained in the diseased site, as compared with sole administration of a drug, in the case of administration to the diseased site such as in the knee joint cavity or its nearby areas, and a strong therapeutic effect can be expected even with a smaller amount of the drug than in the case of oral administration. In addition, by adjusting the sustained release and sustainability, it is possible to reduce the number of administrations clinically.

Hereinafter, for the purpose of explaining the constitution of the water-soluble alginic acid derivative of the present invention, description is provided for the alginic acid, the linker, and the nonsteroidal anti-inflammatory compound, which are the constituent components, and then the water-soluble alginic acid derivative gel, applications thereof, and the like are described in detail.

<<Alginic Acid or Salt Thereof>>

In the present invention, alginic acid or a salt thereof is preferably a "monovalent metal salt of alginic acid," which is a water-soluble salt formed by ion-exchanging the hydrogen atom of the carboxylic acid of D-mannuronic acid or L-guluronic acid of alginic acid, with a monovalent metal ions such as $Na^+$ or $K^+$. The monovalent metal salt of alginic acid is, specifically, sodium alginate or potassium alginate, and sodium alginate is particularly preferable. As described later, the solution of the monovalent metal salt of alginic acid can adjust the form of the water-soluble alginic acid derivative of the present invention by using the property of forming a gel when mixed with a cross-linking agent.

Alginic acid is a type of natural polysaccharide produced by extracting and purifying brown algae seaweed. In addition, it is a polymer obtained by polymerizing D-mannuronic acid (M) and L-guluronic acid (G). The composition ratio (M/G ratio) of D-mannuronic acid and L-guluronic acid of alginic acid differs mainly depending on the type of organism from which alginic acid is derived such as seaweed or the like, and the ratio is affected by the habitat of the organism and the season, and vastly ranges from a high G type having an M/G ratio of about 0.4 to a high M type having an M/G ratio of about 5. The physicochemical properties of alginic acid may differ depending on the M/G ratio of alginic acid, the arrangement of M and G, and the like, and preferable applications may differ. The industrial production method of alginic acid includes an acid method and a calcium method, but in the present invention, any of the production methods can be used. By purification, the quantitative value by the HPLC method is preferably in the range of 80 to 120% by mass, more preferably in the range of 90 to 110% by mass, and further preferably in the range of 95 to 105% by mass. In the present invention, a highly purified one having a quantitative value by the HPLC method within the above range is referred to as high-purity alginic acid. The alginic acid or salt thereof used in the present invention is preferably high-purity alginic acid. As a commercially available product, for example, Kimica Algin Series sold by KIMICA, preferably the high-purity food/pharmaceutical grade can be purchased and used. It is also possible to use a commercially available product with occasionally further purification. For example, low endotoxin treatment is preferable. As the purification method and the low endotoxin treatment method, for example, the method described in Japanese Patent Application Publication No. 2007-75425(Patent Literature 1) can be employed.

As the alginic acid or salt thereof used in the present invention, it is preferable to use one having an appropriate weight average molecular weight depending on the end use application. For example, in the case of use as an arthritis therapeutic agent for intra-articular administration, it is preferable to use one having a weight average molecular weight of 10,000 to 10,000,000, more preferably 100,000 to 5,000,000, and further preferably 200,000 to 3,000,000. More specifically, for example, it is preferable to use any of alginic acid or salt thereof A1 to A4 having the physical properties presented in Table 1 below.

Table 1

| | Weight Average Molecular Weight (Da) |
|---|---|
| A1 | 684,000 to 1,026,000 |
| A2 | 1,352,000 to 2,028,000 |
| A3 | 1,424,000 to 2,136,000 |
| A4 | 1,312,000 to 1,968,000 |

In addition, as the alginate, commercially available sodium alginates (sold by Mochida Pharmaceutical Co., Ltd.) presented below can also be used. Here, in Examples 12 to 22 described later, as the sodium alginate, the sodium alginates of A-1, A-2, A-3, and B-2 presented in the table below were used. Table 2 presents the viscosity, weight average molecular weight, and M/G ratio of a 1 w/w% aqueous solution of each sodium alginate.

Table 2

| Sodium Alginate | Viscosity for 1 w/w% (mPa·s) | Weight Average Molecular Weight | | M/G Ratio |
|---|---|---|---|---|
| | | GPC | GPC-MALS | |
| A-1 | 10 to 40 | 300,000 to 700,000 | 60,000 to 130,000 | 0.5 to 1.8 |
| A-2 | 50 to 150 | 700,000 to 1,400,000 | 130,000 to 200,000 | |
| A-3 | 300 to 600 | 1,400,000 to 2,000,000 | 200,000 to 400,000 | |
| B-1 | 10 to 40 | 150,000 to 800,000 | 60,000 to 130,000 | 0.1 to 0.5 |
| B-2 | 70 to 150 | 800,000 to 1,500,000 | 130,000 to 200,000 | |
| B-3 | 400 to 600 | 1,500,000 to 2,500,000 | 200,000 to 350,000 | |

The physical properties of the sodium alginates A-1, A-2, A-3, B-1, B-2, and B-3 were measured by the method described below. Although the measurement method is not limited to that method, each physical property value may differ from the above depending on the measurement method.

[Viscosity Measurement of Sodium Alginate]

According to the viscosity measurement method of the Japanese Pharmacopoeia (16th Edition), measurement was performed using the rotational viscometer method (cone plate type rotational viscometer). The specific measurement conditions are as follows. The sample solution was prepared using MilliQ water. A cone plate type rotational viscometer (viscosity and viscoelasticity measuring device RheoStress RS600 (Thermo Haake GmbH) sensor: 35/1) was used as a measuring instrument. The rotation speed was 1 rpm when measuring a 1 w/w% sodium alginate solution. The measurement was performed for 2 minutes, and the average value from 1 minute to 2 minutes from the startwas recorded. The average value of three measurements was used as the measured value. The measurement temperature was 20°C.

[Measurement of Weight Average Molecular Weight of Sodium Alginate]

The measurement was performed by two types of measurement methods, (1) gel permeation chromatography (GPC) and (2) GPC-MALS. The measurement conditions are as follows.

[Pretreatment Method]

An eluent was added to the sample for dissolution, which was filtered through a 0.45 μm membrane filter to obtain a measurement solution.

(1) Gel Permeation Chromatography (GPC) Measurement

[Measurement Conditions (Relative Molecular Weight Distribution Measurement)]

Column: TSKgel GMPW-XL × 2 + G2500PW-XL (7.8 mm I.D. × 300 mm × 3)
Eluent: 200 mM sodium nitrate aqueous solution
Flow rate: 1.0 mL/min
Concentration: 0.05%
Detector: RI detector
Column temperature: 40°C
Injection volume: 200 μL
Molecular weight standard: standard pullulan, glucose

(2) GPC-MALS Measurement

[Refractive Index Increment (dn/dc) Measurement (Measurement Conditions)]

Differential refractometer: Optilab T-rEX
Measurement wavelength: 658 nm
Measurement temperature: 40°C
Solvent: 200 mM sodium nitrate aqueous solution
Sample concentration: 0.5 to 2.5 mg/mL (5 concentrations)

[Measurement Conditions (Absolute Molecular Weight Distribution Measurement)]

Column: TSKgel GMPW-XL × 2 + G2500PW-XL (7.8 mm I.D. × 300 mm × 3)
Eluent: 200 mM sodium nitrate aqueous solution
Flow rate: 1.0 mL/min
Concentration: 0.05%
Detector: RI detector, light scattering detector (MALS)
Column temperature: 40°C
Injection volume: 200 μL

In the present specification, the molecular weights of alginic acid, alginic acid derivatives, cross-linked alginic acid, and cross-linked alginic acid are sometimes described with Da (Dalton) as a unit.

The composition ratio (M/G ratio) of D-mannuronic acid and L-guluronic acid of alginic acids differs mainly depending on the type of organism from which the alginic acids are derived such as seaweed or the like, and the ration is affected by the habitat of the organism and the season, and vastly ranges from a high G type having an M/G ratio of about 0.2 to a high M type having an M/G ratio of about 5. It is known that the gelling ability of alginic acids and the properties of produced gel are affected by the M/G ratio, and generally, the gel strength increases when the G ratio is high. The M/G ratio also affects the hardness, brittleness, water absorption, and flexibility of the gel. The M/G ratio of the alginic acids and/or salts thereof used is usually 0.2 to 4.0, more preferably 0.4 to 3.0, and further preferably 0.5 to 3.0.

Here, a polymer substance derived from a natural product does not having a single molecular weight, but is generally an aggregate of molecules having various molecular weights, and thus is measured as a molecular weight distribution having a certain range. A typical measurement method is gel filtration chromatography. Typical information on the molecular weight distribution obtained by gel filtration chromatography includes a weight average molecular weight (Mw), a number average molecular weight (Mn), and a dispersion ratio (Mw/Mn).

The weight average molecular weight emphasizes the contribution of high molecular weight polymers to the average molecular weight, and is represented by the following formula.

$$Mw = \Sigma(WiMi)/W = \Sigma(HiMi)/\Sigma(Hi)$$

The number average molecular weight is calculated by dividing the total weight of polymers by the total number of polymers.

$$Mn = W/\Sigma Ni = \Sigma(MiNi)/\Sigma Ni = \Sigma(Hi)/\Sigma(Hi/Mi)$$

Here, W is the total weight of the polymers, Wi is the weight of the i-th polymer, Mi is the molecular weight at the i-th elution time, Ni is the number of the molecular weights Mi, and Hi is the height at the i-th elution time.

It is known that, in the measurement of the molecular weight of a polymer substance derived from a natural product, the value may differ depending on the measuring method (examples of hyaluronic acid: Chikako YOMOTA et.al. Bull. Natl. Health Sci., Vol. 117, pp 135-139 (1999), Chikako YOMOTA et.al. Bull. Natl. Inst. Health Sci., Vol. 121, pp 30-33 (2003)). Regarding the measurement of the molecular weight of alginic acid, there is a document that describes a method of calculating from intrinsic viscosity and a method of calculating by SEC-MALLS (Size Exclusion Chromatography with Multiple Angle Laser Light Scattering Detection) (ASTM F2064-00 (2006), published by ASTM International). Note that this document states that, when measuring the molecular weight by size exclusion chromatography (= gel filtration chromatography), it is not enough to calculate with a calibration curve using pullulan as a standard substance, and it is recommended to use a multi-angle light scattering detector (MALLS) together (= measurement by SEC-MALLS). There is also an example in which the molecular weight by SEC-MALLS is used as the standard value on the catalog of alginic acid (FMC Biopolymer, PRONOVATM sodium alginates catalogue).

Note that, when the molecular weight of a polymeric polysaccharide is calculated by the above method, a measurement error of 10 to 20% may usually occur. For example, if the value is 400,000, the value may fluctuate in the range of 320,000 to 480,000, and if it is 1,000,000, the value may fluctuate in the range of 800,000 to 1,200,000.

In the present specification, the molecular weight of sodium alginate used is, for example, preferably in the range of 300,000 to 2,500,000, and more preferably in the range of 300,000 to 900,000, or more preferably in the range of 700,000 to 1,700,000, or more preferably in the range of 1,400,000 to 2,000,000 in a weight average molecular weight (GPC).

Unless otherwise specified, when identifying the molecular weight of alginic acid or a salt thereof in the present specification, it is the weight average molecular weight calculated by gel filtration chromatography. As the conditions of the gel filtration chromatography, for example, the conditions of the present example described later can be employed.

Further, as alginic acid or a salt thereof used in the present invention, it is preferable to use one having an appropriate viscosity and an appropriate M/G ratio depending on the end use application. The alginic acid or salt thereof used in the present invention preferably has a reduced endotoxin level. The endotoxin value measured by the endotoxin test of the Japanese Pharmacopoeia is preferably less than 100 EU/g, more preferably less than 75 EU/g, and further preferably less than 50 EU/g. In the present invention, "substantially free of endotoxin" means that the endotoxin value measured by the endotoxin test of the Japanese Pharmacopoeia is within the above numerical range.

«Linker»

The linker of the water-soluble alginic acid derivative of the present invention is not particularly limited as long as it has a functional group capable of binding to one residue derived from alginic acid or a salt thereof via an amide bond, has a functional group capable of binding to one residue of a nonsteroidal anti-inflammatory compound via an ester bond, and has a structure capable of forming a water-soluble alginic acid derivative, as described above, but its preferable example has a structure represented by the following formula (7).

$$-NH-(CH_2)_{n1}-[X^1]_{n2}-(CR^1R^2)_{n3}-[Y]_{n4}-(CH_2)_{n5}-(CR^3R^4)_{n6}-[X^2]_{n7}-(CH_2)_{n8}-[Z]- \quad (7)$$

In the formula (7), -NH represents an end forming an amide bond with one residue of alginic acid or a salt thereof, and [Z]- represents an end forming an ester bond with one residue of a nonsteroidal anti-inflammatory compound. Depending on the structure of the binding moiety of the nonsteroidal anti-inflammatory compound, Z may be O or C(=O), but is preferably O. In the formula (7), $X^1$ and $X^2$ each represent a hetero atom, preferably any atom selected from O, S, and N (in the case of N, strictly, it represents N(H)), and more preferably represent O or N. In the formula (7), $R^1$, $R^2$, $R^3$, and $R^4$ each independently represent hydrogen, a halogen atom, a $C_{1-10}$ alkyl group, a $C_{1-10}$ alkoxy group, or a $C_{1-10}$ alkoxycarbonyl group, and preferably hydrogen, fluorine, a $C_{1-6}$ alkyl group, a $C_{1-6}$ alkoxy group, or a $C_{1-6}$ alkoxycarbonyl group, or $R^1$ and $R^2$ or $R^3$ and $R^4$ together represent =O.

Y represents a cycloalkane ring, an aromatic ring, or a heterocycle (the cycloalkane ring, the aromatic ring, or the heterocycle may be substituted with a halogen atom or a $C_{1-10}$ alkyl group), preferably represents a cycloalkane ring, an aromatic ring, or a heterocycle, and more preferably an aromatic ring.

The n1 represents any integer of 0 to 10, and n2 to n8 independently represent any integer of 0 to 3, provided that not all of n1 to n8 are 0. Preferably, n2, n4, and n7 are independently 0 to 2, and more preferably independently 0 or 1. In addition, n3, n5, n6, and n8 are preferably 1 to 12 in total, and more preferably 2 to 10 in total.

Moreover, the linker of the water-soluble alginic acid derivative of the present invention preferably has a structure represented by, for example, the following formula (LK) [excluding both sides of the broken lines in the formula].

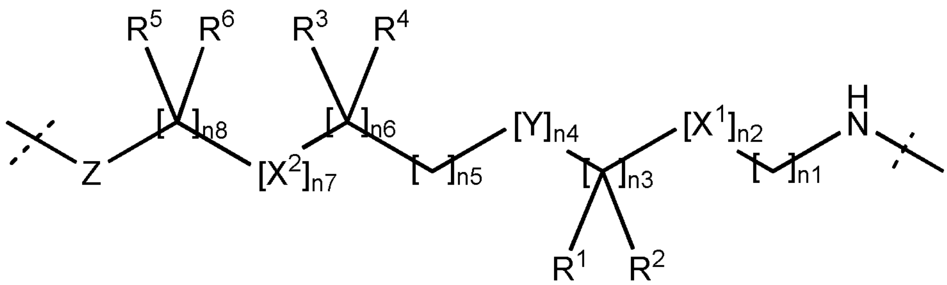

(LK)

wherein -NH is an end forming an amide bond with one residue of alginic acid or a salt thereof;

Z- is an end forming an ester bond with one residue of a nonsteroidal anti-inflammatory compound; Z is an oxygen atom or a carbonyl group, depending on the structure of the binding moiety of the nonsteroidal anti-inflammatory

compound, and preferably an oxygen atom;

$X^1$ and $X^2$ represent a hetero atom, and preferably an oxygen atom or an imino group (NH);

$R^1$, $R^2$, $R^3$, $R^4$, $R^5$, and $R^6$ are each independently a group selected from a hydrogen atom, a halogen atom, a $C_{1-6}$ alkyl group, a $C_{1-6}$ alkoxy group, and a $C_{1-6}$ alkoxycarbonyl group, preferably a group selected from a hydrogen atom, a fluorine atom, a $C_{1-6}$ alkyl group, a $C_{1-6}$ alkoxy group, and a $C_{1-6}$ alkoxycarbonyl group ($R^1$ and $R^2$, $R^3$ and $R^4$, or $R^5$ and $R^6$ can together form =O), and more preferably a group selected from a hydrogen atom, a fluorine atom, a $C_{1-3}$ alkyl group, a $C_{1-3}$ alkoxy group, and a $C_{1-3}$ alkoxycarbonyl group ($R^1$ and $R^2$, $R^3$ and $R^4$, or $R^5$ and $R^6$ can together form =O);

Y is a $C_{3-8}$ cycloalkyl ring, a $C_{6-10}$ aryl ring, or a heterocycle (the $C_{3-8}$ cycloalkyl ring, $C_{6-10}$ aryl ring, or heterocycle may be substituted with a halogen atom or a $C_{1-6}$ alkyl group), preferably a $C_{6-10}$ aryl ring or a heterocycle, and more preferably a benzene ring or a piperidine ring;

n1 or n8 are each independently any integer of 0 to 10;

n3, n5, or n6 are each independently any integer of 0, 1, 2, or 3; n2, n4, or n7 are each independently any integer of 0 or 1;

provided that not all of n1 to n8 are 0;

preferably, n2, n4, and n7 are each independently 0 to 2, and more preferably independently 0 or 1; and

in addition, n3, n5, and n6 are preferably 1 to 12 in total, and more preferably 2 to 10 in total.

In the present specification, unless otherwise specified, the "hetero atom" is, for example, O, S, N, P, or the like.

In the present specification, unless otherwise specified, the "halogen atom" is, for example, a fluorine atom, a chlorine atom, a bromine atom, an iodine atom, and the like.

In the present specification, unless otherwise specified, the "$C_{1-10}$ alkyl (group)" is methyl, ethyl, propyl, isopropyl, butyl, isobutyl, sec-butyl, tert-butyl, pentyl, isopentyl, neopentyl, tert-pentyl, 3-methylbutyl, 1,2-dimethylpropyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, cyclopropyl, cyclobutyl, cyclopentyl, cyclohexyl, cyclopropylmethyl, cyclobutylmethyl, cyclopentylmethyl, 1-cyclopropylethyl, 2-cyclopropylethyl, 2-cyclobutylethyl, 2-methylcyclopropyl, heptyl, 1-methylhexyl, octyl, 2-ethylhexyl, 1,1-dimethylhexyl, nonyl, decyl, cycloheptyl, cyclohexylmethyl, 2-cyclohexylethyl, 4-methylcyclohexyl, 4,4-dimethylcyclohexyl, 3,3,5,5-tetramethylcyclohexyl, and the like.

In the present specification, unless otherwise specified, examples of the "$C_{1-6}$ alkyl (group)" include groups such as methyl, ethyl, n-propyl, isopropyl, n-butyl, isobutyl, sec-butyl, tert-butyl, n-pentyl, isopentyl, neopentyl, n-hexyl, isohexyl, 1-methylpentyl, 2-methylpentyl, and 3-methylpentyl.

In the present specification, unless otherwise specified, examples of the "$C_{1-3}$ alkyl (group)" include groups such as methyl, ethyl, n-propyl, and isopropyl.

In the present specification, unless otherwise specified, the "$C_{1-10}$ alkoxy (group)" is, for example, methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, 2-methylbutoxy, 1,2-dimethylpropoxy, 1-ethylpropoxy, hexyloxy, isohexyloxy, 1-methylpentyloxy, 2-methylpentyloxy, 3-methylpentyloxy, 1,1-dimethylbutyloxy, 1,2-dimethylbutyloxy, 2,2-dimethylbutyloxy, 1,3-dimethylbutyloxy, 2,3-dimethylbutyloxy, 3,3-dimethylbutoxy, 1-ethylbutyloxy, 2-ethylbutyloxy, 1,1,2-trimethylpropyloxy, 1,2,2-trimethylpropyloxy, 1-ethyl-1-methylpropyloxy, 1-ethyl-2-methylpropyloxy, cyclopropyloxy, cyclobutyloxy, cyclopentyloxy, cyclohexyloxy, cyclopropylmethoxy, cyclobutylmethoxy, cyclopentylmethoxy, 1-cyclopropylethoxy, 2-cyclopropylethoxy, 2-cyclobutylethoxy, 2-methylcyclopropyloxy, heptyloxy, octyloxy, 2-ethylhexyloxy, nonyloxy, decyloxy, cycloheptyloxy, cyclohexylmethoxy, 2-cyclohexylethoxy, 4-methylcyclohexyloxy, 4,4-dimethylcyclohexyloxy, 3,3,5,5-tetramethylcyclohexyloxy, and the like.

In the present specification, unless otherwise specified, examples of the "$C_{1-6}$ alkoxy group" include groups such as methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, 2-methylbutoxy, 1,2-dimethylpropoxy, 1-ethylpropoxy, hexyloxy, isohexyloxy, 1-methylpentyloxy, 2-methylpentyloxy, and 3-methylpentyloxy.

In the present specification, unless otherwise specified, examples of the "$C_{1-3}$ alkoxy group" include groups such as methoxy, ethoxy, propoxy, and isopropoxy.

In the present specification, unless otherwise specified, the "$C_{1-10}$ alkoxycarbonyl group" is -C(=O)-R (R is a $C_{1-10}$ alkoxy group).

In the present specification, unless otherwise specified, the "$C_{1-6}$ alkoxycarbonyl group" is -C(=O)-R (R is a $C_{1-6}$ alkoxy group), and examples thereof include groups such as a methoxycarbonyl group, an ethoxycarbonyl group, and a tert-butoxycarbonyl group.

In the present specification, unless otherwise specified, the "$C_{1-3}$ alkoxycarbonyl group" is -C(=O)-R (R is a $C_{1-3}$ alkoxy group), examples thereof include groups such as a methoxycarbonyl group, an ethoxycarbonyl group, a propoxycarbonyl group, and an isopropoxycarbonyl group.

In addition, $C_{1-10}$ in the above $C_{1-10}$ alkyl group, $C_{1-10}$ alkoxy group, and $C_{1-10}$ alkoxycarbonyl group is preferably $C_{1-6}$, and more preferably $C_{1-3}$.

In the present specification, unless otherwise specified, the "cycloalkane ring" is cyclopropane, cyclobutane, cyclopentane, cyclohexane, cycloheptane, cyclooctane, cyclononane, cyclodecane, and the like.

In the present specification, unless otherwise specified, the "aromatic ring" is a benzene ring, a 1-naphthalene ring, a 2-naphthalene ring, a 2-, 3-, 4-biphenylanthrone ring, a phenanthrene ring, an acenaphthene ring, and the like.

In the present specification, unless otherwise specified, examples of the "$C_{3-8}$ cycloalkyl ring" include cycloalkyl rings such as cyclopropane, cyclobutane, cyclopentane, and cyclohexane.

In the present specification, unless otherwise specified, examples of the "$C_{6-10}$ aryl ring" include rings such as a benzene ring, a 1-naphthalene ring, a 2-naphthalene ring, a 2-, 3-, 4-biphenylanthrone ring, a phenanthrene ring, and an acenaphthene ring.

In the present specification, unless otherwise specified, the "heterocycle" means a 3- to 14-membered monocyclic or fused ring containing 1 to 5 heteroatoms of nitrogen atom, sulfur atom, or oxygen atom.

In the present specification, unless otherwise specified, the "heterocycle" includes "aromatic heterocycle," "partially hydrogenated fused heterocycle," and "non-aromatic heterocycle".

In the present specification, unless otherwise specified, the "aromatic heterocycle" includes a monocyclic aromatic heterocycle having 5 to 7 ring members, and preferably includes, for example, pyrrole, furan, thiophene, imidazole, pyrazole, oxazole, isoxazole, thiazole, isothiazole, 1,2,3-triazole, 1,2,4-triazole, 1,2,3-oxadiazole, 1,2,4-oxadiazole, 1,3,4-oxadiazole, furazar, 1,2,3-thiadiazole, 1,2,4-thiadiazole, 1,3,4-thiadiazole, tetrazole, pyridine, pyridazine, pyrimidine, pyrazine, 1,2,3-triazine, 1,2,4-triazine, 1,3,5-triazine, 2H-1,2,3-thiadiazine, 4H-1,2,4-thiadiazine, 6H-1,3,4-thiadiazine, 1,4-diazepine, 1,4-oxazepine, and the like.

In the present specification, unless otherwise specified, the "aromatic heterocycle" includes a fused aromatic heterocycle having 8 to 12 ring members (fused heterocycle), and preferably includes, for example, indole, isoindole, benzofuran, isobenzofuran, benzothiophene, isobenzothiophene, benzoxazole, 1,2-benzisoxazole, benzothiazole, 1,2-benzisothiazole, 1H-benzimidazole, 1H-indazole, 1H-benzotriazole, 2,1,3-benzothiadiazine, chromene, isochromene, 4H-1,4-benzoxazine, 4H-1,4-benzothiazine, quinoline, isoquinoline, cinnoline, quinazoline, quinoxaline, phthalazine, benzoxazepine, benzazepine, benzodiazepine, naphthyridine, purine, pteridine, carbazole, carboline, acridinine, phenoxazine, phenothiazine, phenazine, phenoxathiin, cyananthrene, thianthrene, phenanthridine, phenanthroline, indolizine, thieno[3,2-c]pyridine, thiazolo[5,4-c]pyridine, pyrrolo[1,2-b]pyridazine, pyrazolo[1,5-a]pyridine, imidazo[1,2-a]pyridine, imidazo[1,5-a]pyridine, imidazo[1,2-b]pyridazine, imidazo[1,5-a]pyrimidine, 1,2,4-triazolo[4,3-a]pyridine, 1,2,4-triazolo[4,3-b]pyridazine, 1H-pyrazolo[3,4-b]pyridine, 1,2,4-triazolo[1,5-a]pyrimidine, and the like.

In the present specification, unless otherwise specified, the "partially hydrogenated fused heterocycle" means a fused ring obtained by partially hydrogenating any ring in a fused ring formed by fusion of a "heterocycle" and a "$C_{6-10}$ aryl ring," or a "heterocycle" and an "aromatic heterocycle."

In the present specification, unless otherwise specified, the "partially hydrogenated fused heterocycle" is preferably one having 8 to 12 ring members, and examples thereof include rings such as indoline, 4,5,6,7-tetrahydro-1H-indoline, 2,3-dihydrobenzofuran, 4,5,6,7-tetrahydro-benzofuran, 2,3-dihydrobenzo[d]oxazoline, 2,3-dihydrobenzo[d]thiazoline, chroman, 2H-chromene, 4H-chromene, isochroman, 1H-isochromene, 3,4-dihydro-2H-1,4-benzoxazine, 3,4-dihydro-2H-1,4-benzothiazine, 5,6,7,8-tetrahydroquinoline, 1,2,3,4-tetrahydroquinoline, 1,2-dihydroquinoline, 1,2,3,4-tetrahydroquinazoline, 1,2-dihydroquinazoline, 2,4-dihydro-1H-benzo[d][1,3]oxazine, 2,4-dihydro-1H-benzo[d][1,3]thiazine, 5,6,7,8-tetrahydroisoquinoline, 1,2-dihydroisoquinoline, 1,2,3,4-tetrahydroisoquinoline, 1,2-dihydroquinoxaline, 1,4-dihydroquinoxaline, 1,2,3,4-tetrahydroquinoxaline, 4H-benzo[d][1,3]dioxane, 2,3-dihydrobenzo[b][1,4]dioxane, 1,3-benzodioxoline, 2,3,4,5-tetrahydrobenzo[b][1,4]oxazepine, 2,3,4,5-tetrahydro-1H-benzo[b]azepine, 2,3,4,5-tetrahydro-1H-benzo[b]oxepin, 2,3,4,5-tetrahydro-1H-benzo[b]thiepine, and 6,7,8,9-tetrahydro-5H-cyclohepta[b]pyridine.

In the present specification, unless otherwise specified, the "non-aromatic heterocycle" is preferably one having 3 to 14 ring members, and examples thereof include rings such as aziridine, oxirane, thiirane, azetidine, oxetane, thietane, pyrrolidine, tetrahydrofuran, thiolane, pyrazoline, pyrazolidine, imidazolidine, piperidine, tetrahydropyran, tetrahydrothiopyran, piperazine, morpholine, thiomorpholine, dioxane, oxazoline, isoxazoline, 1,3-oxazolidine, isoxazolidine, thiazoline, isothiazoline, 1,3-thiazolidine, isothiazolidine, oxadiazoline, 1,3,4-oxadiazolidine, quinuclidine, azepan, diazepine, oxepane, and thiepan.

In the compound of the present invention, the ester bond between the linker and the nonsteroidal anti-inflammatory compound is hydrolyzed to release the nonsteroidal anti-inflammatory compound. The rate of hydrolysis of the ester bond changes depending on the surrounding environment. For this reason, in the form of an ester bond, there is a case which makes it possible to provide a longer-term sustained release effect. For example, by introduction or substitution of an electron donating group or a bulky group in the vicinity of linker Z, the rate of hydrolysis may be slowed down, and examples thereof include an alkyl group, particularly a branched alkyl group. On the contrary, by introduction or substitution of an electron-withdrawing group in the vicinity of the linker Z, the rate of hydrolysis may increase, and examples thereof include a haloalkyl group and a halogen atom. As above, introduction of a group into the linker or

introduction of a substituent into the linker can be selected according to the desired hydrolysis rate, that is, the sustained release effect.

More preferably, alginic acid or a salt thereof is bound to the nonsteroidal anti-inflammatory compound with one of the following groups of linkers. Note that, in the linkers below, -NH represents an end forming an amide bond with one residue of alginic acid or a salt thereof, and -O represents an end forming an ester bond with the carboxyl group of a nonsteroidal anti-inflammatory compound.

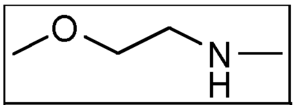
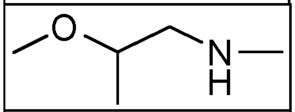
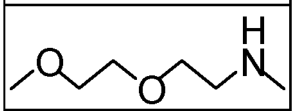
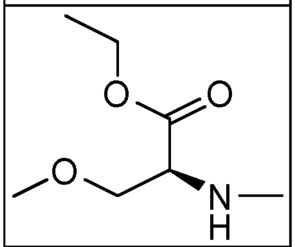
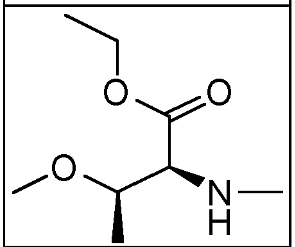
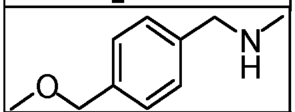
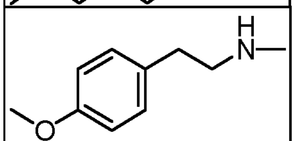
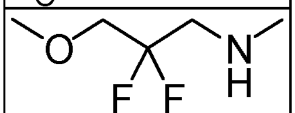
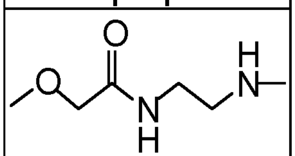
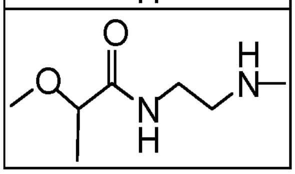

Alternatively, it is preferable that alginic acid or a salt thereof is bound to the nonsteroidal anti-inflammatory compound through any one selected from the group consisting of linkers represented by the following formulas (LK-1) to (LK-17) (excluding the outside of the broken lines in the formula). Note that, in the linkers of the following formulas,

the imino group (-NH) side represents an end forming an amide bond with one residue of alginic acid or a salt thereof, and the -O side represents an end forming an ester bond with the carboxyl group of the nonsteroidal anti-inflammatory compound.

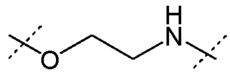 (LK-1)

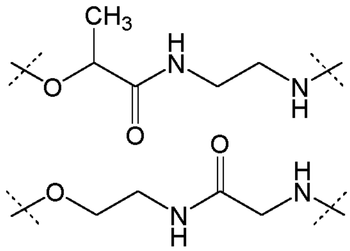 (LK-10)

(LK-11)

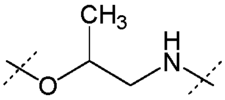 (LK-2)

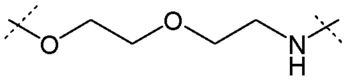 (LK-3)

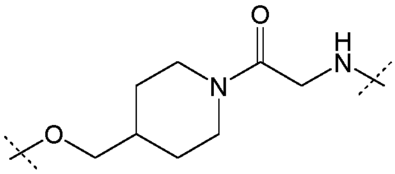 (LK-12)

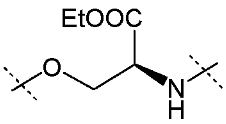 (LK-4)

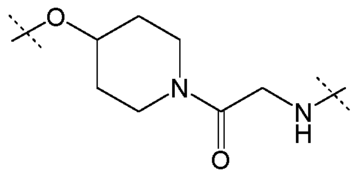 (LK-13)

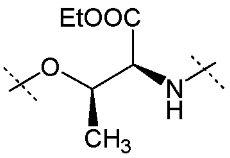 (LK-5)

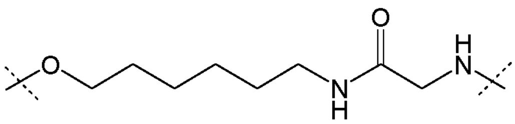 (LK-14)

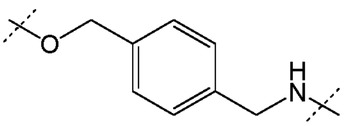 (LK-6)

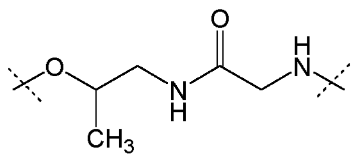 (LK-15)

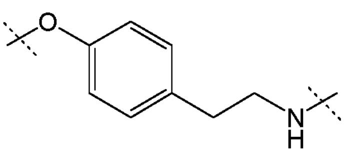 (LK-7)

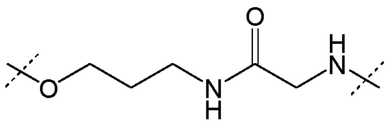 (LK-16)

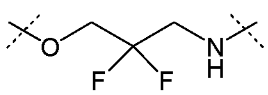 (LK-8)

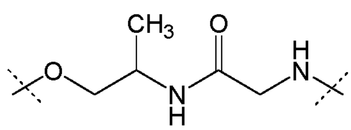 (LK-17)

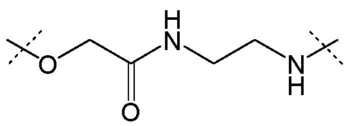 (LK-9)

<Nonsteroidal Anti-Inflammatory Compound>

The nonsteroidal anti-inflammatory compound used is one having a residue derived from nonsteroidal anti-inflammatory drugs (NSAIDs) and having a functional group such as a carboxyl group, a hydroxyl group, or an amino

group in its chemical structure. In addition, the nonsteroidal anti-inflammatory compound may be in the form of salt. The NSAIDs in the present invention are not particularly limited because they generally include all compounds called nonsteroidal anti-inflammatory drugs, but among them, those particularly applicable to arthritis are desirable, and NSAIDs having at least a carboxyl group are particularly preferable from the viewpoint of binding with a linker. Preferably, the nonsteroidal anti-inflammatory compound has a carboxyl group, and the carboxyl group is bound to a linker. Examples of NSAIDs having a carboxyl group include nonsteroidal anti-inflammatory drugs of (1) salicylic acid-based, (2) propionic acid-based or (3) acetic acid-based (phenylacetic acid-based), (4) fenamic acid-based, (5) oxicam-based, (6) pyrrolopyrrole derivatives, (7) coxib-based (COX-2 inhibitors), and the like, and acetic acid-based (phenylacetic acid-based) nonsteroidal anti-inflammatory drugs (NSAIDs) are preferable, and the nonsteroidal anti-inflammatory compound is most preferably diclofenac. In the nonsteroidal anti-inflammatory drugs, (1) salicylic acid-based nonsteroidal anti-inflammatory drugs include salicylic acid, sazapyrine, aspirin, diflunisal, salicylamide, and the like, (2) propionic acid-based nonsteroidal anti-inflammatory drugs include ibuprofen, flurbiprofen, ketoprofen, naproxen, pranoprofen, fenoprofen, tiaprofenic acid, oxaprozin, loxoprofen sodium, alminoprofen, zaltoprofen, tiaprofenic acid, and the like, and (3) aryl acetic acid-based (phenylacetic acid-based) nonsteroidal anti-inflammatory drugs include felbinac, diclofenac, tolmetin sodium, sulindac, fenbufen, indomethacin, indomethacin farnesyl, acemetacin, proglumetacin maleate, amfenac sodium, nabumetone, mofezolac, etodolac, alclofenac, and the like.

In practicing the exemplary embodiments of the present invention, ketoprofen or naproxen belonging to (2) propionic acid-based nonsteroidal anti-inflammatory drugs, or felbinac or diclofenac belonging to (3) aryl acetic acid-based (phenylacetic acid-based) nonsteroidal anti-inflammatory drugs are more preferable, and diclofenac is particularly preferable.

<Method of Synthesizing Water-Soluble Alginic Acid Derivative>

In the synthesis of the water-soluble alginic acid derivative, the linker may be first bonded to the nonsteroidal anti-inflammatory compound, or the linker may be first bonded to alginic acid or a salt thereof. However, it is preferable to first bond the nonsteroidal anti-inflammatory compound to the linker for reasons such as it is difficult to perform esterification in a water solvent. Examples of methods of achieving such a bond include a method using a condensing agent such as DCC (N,N'-dicyclohexylcarbodiimide), EDCI (1-ethyl-3-(3-dimethylaminopropyl)carbodiimide), or DMT-MM (4-(4,6-dimethoxy-1,3,5-triazin-2-yl)-4-methylmorpholinium chloride), a condensation reaction using a condensation auxiliary agent such as HOSu (N-hydroxysuccinimide) or HOBt (1-hydroxybenzotriazole) and the above-described condensation agent, a nucleophilic substitution reaction, an activated ester method, and an acid anhydride method, and bonding using a condensation reaction or a nucleophilic substitution reaction is preferable for reasons of suppressing side reactions and the like.

More specifically, it can be synthesized by a method using a condensation reaction (esterification reaction) or a method using a nucleophilic substitution reaction, as in a scheme showing the following concept, for example. For convenience, in the following reaction scheme, the linker is interpreted as "(O)-Linker-NH," AL is interpreted as a residue derived from alginic acid or the salt thereof which has a C(=O)-group in a monosaccharide of either L-guluronic acid or D-mannuronic acid constituting alginic acid, and Boc is interpreted as a butoxycarbonyl group (protecting group). Then, the outline of the reaction can be understood. Note that although DF is an abbreviation for diclofenac, this is an example and does not mean that NSAIDs are limited to diclofenac in the present invention.

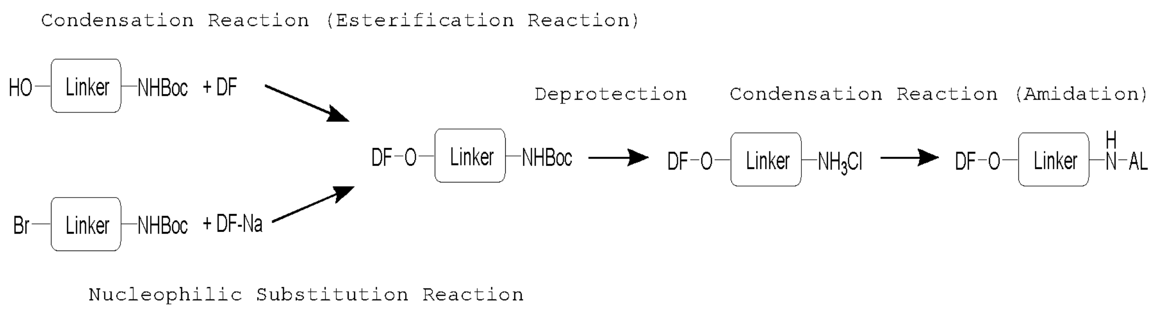


In addition, the introduction rate of the nonsteroidal anti-inflammatory compound in the water-soluble alginic acid derivative of the present invention can be adjusted by changing the amounts charged of the condensing agent, the condensation auxiliary agent, and the linker-bonded nonsteroidal anti-inflammatory compound in the step of synthesizing the water-soluble alginic acid derivative of the present invention. Note that the introduction rate can be measured by a method using absorbance measurement, HPLC, NMR, or the like. It is also possible to appropriately adjust the water solubility of the water-soluble alginic acid derivative depending on the structure of the linker and introduction rate.

<Amino Compound Used for Binding to Alginic Acid or Salt Thereof>

In the synthesis of the alginic acid derivative, the amino compound which is used for bonding with alginic acid or a salt thereof, and which is generated after binding of the linker to the nonsteroidal anti-inflammatory compound is an amino compound represented by the following formula (AM).

An amino compound represented by the formula (AM), a salt thereof, or a solvate thereof:

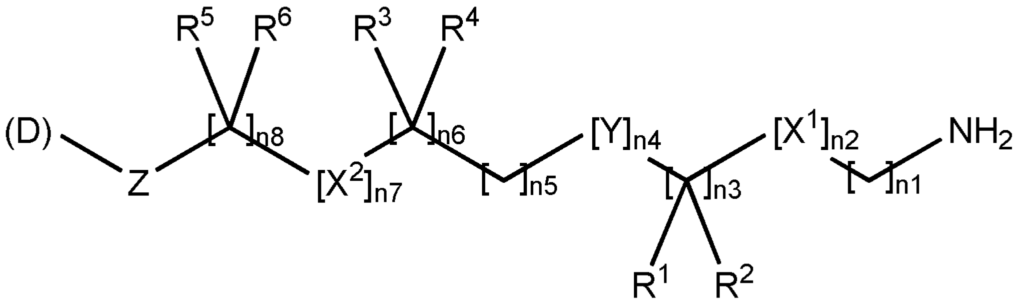 (AM)

(wherein

(D) represents one residue derived from a nonsteroidal anti-inflammatory compound;
$X^1$ and $X^2$ are oxygen atoms or NH (imino groups);
$R^1$, $R^2$, $R^3$, $R^4$, $R^5$, and $R^6$ are each independently a group selected from a hydrogen atom, a halogen atom, a $C_{1-6}$ alkyl group, a $C_{1-6}$ alkoxy group, and a $C_{1-6}$ alkoxycarbonyl group ($R^1$ and $R^2$, $R^3$ and $R^4$, or $R^5$ and $R^6$ can together form an oxo group (=O)), preferably a group selected from a hydrogen atom, a halogen atom, a $C_{1-6}$ alkyl group, and a $C_{1-6}$ alkoxycarbonyl group ($R^1$ and $R^2$ can together form an oxo group (=O)), more preferably a group selected from a hydrogen atom, a halogen atom, a $C_{1-3}$ alkyl group, and a $C_{1-3}$ alkoxycarbonyl group ($R^1$ and $R^2$ can together form an oxo group (=O)), and further preferably a hydrogen atom, fluorine, or a methyl group ($R^1$ and $R^2$ can together form an oxo group (=O));
Y is a heterocycle (the heterocycle may be substituted with 1 to 3 halogen atoms or $C_{1-6}$ alkyl groups, and preferably 1 to 3 halogen atoms or $C_{1-3}$ alkyl groups), and preferably piperidine;
Z is an oxygen atom;
n1 or n8 is any integer of 0 to 10;
n3, n5, or n6 are each independently any integer of 0, 1, 2, or 3;
n2, n4, or n7 are each independently any integer of 0 or 1; and provided that not all of n1 to n8 can be 0).

In addition, in the amino compounds represented by the formula (AM), a preferable one is an amino compound represented by the following formula (AM-1), a salt thereof, or a solvate thereof:

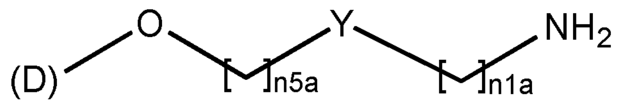 (AM-1)

(wherein

(D) represents one residue of a nonsteroidal anti-inflammatory compound; preferably one residue of diclofenac;
Y is a $C_{6-10}$ aryl ring, a $C_{3-8}$ alkyl ring, or a heterocycle; preferably a benzene ring; and
n1a and n5a are each independently an integer of 1 to 4).

In addition, a specific example of formula (AM-1) is an amino compound selected from the following formulas, a salt thereof, or a solvate thereof.

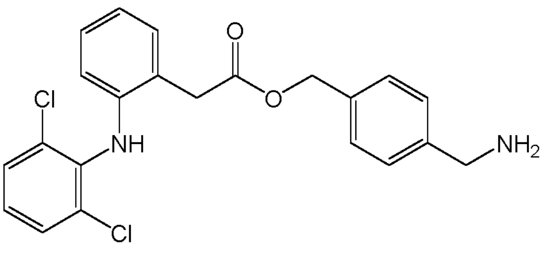

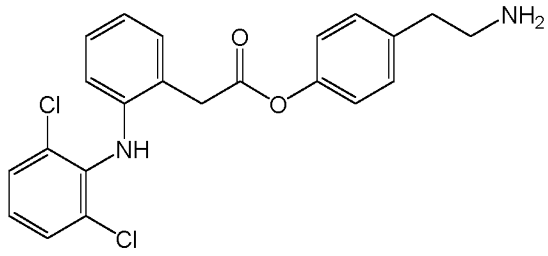

In addition, in the amino compounds represented by the formula (AM), a preferable one is an amino compound represented by the following formula (AM-2), a salt thereof, or a solvate thereof.

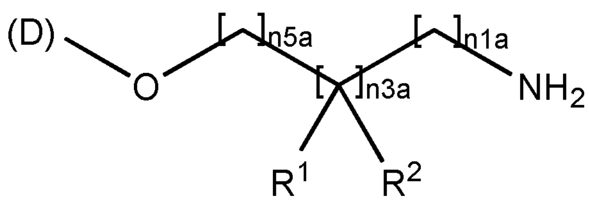

(AM-2)

(wherein,

(D) represents one residue of a nonsteroidal anti-inflammatory compound; preferably one residue of diclofenac;
$R^1$ and $R^2$ are each independently a group selected from a hydrogen atom and a halogen atom; and
n1a, n3a, and n5a are each independently an integer of 1 to 4
(here excluding (αR)-3-benzoyl-α-methyl-phenylacetic acid 3-amino-2-fluoropropyl ester [CAS No. 1644429-26-4], (αR)-3-benzoyl-α-methyl-phenylacetic acid 3-amino-propyl ester [CAS No. 1644429-25-3], 2-fluoro-α-methyl-[1,1'-biphenyl]-4-acetic acid 3-amino-2,2-difluoropropyl ester [CAS No. 1644429-24-2], (αR)-3-benzoyl-α-methyl-phenylacetic acid 3-amino-2,2-difluoropropyl ester [CAS No. 1644429-23-1], [1,1'-biphenyl]-4-acetic acid 3-amino-2,2-difluoropropyl ester [CAS No. 1644429-21-9], (αS)-α-methyl-4-(2-methylpropyl)-phenylacetic acid 3-amino-propyl ester [CAS No. 1384127-03-0], 2-[(2,6-dichlorophenyl)amino]-phenylacetic acid 3-amino-propyl ester [CAS No. 918636-69-8], [1,1'-biphenyl]-4-acetic acid 3-amino-propyl ester [CAS No. 918636-66-5], 2-fluoro-α-methyl-[1,1'-biphenyl]-4-acetic acid 3-amino-propyl ester [CAS No. 918636-63-2], α-methyl-4-(2-methylpropyl)-phenylacetic acid 3-amino-propyl ester [CAS No. 918636-60-9], (αS)-6-methoxy-α-methyl-2-naphthalene acetic acid 3-amino-propyl ester [CAS No. 918636-57-4], salts thereof, or solvates thereof).

In addition, a specific example of the formula (AM-2) is an amino compound of the following formula, a salt thereof, and a solvate thereof.

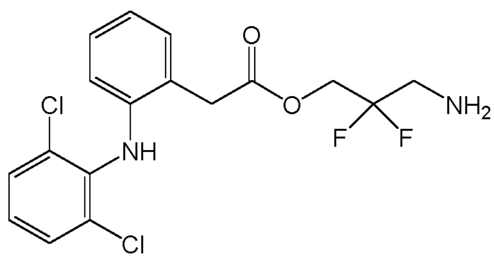

In addition, in the amino compounds represented by the formula (AM), a preferable one is an amino compound represented by the following formula (AM-3), a salt thereof, or a solvate thereof.

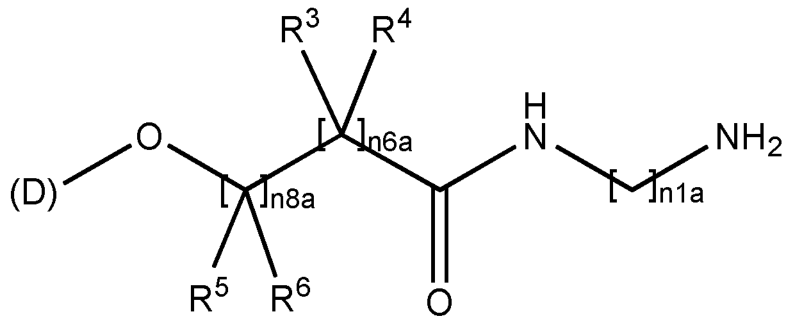

(AM-3)

(wherein

(D) represents one residue of a nonsteroidal anti-inflammatory compound; preferably one residue of diclofenac;
$R^3$, $R^4$, $R^5$, and $R^6$ are each independently a group selected from a hydrogen atom, a halogen atom, and a methyl group; and
n1a, n6a, and n8a are each independently an integer of 1 to 4).

In addition, specific examples of the formula (AM-3) are amino compounds of the following formulas, salts thereof, and solvates thereof.

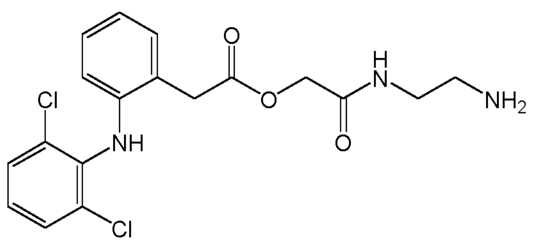

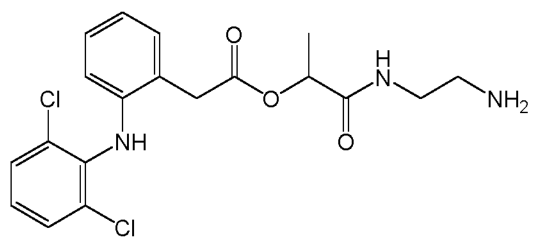

In addition in the amino compounds represented by the formula (AM), a preferable one is an amino compound represented by the following formula (AM-4), a salt thereof, or a solvate thereof.

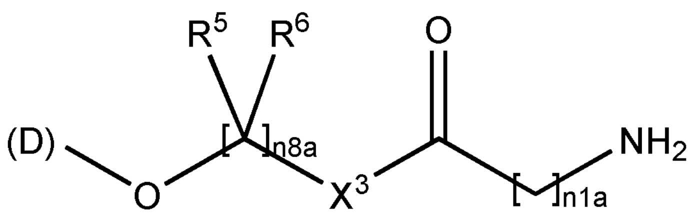

(AM-4)

(wherein

(D) represents one residue of a nonsteroidal anti-inflammatory compound; preferably one residue of a nonsteroidal anti-inflammatory compound selected from diclofenac, ketoprofen, naproxen, and felbinac;
$R^5$ and $R^6$ are each independently a hydrogen atom, a halogen atom, or a methyl group; preferably, $R^5$ is a hydrogen atom, and $R^6$ is a hydrogen atom or a methyl group;
$X^3$ is an imino group (NH), a $C_{3-8}$ cycloalkyl ring, a $C_{6-10}$ aryl ring, or a heterocycle (the $C_{3-8}$ cycloalkyl ring, $C_{6-10}$ aryl ring, or heterocycle may be substituted with 1 to 3 halogen atoms or $C_{1-6}$ alkyl groups), preferably an imino group (NH) or a heterocycle, and more preferably an imino group (NH) or piperidine;
n1a is any integer of 1 to 4; and
n8a is any integer of 0 to 8).

In addition, specific examples of the formula (AM-4) are amino compounds of the following formulas, salts thereof, and solvates thereof.

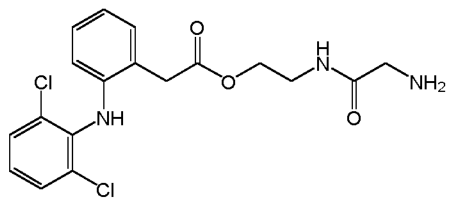

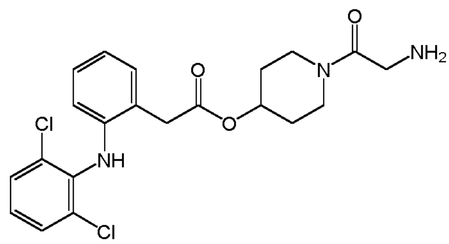

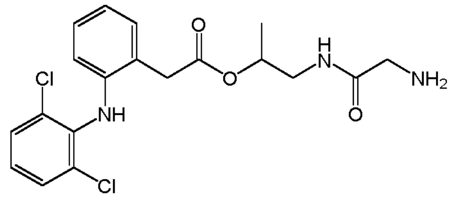

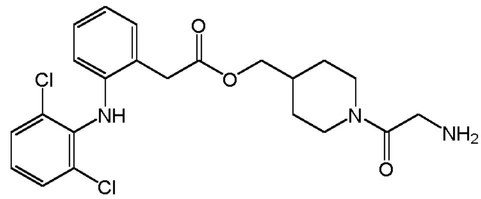

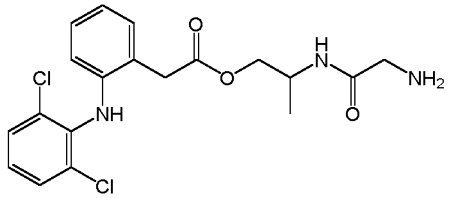

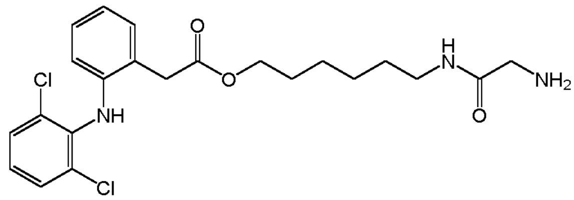

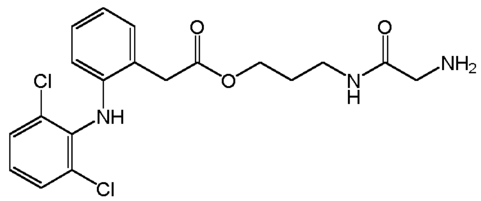

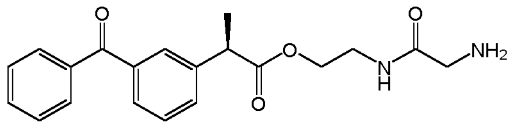

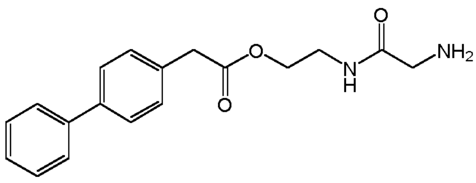

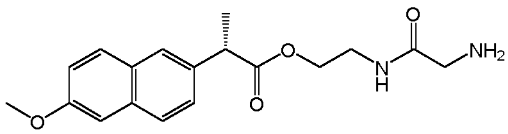

In the present specification, the amino compound represented by formula (AM), formula (AM-1), formula (AM-2), formula (AM-3), or formula (AM-4), or the nonsteroidal anti-inflammatory compound substituted by a basic substituent may form a pharmaceutically acceptable salt (such as an acid addition salt). Such a salt is not particularly limited as long as it is a pharmaceutically acceptable salt, and examples thereof include a salt with an inorganic acid, a salt with an organic acid, and a salt with an acidic amino acid. Preferable examples of the salt with an inorganic acid include salts with hydrochloric acid, hydrobromic acid, hydroiodic acid, nitric acid, sulfuric acid, and phosphoric acid. Preferable examples of the salt with an organic acid include salts with aliphatic monocarboxylic acids such as formic acid, acetic acid, trifluoroacetic acid, propionic acid, butyric acid, valeric acid, enanthic acid, capric acid, myristic acid, palmitic acid, stearic acid, lactic acid, sorbic acid, and mandelic acid, salts with aliphatic dicarboxylic acids such as oxalic acid, malonic acid, succinic acid, fumaric acid, maleic acid, malic acid, and tartaric acid, salts with aliphatic tricarboxylic acids such as citric acid, salts with aromatic monocarboxylic acids such as benzoic acid and salicylic acid, salts of aromatic dicarboxylic acids such as phthalic acid, salts with organic carboxylic acids such as cinnamic acid, glycolic acid, pyruvic acid, oxylic acid, salicylic acid, and N-acetylcysteine, salts with organic sulfonic acids such as methanesulfonic acid, benzenesulfonic acid, and p-toluenesulfonic acid, and acid addition salts with acidic amino acids such as aspartic acid and glutamic acid. Preferable examples of the salt with an acidic amino acid include salts with aspartic acid, glutamic acid, and the like. Among these, pharmaceutically acceptable salts are preferable.

The salt can be obtained by a conventional method, for example by mixing the compound of the present invention with a solution containing an appropriate amount of an acid to form a desired salt, and then performing fractionation filtration or distilling off the mixed solvent. As a review article on salts, Handbook of Pharmaceutical Salts: Properties, Selection, and Use, Stahl & Wermuth (Wiley-VCH, 2002) has been published, and detailed descriptions are

given in this book.

<Alginic Acid Derivative Gel>

The water-soluble alginic acid derivative of the present invention can form an alginic acid derivative gel by being mixed with a substance generally used as a cross-linking agent for alginic acid. Such a cross-linking agent is not particularly limited as long as it can immobilize the surface by cross-linking a solution of a monovalent metal salt of alginic acid, and examples thereof include divalent or higher valent metal ionic compounds such as $Ca^{2+}$, $Mg^{2+}$, $Ba^{2+}$, and $Sr^{2+}$, and cross-linkable reagents having 2 to 4 amino groups in the molecule. More specific examples of divalent or higher metal ion compounds include $CaCl_2$, $MgCl_2$, $CaSO_4$, $BaCl_2$, or the like, and the specific evamples of a cross-linking reagent having 2 to 4 amino groups in the molecule include a diaminoalkane that may have a lysyl group ($-COCH(NH_2)-(CH_2)_4-NH_2$) on the nitrogen atom, that is, a derivative in which a diaminoalkane and an amino group thereof are substituted with a lysyl group to form a lysylamino group. Specific examples thereof include diaminoethane, diaminopropane, and N-(lysyl)-diaminoethane, but a $CaCl_2$ solution is particularly preferable because of its easy availability, gel strength, and the like.

Here, it is known that, when calcium is contained in a cross-linking agent, the higher the concentration of calcium, the faster the gelation and the formation of a harder gel. However, since calcium has cytotoxicity, if the concentration is too high, it may adversely affect the body (core) when administered in the body, and therefore, an appropriate amount should be used according to the amount of alginic acid.

<Sustained-Release Pharmaceutical Composition>

Since the water-soluble alginic acid derivative or the alginic acid derivative gel of the present invention exhibits a behavior of sustained release of a nonsteroidal anti-inflammatory compound *in vivo*, it can be used as a sustained-release pharmaceutical composition. Moreover, in the sustained-release pharmaceutical composition of the present invention, alginic acid or a salt thereof is used as the sustained release base material. Alginic acid or a salt thereof has an effect on wound coating, cartilage disease treatment, and rheumatoid arthritis treatment. For example, it is expected that the effect of cartilage regeneration is exhibited in knee joint deformity, and the therapeutic effect of cartilage regeneration or rheumatoid arthritis itself is exhibited in rheumatoid arthritis. That is, the sustained-release pharmaceutical composition of the present invention is expected to have a combination of analgesic and anti-inflammatory therapeutic effects of sustained release NSAIDs and therapeutic effects of alginic acid. The target disease and administration route of the sustained-release pharmaceutical composition of the present invention are not particularly limited, but the purpose is preferably treatment of arthropathy, suppression of inflammation and suppression of pain, prevention and alleviation of symptoms, and the like, and administration by the route of direct injection into the joint cavity is preferable.

For example, when the sustained-release pharmaceutical composition of the present invention is used as an arthritis therapeutic agent for intra-articular administration of the knee joint, and the pH of the inflamed diseased site exhibits weakly acidic behavior, it is expected that the sustained release of the nonsteroidal anti-inflammatory compound stably continues for 7 days or longer, preferably 15 days or longer, more preferably 30 days or longer, and further preferably 100 days or longer after administration to the diseased site by injection or the like.

In addition, the dose of the sustained-release pharmaceutical composition of the present invention is not particularly limited and individually determined so that the therapeutic effect is most appropriately exhibited depending on the amount of the nonsteroidal anti-inflammatory compound contained, the administration route, the dosage form, the purpose of use, the specific symptoms of the animal to be administered, the age, the weight, and the like. For example, an amount that can maintain a concentration 1/100 to 10 times the working concentration exhibiting the effect of NSAIDs is preferable.

The application site of the sustained-release pharmaceutical composition of the present invention is not particularly limited as long as the site is capable of administration by parenteral administration, and among others, joints are preferable, and knee joints, shoulder joints, hip joints, jaw joints, and the like are particularly preferable. In particular, application to arthritis such as osteoarthritis (OA) and rheumatoid arthritis (RA) is desirable. Moreover, application to kee osteoarthritis (gonarthrosis) and rheumatoid knee arthritis is desirable.

In the present invention, a water-soluble alginic acid derivative may be applied in the diseased site, for example the knee joint cavity. If an appropriate viscosity cannot be maintained after application, a cross-linking agent may be applied to the surface of the derivative by applying it. By gelling the surface of the derivative and hardening the surface, it is possible to effectively prevent leakage from the knee joint cavity.

When the water-soluble alginic acid derivative is administered to the diseased site first and then the cross-linking agent is added later, it is desirable that the cross-linking agent gradually penetrates from the surface of the applied composition to the inside to promote the cross-linking. For the purpose of preventing the strong influence of the cross-linking agent on the contact area with the diseased site, the cross-linking agent is adjusted so that the applied amount

is not excessive. The application amount of the divalent or higher valent metal ion is not particularly limited as long as it is an amount that can solidify the surface of the composition containing the monovalent metal salt of alginic acid.

Before applying the water-soluble alginic acid derivative of the present invention to the diseased site, if the surface is gelled with a cross-linking agent or the derivative is mixed with a cross-linking agent in advance so that the whole is formed into a gel, and then the gel is applied, the water-soluble alginic acid derivative of the present invention is hardened in the diseased site and can be localized in a state of being in close contact with the diseased site of application. This allows components such as cells to be localized in the diseased site when the cells and the like are embedded.

In addition, in the case of using the alginic acid derivative gel in a sustained-release pharmaceutical composition, it is also possible to form a composition that, for example, maintains the liquid state before administration and undergoes self-gelling after administration *in vivo*, and that adjust the concentration of cross-linking agent that promotes gelation by using environmental changes such as time difference, temperature difference, or contact with calcium ions *in vivo.* Examples of such a cross-linking agent include calcium gluconate, $CaSO_4$, calcium alginate, and the like.

In addition, the method of adding a divalent or higher valent metal ion to the pharmaceutical composition containing the water-soluble alginic acid derivative is not particularly limited. For example, it is possible to use a method of applying a solution of divalent or higher valent metal ions to the surface of the composition with a syringe, an injector (spray), or the like. The timing of applying the cross-linking agent to the surface of the composition of the present invention may be after the composition of the present invention is applied to the diseased site, or at the same time.

In addition, the sustained-release pharmaceutical composition containing the alginic acid derivative gel of the present invention may be contained in a form of microbeads having an average particle size of less than 500 μm, for example.

Examples

Next, Examples and Test Examples are given to explain the present invention in further detail, but these examples are merely implementations, are not intended to limit the present invention, and may be modified without departing from the scope of the present invention.

JEOL JNM-ECX400 FT-NMR (JEOL) was used for the measurement of the nuclear magnetic resonance spectrum (NMR).

In the NMR signal pattern in the $^1H$-NMR data, s means singlet, d means doublet, t means triplet, q means quartet, m means multiplet, br means broad, J means coupling constant, Hz means hertz, $CDCl_3$ means deuterated chloroform, and DMSO-$d_6$ means deuterated dimethyl sulfoxide. In the $^1H$-NMR data, signals that cannot be confirmed because they are broadband, such as protons of hydroxyl group (OH), amino group ($NH_2$), and carboxyl group (COOH), are not described in the data.

The introduction rate (mol%) of the drug (nonsteroidal anti-inflammatory compound) in Examples indicates the ratio of the number of moles of the introduced drug to 100 units (moles) of the monosaccharide constituting alginic acid, where the monosaccharide of D-mannuronic acid or L-guluronic acid constituting alginic acid calculated from $^1H$-NMR is defined as 1 unit (mol).

The molecular weight was measured by the following method. The solid of the water-soluble alginic acid derivative according to the present invention obtained in Examples was weighed and added with 10 mmol/L phosphate buffer (pH 7.7), and the mixture was stirred and dissolved at room temperature for 1 hour or more, and then diluted to prepare a 0.05% solution. This solution was passed through a hydrophilic PVDF filter having a pore size of 0.22 μm (Mylex GV 33 Filter, Merck Millipore) to remove insoluble matter, 200 μL of which was then subjected to Superose 6 Increase 10/300 GL Column (GE Healthcare Science) to perform gel filtration. The gel filtration was performed using AKTA Explorer 10S as a chromatographic apparatus and 10 mmol/L phosphate buffer (pH 7.7) as a developing solvent under the conditions of room temperature and a flow rate of 0.8 mL/mim. The chromatogram of each sample was prepared by monitoring the absorbance at a wavelength of 220 nm. The obtained chromatogram was analyzed by Unicorn 5.31 software (GE Healthcare Science) to determine the elution range of the peak.

The molecular weight of the water-soluble alginic acid derivative according to the present invention was determined by the following method. The gel filtration of standard products of blue dextran (molecular weight 2,000,000 Da, SIGMA), thyroglobulin (molecular weight 669,000 Da, GE Healthcare Science), ferritin (molecular weight 440,000 Da, GE Healthcare Science), conalbumin (molecular weight 75,000 Da, GE Healthcare Science), and ribonuclease A (molecular weight 13,700 Da, GE Healthcare Science) were performed under the same conditions as those for the samples, and the amount of liquid eluted for each component was determined. The amount of liquid eluted for each component was plotted on the horizontal axis, and the logarithmic value of the molecular weight was plotted on the vertical axis, and quadratic regression was performed to prepare a calibration curve. This calibration curve was used to calculate the molecular weight (Mi) at the elution time i of the chromatogram of the sample previously obtained. Subsequently, the absorbance at the elution time i was read and denoted by Hi. From these data, the weight average molecular weight

(Mw) was calculated from the following formula.

$$\mathrm{Mw}=\frac{\sum_{i=1}^{\infty}(Hi \times Mi)}{\sum_{i=1}^{\infty} Hi}$$

The molecular weight of the raw material alginic acid or a salt thereof was obtained by the following method. Alginic acid was each weighed in consideration of loss on drying, and ultrapure water was added to prepare a 1% aqueous solution. Next, a 0.05% solution was prepared by diluting with 100 mmol/L phosphate buffer and ultrapure water so that the final concentration was 10 mmol/L phosphate buffer (pH 7.7). The insoluble matter was removed with a hydrophilic PVDF filter having a pore size of 0.22 μm (Mylex GV 33 Filter, Merck Millipore), 200 μL of which was then subjected to gel filtration, and gel filtration was performed under the same conditions as for the water-soluble alginic acid derivative according to the present invention. The detection was carried out by a differential refractometer, and the weight average molecular weight (Mw) was obtained by the same method as for the water-soluble alginic acid derivative according to the present invention.

Note that the "AL" in the scheme of the present examples means a residue derived from alginic acid or the salt thereof which has a C(=O)- group of a monosaccharide of either L-guluronic acid or D-mannuronic acid constituting alginic acid.

(Example 1) Synthesis of Diclofenac-(2-Aminoethanol)-Alginic Acid Derivative

Scheme 1

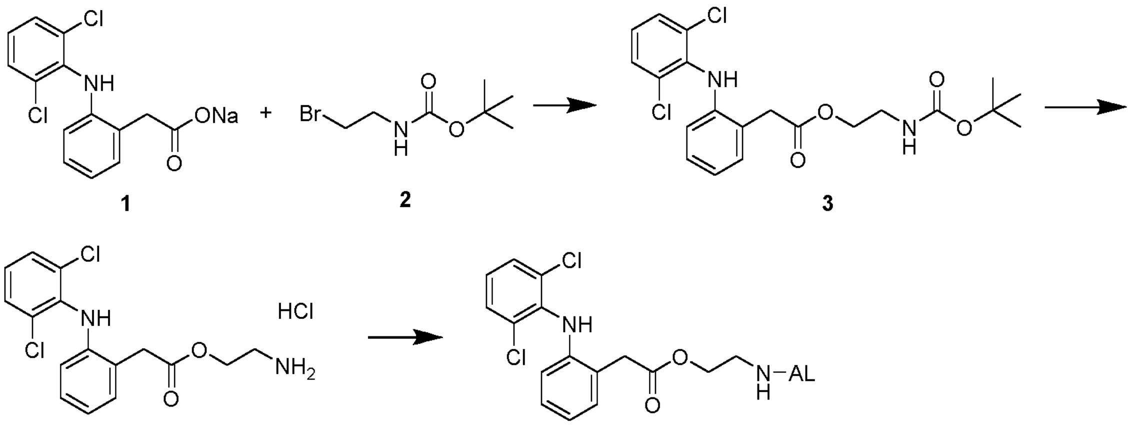

1 2 3

4 5a, 5b, 5c, 5d

<Step 1> Synthesis of Compound 3

A mixture of commercially available sodium diclofenac (compound 1, 1.59 g), commercially available tert-butyl(2-bromoethyl)carbamate (compound 2, 1.12 g), and N-methylpyrrolidone (5.0 mL) was stirred at 60°C for 18 hours. The reaction solution was cooled to room temperature, and then added with ethyl acetate (40 mL) and heptane (20 mL), which was washed successively with saturated sodium hydrogen carbonate aqueous solution (20 mL) and water (20 mL), and dried over anhydrous sodium sulfate, and the solvent was removed under reduced pressure. The residue was purified by crystallization with ethyl acetate to obtain 1.19 g of compound 3. $^{1}$H-NMR (400 MHz, $CDCl_3$) δ 7.34 (2H, d, J = 8 Hz), 7.22 (1H, dd, J = 2, 7 Hz), 7.13 (1H, dt, J = 2, 8 Hz), 7.01-6.92 (2H, m), 6.85 (1H, s), 6.55 (1H, d, 8 Hz)4.72 (1H, br), 4.21 (2H, t, J = 5 Hz), 3.83 (2H, s), 3.45-3.36 (2H, m), 1.43 (9H, s) ppm.

<Step 2> Synthesis of Compound 4

A mixture of compound 3 (1.1 g) and 4 N hydrochloric acid-ethyl acetate solution (11 mL) was stirred at room temperature for 30 minutes. The solvent was removed from the reaction liquid under reduced pressure to obtain 1.0 g of compound 4. $^{1}$H-NMR (400 MHz, DMSO-$d_6$) δ 8.15 (3H, br), 7.53 (2H, d, J = 8 Hz), 7.25-7.16 (2H, m), 7.07-7.01 (2H,

m), 6.85-6.79 (1H, m), 6.20 (1H, d, J = 8 Hz), 4.27 (2H, t, J = 5 Hz), 3.85 (2H, s), 3.09 (2H, t, J = 5 Hz) ppm.

<Step 3-1> Synthesis of Diclofenac-(2-Aminoethanol)-Alginic Acid Derivative (Compound 5a)

In water (20 mL), 200 mg of sodium alginate (manufactured by KIMICA, A1) was dissolved, 4-(4,6-dimethoxy-1,3,5-triazin-2-yl)-4-methylmorpholinium chloride (102 mg) and 1 M sodium hydrogen carbonate aqueous solution (0.28 mL) were added, and an ethanol (5 mL) solution of compound 4 (70 mg) was added dropwise, which was stirred at room temperature for 20 hours. After adding ethanol (40 mL), 0.1 g/mL sodium chloride aqueous solution (2 mL) was added, and the mixture was stirred for 10 minutes. The obtained precipitate was collected by filtration, washed with ethanol, and dried under reduced pressure to obtain the title compound (227 mg) as a white solid. The drug introduction rate was 10.2 mol%.

<Step 3-2> Synthesis of Diclofenac-(2-Aminoethanol)-Alginic Acid Derivative (Compound 5b)

Using 200 mg of sodium alginate (manufactured by KIMICA, A2), the same operation as in (Example 1) <Step 3-1> was performed to obtain the title compound (194 mg) as a white solid. The drug introduction rate was 13.5 mol%.

<Step 3-3> Synthesis of Diclofenac-(2-Aminoethanol)-Alginic Acid Derivative (Compound 5c)

Using 200 mg of sodium alginate (manufactured by KIMICA, A3), the same operation as in (Example 1) <Step 3-1> was performed to obtain the title compound (245 mg) as a white solid. The drug introduction rate was 13.9 mol%.

<Step 3-4> Synthesis of Diclofenac-(2-Aminoethanol)-Alginic Acid Derivative (Compound 5d)

Using 200 mg of sodium alginate (manufactured by KIMICA, A4), the same operation as in (Example 1) <Step 3-1> was performed to obtain the title compound (239 mg) as a white solid. The drug introduction rate was 9.6 mol%.

(Example 2) Synthesis of Diclofenac-(1-Amino-2-Propanol)-Alginic Acid Derivative

Scheme 2

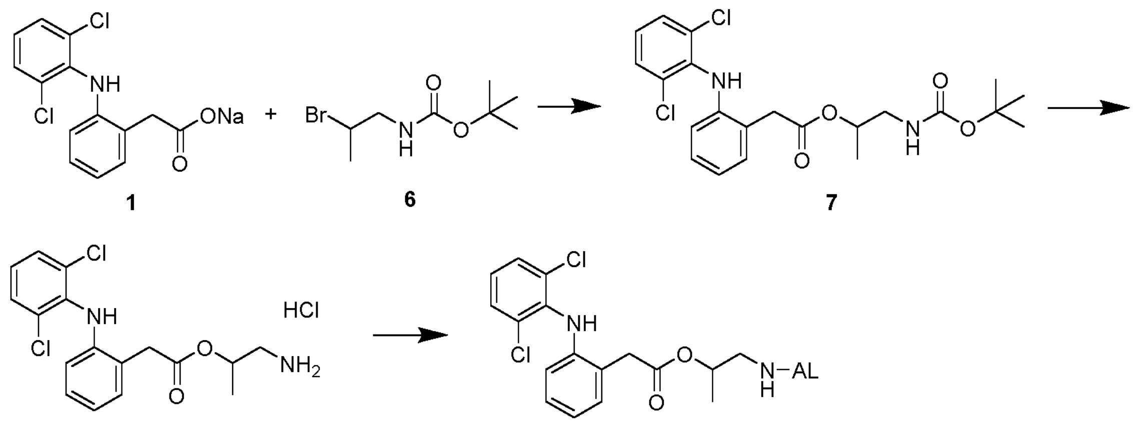

1 6 7

8 9

<Step 1> Synthesis of Compound 7

A mixture of commercially available sodium diclofenac (compound 1, 668 mg), commercially available tert-butyl(2-bromopropyl)carbamate (compound 6, 500 mg), and N-methylpyrrolidone (5.0 mL) was stirred at 60°C for 2 days. The compound 2 (500 mg) was further added, and the mixture was stirred at 60°C for 1 day. The reaction solution was cooled to room temperature, and then added with ethyl acetate (40 mL) and heptane (20 mL), which was washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (10-15% ethyl acetate/heptane) to obtain compound 7 (343 mg) as a colorless gum-like matter.

$^1$H-NMR (400 MHz, $CDCl_3$) δ 7.34 (2H, d, J = 8 Hz), 7.22 (1H, dd, J = 1, 8 Hz), 7.12 (1H, dt, J = 1, 8 Hz), 7.01-6.92 (2H, m), 6.84 (1H, br), 6.55 (1H, d, J = 8 Hz), 5.06-4.92 (1H, m), 4.66-4.57 (1H, m), 3.82 (1H, d, J = 15 Hz), 3.78 (1H, d, J = 15 Hz), 3.42-3.16 (2H, m), 1.40 (9H, s), 1.25 (3H, d, J = 6 Hz) ppm.

<Step 2> Synthesis of Compound 8

A mixture of compound 7 (334 mg) and 4 N hydrochloric acid-ethyl acetate solution (3 mL) was stirred at room temperature for 1 hour. The solvent was removed from the reaction liquid under reduced pressure, and the residue was purified by crystallization from ethyl acetate to obtain compound 8 (190 mg) as a white solid. $^1$H-NMR (400 MHz, DMSO-$d_6$) δ 8.06 (3H, s), 7.55 (2H, d, J = 8 Hz), 7.26-7.18 (2H, m), 7.06 (1H, dt, J = 2, 7 Hz), 6.99 (1H, s), 6.84 (1H, dt, J = 1, 7 Hz), 6.23 (1H, d, J = 8 Hz), 5.09-4.98 (1H, m), 3.86 (1H, d, J = 16 Hz), 3.82 (1H, d, J = 16 Hz), 3.11-2.95 (2H, m), 1.22 (3H, d, J = 6 Hz) ppm.

<Step 3> Synthesis of Diclofenac-(1-Amino-2-Propanol)-Alginic Acid Derivative (Compound 9)

To a 1% (w/w) aqueous solution (10 g) of sodium alginate (manufactured by KIMICA, A2), 4-(4,6-dimethoxy-1,3,5-triazin-2-yl)-4-methylmorpholinium chloride (49 mg) and 1 M sodium hydrogen carbonate aqueous solution (0.13 mL) were added, and an ethanol (5 mL) solution of compound 8 (52 mg) was added dropwise, which was stirred at room temperature for 16 hours. After adding ethanol (15 mL), 0.1 g/mL sodium chloride aqueous solution (1 mL) was added, and the mixture was stirred for 30 minutes. The obtained precipitate was collected by filtration, washed with ethanol, and dried under reduced pressure to obtain the title compound (111 mg) as a white solid. The drug introduction rate was 9.9 mol%.

(Example 3) Synthesis of Diclofenac-(2-Aminoethoxyethanol)-Alginic Acid Derivative

Scheme 3

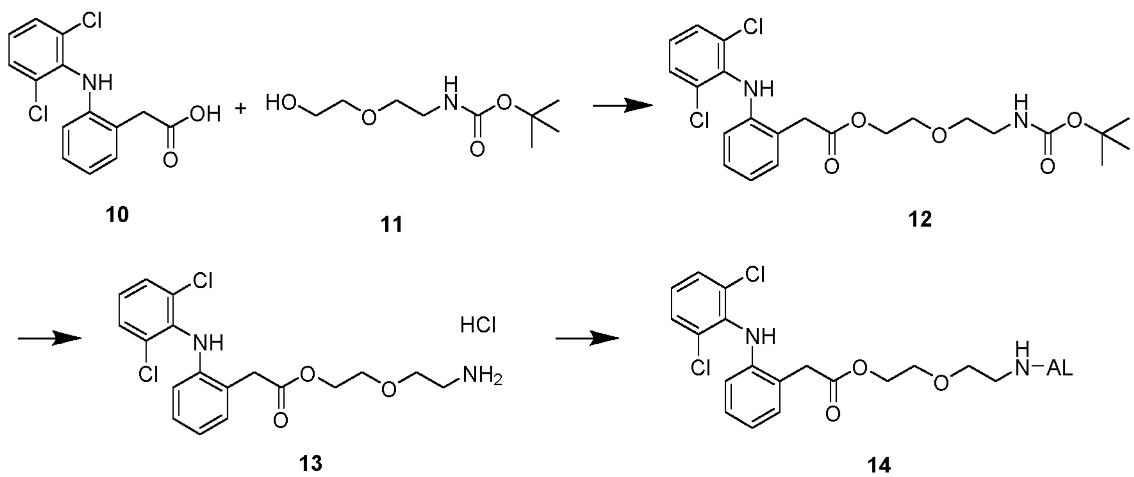

10 11 12

13 14

<Step 1> Synthesis of Compound 12

To a mixture of commercially available diclofenac (compound 10, 2.0 g), commercially available tert-butyl(2-(2-hydroxyethoxy)ethyl)carbamate (compound 11, 1.39 g), N,N-dimethyl-4-aminopyridine (0.17 g), and dichloromethane (7 mL), a dichloromethane (7 mL) solution of N,N'-dicyclohexylcarbodiimide (1.39 g) was added dropwise under ice cooling. The reaction liquid was stirred at room temperature for 2 hours. The reaction liquid was filtered using ethyl acetate (60 mL), then washed successively with saturated sodium hydrogen carbonate aqueous solution (20 mL) and water (20 mL), and dried over anhydrous sodium sulfate, and the solvent was distilled off under reduced pressure. The residue was purified by silica gel column chromatography (10-50% ethyl acetate/heptane) to obtain compound 12 (2.44 g) as a colorless gum-like matter.
$^1$H-NMR (400 MHz, $CDCl_3$) δ 7.34 (2H, d, J = 8 Hz), 7.24 (1H, dd, J = 1, 8 Hz), 7.12 (1H, dt, J = 1, 8 Hz), 7.01-6.93 (2H,

m), 6.89 (1H, s), 6.55 (1H, d, J = 8 Hz), 4.86 (1H, br), 4.32-4.27 (2H, m), 3.85 (2H, s), 3.69-3.64 (2H, m), 3.48 (2H, t, J = 5 Hz), 3.31-3.21 (2H, m), 1.44 (9H, s) ppm.

<Step 2> Synthesis of Compound 13

A mixture of compound 12 (2.4 g) and 4 N hydrochloric acid-ethyl acetate solution (24 mL) was stirred at room temperature for 1 hour. The solvent was removed from the reaction liquid under reduced pressure, and the residue was purified by crystallization from ethyl acetate to obtain compound 13 (1.9 g) as a white solid.
$^1$H-NMR (400 MHz, DMSO-$d_6$) $\delta$ 7.97 (3H, br), 7.53 (2H, d, J = 8 Hz), 7.24-7.18 (2H, m), 7.10-7.03 (2H, m), 6.87-6.83 (1H, dt, J = 1, 7 Hz), 6.26 (1H, d, J = 8 Hz), 4.26-4.20 (2H, m), 3.83 (2H, s), 3.70-3.65 (2H, m), 3.60 (2H, t, J = 5 Hz), 2.93 (2H, t, J = 5 Hz) ppm.

<Step 3> Synthesis of Diclofenac-(2-Aminoethoxyethanol)-Alginic Acid Derivative (Compound 14)

To a 1% (w/w) aqueous solution (10 g) of sodium alginate (manufactured by KIMICA, A2), 1 M sodium hydrogen carbonate aqueous solution (0.13 mL), compound 13 (56 mg), ethanol (5 mL), and 4-(4,6-dimethoxy-1,3,5-triazin-2-yl)-4-methylmorpholinium chloride (37 mg) were added, which was stirred at room temperature overnight. After adding ethanol (15 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (91.3 mg) as a white solid. The drug introduction rate was 7.0 mol%.

(Example 4) Synthesis of Diclofenac-(Serine Ethyl Ester)-Alginic Acid Derivative

Scheme 4

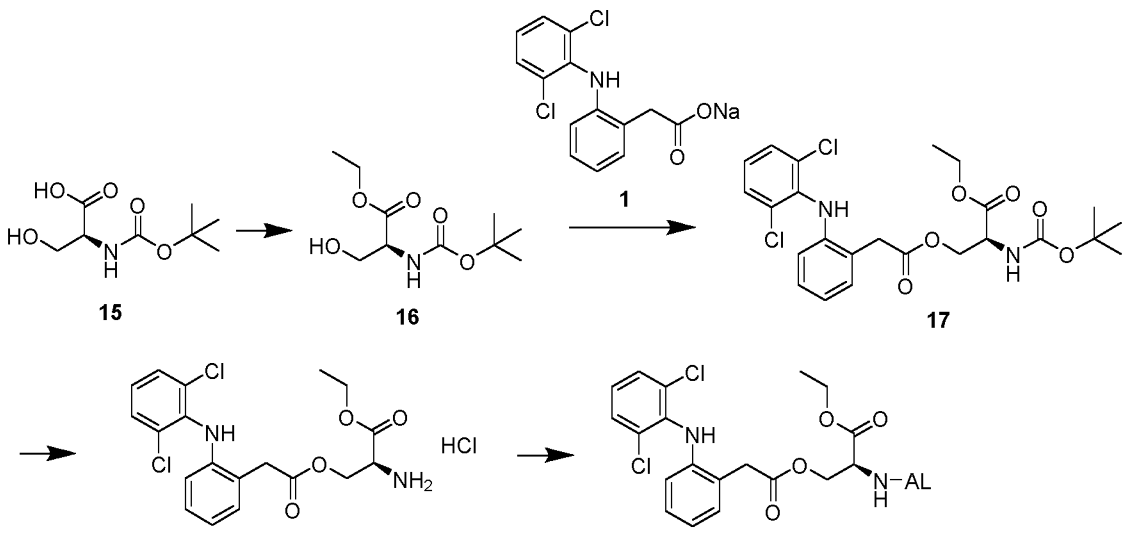

15 16 1 17 18 19

<Step 1> Synthesis of Compound 18

To an ethanol (100 mL) solution of commercially available (tert-butoxycarbonyl)-L-serine (compound 15, 2.0 g), 1-(3-dimethylaminopropyl)-3-ethylcarbodiimide hydrochloride (3.74 g) and N,N-dimethyl-4-aminopyridine (0.12 g) were added at room temperature. The reaction liquid was stirred at room temperature for 3 days. The solvent was removed from the reaction liquid under reduced pressure, which was dissolved in ethyl acetate (100 mL), and its solution was washed successively with a 5% citric acid aqueous solution (50 mL), a saturated sodium hydrogen carbonate aqueous solution (50 mL), and a saturated sodium chloride aqueous solution (50 mL), and dried over anhydrous sodium sulfate, and the solvent was removed under reduced pressure to obtain a crude product of compound 16 (1.08 g).

To an N-methylpyrrolidone (9 mL) solution of the crude product of compound 16 (1.08 g) and commercially available sodium diclofenac (compound 1, 2.95 g), 1-(3-dimethylaminopropyl)-3-ethylcarbodiimide hydrochloride (2.66 g) and N,N-dimethyl-4-aminopyridine (0.11 g) were added at room temperature. The reaction liquid was stirred at room

temperature for 2 days. A saturated sodium hydrogen carbonate aqueous solution (20 mL) and water (20 mL) were added to the reaction liquid, and the mixture was extracted with ethyl acetate (100 mL). The extract liquid was washed with water (20 mL), and dried over anhydrous sodium sulfate, and the solvent was removed under reduced pressure. The residue was purified by silica gel column chromatography (20% ethyl acetate/heptane) to obtain a fraction containing compound 17 (1.09 g).

A mixture of the fraction containing compound 17 (1.09 g) and a 4 N hydrochloric acid-ethyl acetate solution (10 mL) was stirred at room temperature for 1 hour. The solvent was removed from the reaction liquid under reduced pressure, and the residue was purified by crystallization from ethyl acetate to obtain compound 18 (91 mg) as a white solid.

$^1$H-NMR (400 MHz, DMSO-$d_6$) δ 8.61 (3H, br), 7.54 (2H, d, J = 8 Hz), 7.25-7.15 (2H, m), 7.06 (1H, dt, J = 1, 8 Hz), 6.96 (1H, s), 6.84 (1H, dt, J = 1, 7 Hz), 6.23 (1H, d, J = 8 Hz), 4.55-4.22 (3H, m), 4.23-4.14 (2H, m), 3.87 (1H, d, H = 16 Hz), 3.82 (2H, d, J = 16 Hz), 1.20 (3H, t, J = 7 Hz) ppm.

<Step 2> Synthesis of Diclofenac-(Serine Ethyl Ester)-Alginic Acid Derivative (Compound 19)

To a 1% (w/w) aqueous solution (10 g) of sodium alginate (manufactured by KIMICA, A2), 1 M sodium hydrogen carbonate aqueous solution (84 μL), an ethanol (5 mL) solution of compound 18 (30 mg), and 4-(4,6-dimethoxy-1,3,5-triazin-2-yl)-4-methylmorpholinium chloride (37 mg) were added, which was stirred at room temperature for 3 days. After adding a 0.1 g/mL sodium chloride aqueous solution (1 mL) and ethanol (15 mL), the mixture was stirred for 30 minutes. The obtained precipitate was collected by filtration, washed with ethanol, and dried under reduced pressure to obtain the title compound (61.9 mg) as a white solid. The drug introduction rate was 13.1 mol%.

(Example 5) Synthesis of Diclofenac-(Threonine Ethyl Ester)-Alginic Acid Derivative

Scheme 5

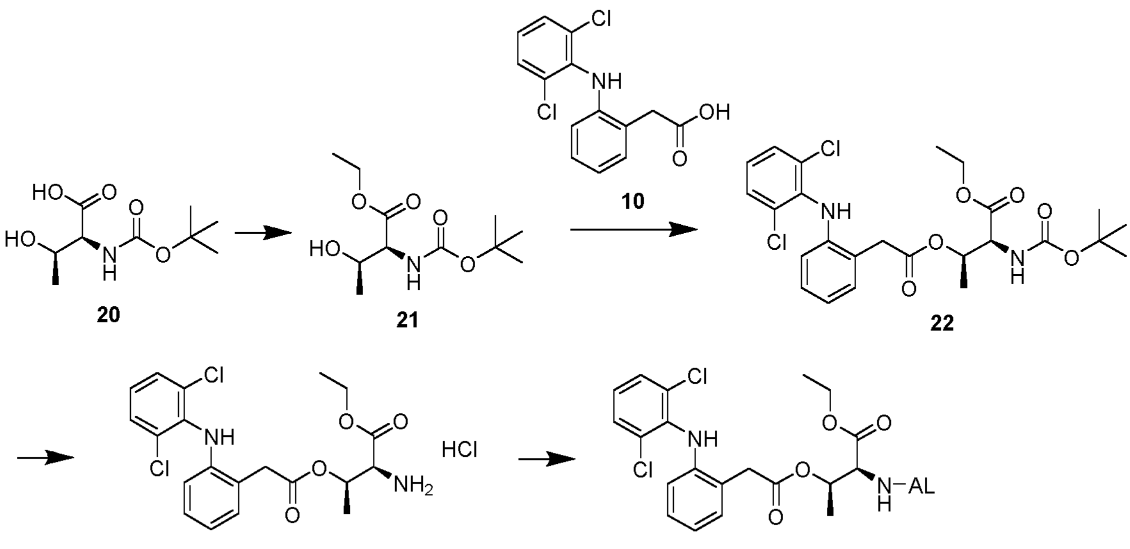

20 21 10 22

23 24

<Step 1> Synthesis of Compound 22

To an ethanol (100 mL) solution of commercially available (tert-butoxycarbonyl)-L-threonine (compound 20, 2.14 g), 1-(3-dimethylaminopropyl)-3-ethylcarbodiimide hydrochloride (3.74 g) and N,N-dimethyl-4-aminopyridine (0.12 g) were added at room temperature. The reaction liquid was stirred overnight. The solvent was removed from the reaction liquid under reduced pressure, which was dissolved in ethyl acetate (100 mL), and its solution was washed successively with a 5% citric acid aqueous solution (50 mL), a saturated sodium hydrogen carbonate aqueous solution (50 mL), and a saturated sodium chloride aqueous solution (50 mL), and dried over anhydrous sodium sulfate, and the solvent was removed under reduced pressure to obtain a crude product of compound 21 (0.96 g).

To a dichloromethane (4 mL) solution of the crude product of compound 21 (0.96 g), commercially available diclofenac (compound 10, 1.15 g), and N,N-dimethyl-4-aminopyridine (0.09 g), a dichloromethane (4 mL) solution of

N,N'-dicyclohexylcarbodiimide (0.8 g) was added dropwise. The reaction liquid was stirred at room temperature for 2 hours. The reaction liquid was filtered using ethyl acetate (60 mL), then washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (15% ethyl acetate/heptane) to obtain compound 22 (0.96 g) as a colorless oil-like matter.
$^1$H-NMR (400 MHz, $CDCl_3$) δ 7.33 (2H, d, J = 8 Hz), 7.17 (1H, dd, J = 1, 7 Hz), 7.11 (1H, dt, J = 1, 8 Hz), 7.00-6.92 (2H, m), 6.82 (1H, s), 6.54 (1H, d, J = 8 Hz), 5.41-5.43 (1H, m), 5.23 (1H, d, J = 10 Hz), 4.43 (1H, dd, J = 3, 10 Hz), 4.07-3.91 (2H, m), 3.78 (1H, d, J = 14 Hz), 3.72 (1H, d, J = 14 Hz), 1.47 (9H, s), 1.33 (3H, d, J = 6 Hz), 1.11 (3H, t, J = 7 Hz) ppm.

<Step 2> Synthesis of Compound 23

A mixture of compound 22 (0.96 g) and 4 N hydrochloric acid-ethyl acetate solution (5 mL) was stirred at room temperature for 1 hour. The solvent was removed from the reaction liquid under reduced pressure, and the residue was purified by crystallization from ethyl acetate to obtain compound 23 (0.41 g) as a white solid.
$^1$H-NMR (400 MHz, DMSO-$d_6$) δ 8.77 (3H, br), 7.54 (2H, d, J = 8 Hz), 7.22 (1H, t, J = 8 Hz), 7.17 (1H, dd, J = 1, 7 Hz), 7.07 (1H, dt, J = 1, 8 Hz), 6.94 (1H, s), 6.85 (1H, dt, J = 1, 7 Hz), 6.24 (1H, d, J = 8 Hz), 5.35-5.27 (1H, m), 4.35 (1H, d, J = 4 Hz), 4.12 (2H, q, J = 7 Hz), 3.86 (1H, d, J = 16 Hz), 3.78 (1H, d, J = 16 Hz), 1.36 (3H, d, J = 7 Hz), 1.15 (3H, t, J = 7 Hz) ppm.

<Step 3> Synthesis of Diclofenac-(Threonine Ethyl Ester)-Alginic Acid Derivative (Compound 24)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (37 mg), 2-morpholinoethanesulfonic acid monohydrate (213 mg), and compound 23 (31 mg) was added. A 0.1 M sodium hydroxide aqueous solution (0.2 mL) was added 2 hours later, 4-(4,6-dimethoxy-1,3,5-triazin-2-yl)-4-methylmorpholinium chloride (16 mg) was added 3 hours later, a 0.1 M sodium hydroxide aqueous solution (0.1 mL) was added 4 hours and 5 hours later, and 4-(4,6-dimethoxy-1,3,5-triazin-2-yl)-4-methylmorpholinium chloride (23 mg) and a 0.1 M sodium hydroxide aqueous solution (0.2 mL) were added 6 hours later. After 24 hours, ethanol (15 mL) and 0.1 g/mL sodium chloride aqueous solution (1 mL) were added, and the mixture was stirred for 10 minutes. The obtained precipitate was collected by filtration, washed with ethanol, and dried under reduced pressure to obtain the title compound (111.2 mg) as a white solid. The drug introduction rate was 5.6 mol%.

(Example 6) Synthesis of Diclofenac-((4-(Aminomethyl)Phenyl)Methanol)-Alginic Acid Derivative

Scheme 6

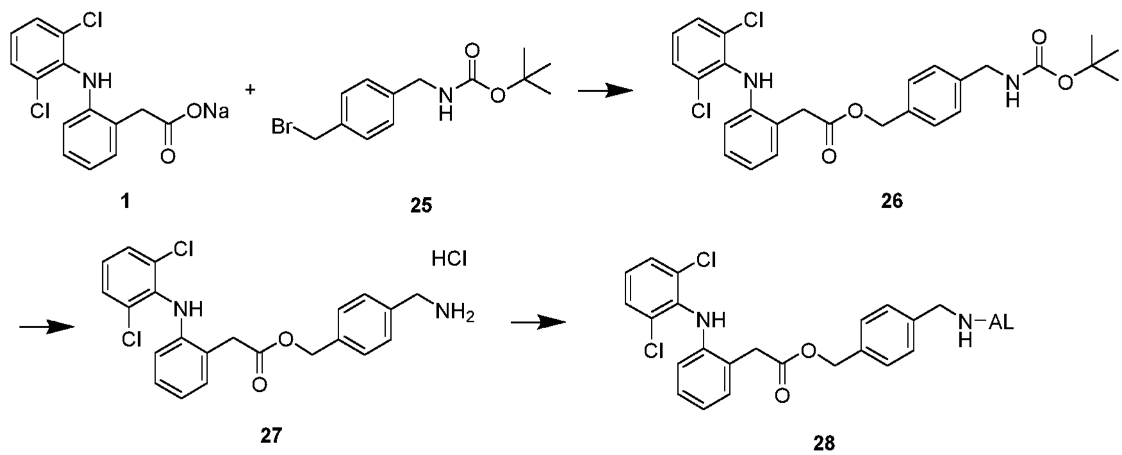

1 25 26

27 28

<Step 1> Synthesis of Compound 26

A mixture of commercially available sodium diclofenac (compound 1, 0.48 g), commercially available tert-butyl(4-(bromomethyl)benzyl)carbamate (compound 25, 0.45 g), and N-methylpyrrolidone (3.0 mL) was stirred at 40°C for 2 hours. The reaction solution was cooled to room temperature, and then added with ethyl acetate (40 mL) and heptane (20 mL), which was washed twice with water (20 mL), and dried over anhydrous sodium sulfate, and the solvent was removed under reduced pressure. The residue was purified by silica gel column chromatography (15% ethyl acetate/heptane) to obtain compound 26 (0.75 g) as a white solid.
$^1$H-NMR (400 MHz, $CDCl_3$) δ 7.33-7.21 (7H, m), 7.12 (1H, dt, J = 1, 8 Hz), 7.00-6.92 (2H, m), 6.86 (1H, s), 6.55 (1H, d, J = 8 Hz), 5.15 (2H, s), 4.81 (1H, br), 4.30 (2H, d, J = 6 Hz), 3.85 (2H, s), 1.46 (9H, s) ppm.

<Step 2> Synthesis of Compound 27

A mixture of compound 26 (0.75 g) and 4 N hydrochloric acid-ethyl acetate solution (20 mL) was stirred at room temperature for 1 hour. The reaction suspension was filtered, and the solid collected by filtration was washed with ethyl acetate to obtain compound 27 (0.36 g) as a white solid.
$^1$H-NMR (400 MHz, DMSO-$d_6$) δ 7.53 (2H, d, J = 8 Hz), 7.45-7.36 (4H, m), 7.23-7.18 (2H, m), 7.10-7.03 (2H, m), 6.84 (1H, dt, J = 1, 7 Hz), 6.25 (1H, d, J = 8 Hz), 5.16 (2H, s), 4.00 (2H, s), 3.88 (2H, s) ppm.

<Step 3> Synthesis of Diclofenac-((4-(Aminomethyl)Phenyl)Methanol)-Alginic Acid Derivative (Compound 28)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (49 mg), 1 M sodium hydrogen carbonate aqueous solution (0.16 mL), and compound 27 (60 mg) was added, and the mixture was stirred at room temperature overnight. After adding ethanol (20 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (88.8 mg) as a white solid. The drug introduction rate was 5.3 mol%.

(Example 7) Synthesis of Diclofenac-(Tyramine)-Alginic Acid Derivative

Scheme 7

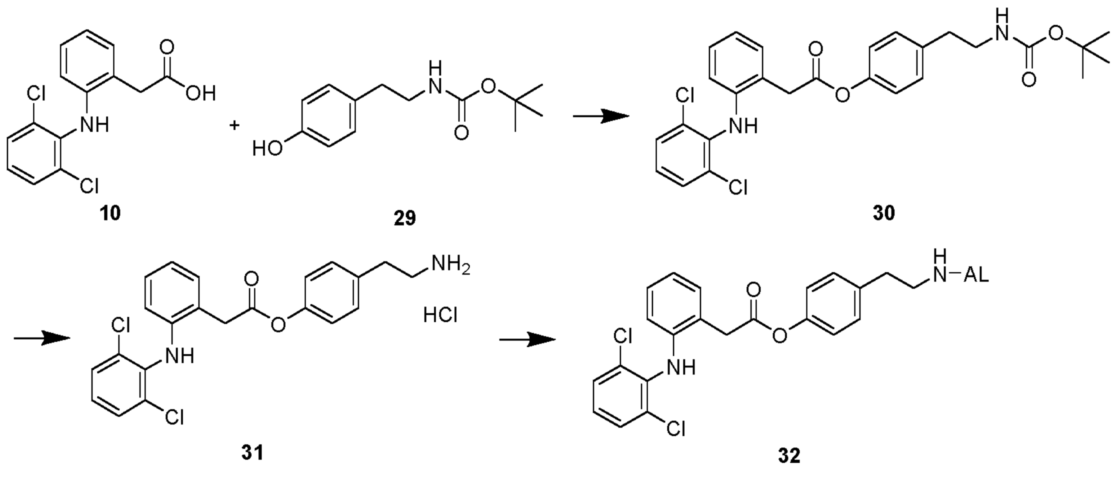

10 29 30 31 32

<Step 1> Synthesis of Compound 30

To a mixture of commercially available diclofenac (compound 10, 0.7 g), commercially available N-(tert-butoxycarbonyl)tyramine (compound 29, 0.56 g), N,N-dimethyl-4-aminopyridine (0.06 g), and dichloromethane (2.5 mL), a dichloromethane (2.5 mL) solution of N,N'-dicyclohexylcarbodiimide (0.49 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (15 mL) and water (15 mL), and dried over anhydrous sodium sulfate, and the solvent was removed under reduced pressure. The residue was

purified by crystallization from ethyl acetate to obtain compound 30 (0.64g) as a white solid.
$^1$H-NMR (400 MHz, $CDCl_3$) δ 7.36-7.30 (3H, m), 7.20-7.13 (3H, m), 7.05-6.95 (4H, m), 6.77 (1H, s), 6.58 (1H, d, J = 8 Hz), 4.60-4.46 (1H, m), 4.04 (2H, s), 3.40-3.30 (2H, m), 2.78 (2H, t, J = 7 Hz), 1.43 (9H, s) ppm.

<Step 2> Synthesis of Compound 31

A mixture of compound 30 (0.64 g) and 4 N hydrochloric acid-ethyl acetate solution (7 mL) was stirred at room temperature for 1 hour. The solvent was removed from the reaction liquid under reduced pressure, and the residue was washed with ethyl acetate-heptane (2:1) to obtain compound 31 (0.56 g) as a white solid.
$^1$H-NMR (400 MHz, DMSO-$d_6$) δ 7.91 (3H, br), 7.54 (2H, d, J = 8 Hz), 7.33-7.27 (3H, m), 7.25-7.21 (1H, m), 7.15-7.05 (4H, m), 6.86 (1H, dt, J = 1, 7 Hz), 6.24 (1H, d, J = 8 Hz), 4.09 (2H, s), 3.08-3.00 (2H, m), 2.91-2.83 (2H, m) ppm.

<Step 3> Synthesis of Diclofenac-(Tyramine)-Alginic Acid Derivative (Compound 32)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (37 mg), 1 M sodium hydrogen carbonate aqueous solution (0.11 mL), and compound 31 (40 mg) was added, and the mixture was stirred at room temperature overnight. After adding ethanol (20 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (81.0 mg) as a white solid. The drug introduction rate was 5.0 mol%.

(Example 8) Synthesis of Diclofenac-(3-Amino-2,2-Difluoropropan-1-Ol)-Alginic Acid Derivative

Scheme 8

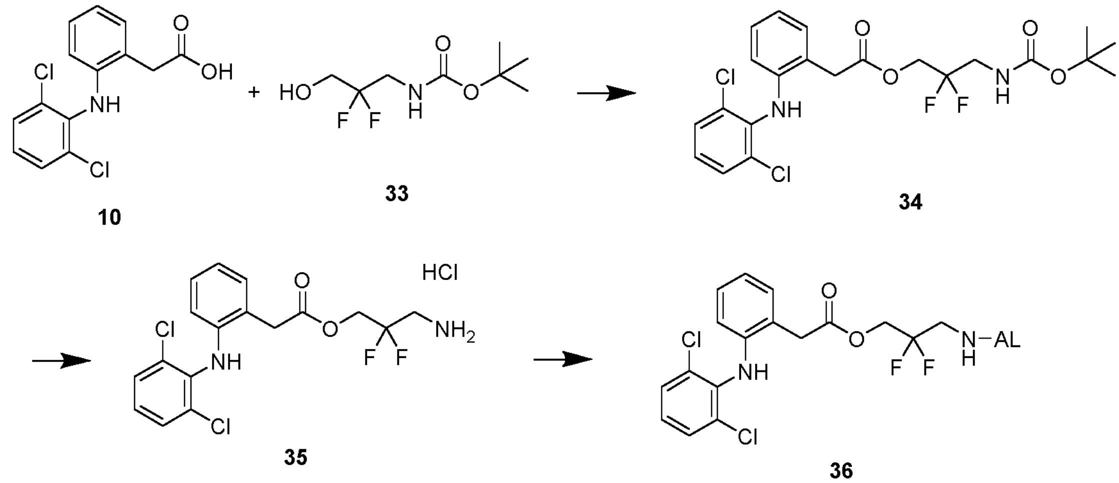

10 33 34 35 36

<Step 1> Synthesis of Compound 3

To a mixture of commercially available diclofenac (compound 10, 0.7 g), commercially available tert-butyl(2,2-difluoro-3-hydroxypropyl)carbamate (compound 33, 0.5 g), N,N-dimethyl-4-aminopyridine (0.06 g), and dichloromethane (2.5 mL), a dichloromethane (2.5 mL) solution of N,N'-dicyclohexylcarbodiimide (0.49 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (15 mL) and water (15 mL), and dried over anhydrous sodium sulfate, and the solvent was removed under reduced pressure. The residue was purified by silica gel column chromatography (5-30% ethyl acetate/heptane) to obtain compound 34 (1.02 g) as a colorless gum-like matter.
$^1$H-NMR (400 MHz, $CDCl_3$) δ 7.33 (2H, d, J = 8 Hz), 7.27-7.22 (1H, m), 7.13 (1H, dt, J = 1, 8 Hz), 7.01-6.94 (2H, m), 6.72 (1H, s), 6.56 (1H, d, J = 8 Hz), 4.86-4.76 (1H, m), 4.37 (2H, t, J = 13 Hz), 3.91 (2H, s), 3.60 (2H, dt, J = 7, 13 Hz),

1.42 (9H, s) ppm.

<Step 2> Synthesis of Compound 35

A mixture of compound 34 (1.0 g) and 4 N hydrochloric acid-ethyl acetate solution (10 mL) was stirred at room temperature for 1 hour. The solvent was removed from the reaction liquid under reduced pressure, and the residue was washed with ethyl acetate-heptane (2:1) to obtain compound 35 (0.48 g) as a white solid.
$^1$H-NMR (400 MHz, DMSO-$d_6$) $\delta$ 8.54 (3H, br), 7.53 (2H, d, J = 8 Hz), 7.25-7.18 (2H, m), 7.09-7.02 (2H, m), 6.84 (1H, dt, J = 1, 7 Hz), 6.23 (1H, d, J = 8 Hz), 4.56 (2H, t, J = 14 Hz), 3.94 (2H, s), 3.50 (2H, t, J = 16 Hz) ppm.

<Step 3> Synthesis of Diclofenac-(3-Amino-2,2-Difluoropropan-1-Ol)-Alginic Acid Derivative (Compound 36)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (37 mg), 1 M sodium hydrogen carbonate aqueous solution (0.11 mL), and compound 35 (38 mg) was added, and the mixture was stirred at room temperature overnight. After adding ethanol (20 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (93.6 mg) as a white solid. The drug introduction rate was 14.9 mol%.

(Example 9) Synthesis of Diclofenac-(N-(Aminoethyl)-2-Hydroxyacetamide)-Alginic Acid Derivative

Scheme 9

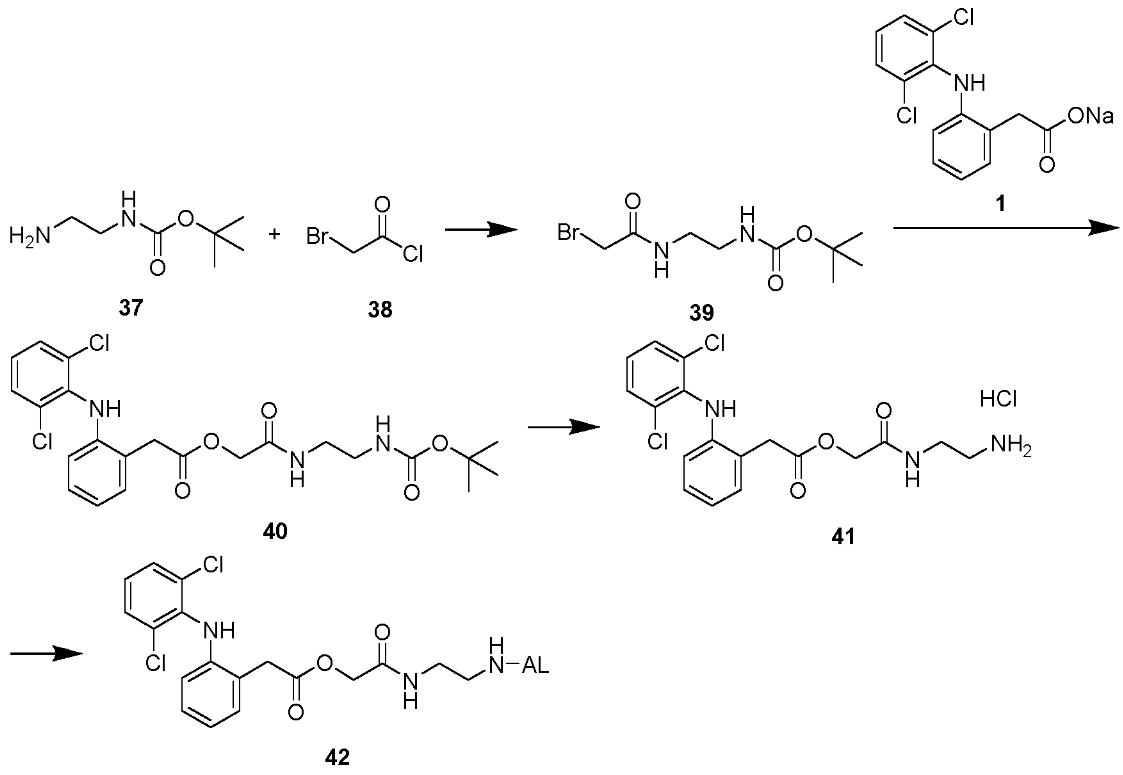


<Step 1> Synthesis of Compound 39

To a mixture liquid of ice-cooled commercially available tert-butyl(2-aminoethyl)carbamate (compound 37, 1.6 g), saturated sodium hydrogen carbonate aqueous solution (5 mL), water (20 mL), and 1,2-dimethoxyethane (20 mL), commercially available 2-bromoacetyl chloride (compound 38, 1.66 mL) was added dropwise, saturated sodium hydrogen carbonate aqueous solution (25 mL) was further added, and the mixture was stirred for 10 minutes. The reaction solution was extracted twice with ethyl acetate (80 mL), the combined extract liquid was dried over anhydrous sodium sulfate,

and the solvent was removed under reduced pressure. The residue was washed with heptane-ethyl acetate (1:1, 10 mL) to obtain compound 39 (0.62 g) as a white solid.
$^1$H-NMR (400 MHz, $CDCl_3$) δ 7.09 (1H, br), 4.86 (1H, br), 3.87 (2H, s), 3.43-3.37 (2H, m), 3.36-3.28 (2H, m), 1.45 (9H, s) ppm.

<Step 2> Synthesis of Compound 40

A mixture of commercially available sodium diclofenac (compound 1, 1.4 g), compound 39 (0.62 g), and N-methylpyrrolidone (4.4 mL) was stirred at 50°C for 1 hour. The reaction solution was cooled to room temperature, and then added with ethyl acetate (40 mL) and heptane (20 mL), which was washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (50-100% ethyl acetate/heptane) to obtain compound 40 (0.87 g) as a white solid.
$^1$H-NMR (400 MHz, $CDCl_3$) δ 7.34 (2H, d, J = 8 Hz), 7.29 (1H, dd, J = 1, 7 Hz), 7.15 (1H, dt, J = 1, 8 Hz), 7.03-6.91 (3H, m), 6.69 (1H, br), 6.55 (1H, d, J = 8 Hz), 4.92-4.79 (1H, m), 4.63 (2H, s), 3.96 (2H, s), 3.36-3.27 (2H, m), 3.25-3.16 (2H, m), 1.44 (9H, s) ppm.

<Step 3> Synthesis of Compound 41

A mixture of compound 40 (0.87 g) and 4 N hydrochloric acid-ethyl acetate solution (10 mL) was stirred at room temperature for 1 hour. The reaction suspension was filtered, and the solid collected by filtration was washed with ethyl acetate-heptane (2:1) to obtain compound 41 (0.62 g) as a white solid.
$^1$H-NMR (400 MHz, DMSO-$d_6$) δ 8.42 (1H, t, J = 5 Hz), 7.99 (3H, br), 7.53 (2H, d, J = 8 Hz), 7.26-7.19 (2H, m), 7.09-7.02 (2H, m), 6.85 (1H, dt, J = 1, 7 Hz), 6.23 (1H, d, J = 8 Hz), 4.56 (2H, s), 3.94 (2H, s), 3.39-3.30 (5H, m), 2.85 (2H, t, J = 6 Hz) ppm.

<Step 4> Synthesis of Diclofenac-(N-(Aminoethyl)-2-Hydroxyacetamide)-Alginic Acid Derivative (Compound 42)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (37 mg), 1 M sodium hydrogen carbonate aqueous solution (0.11 mL), and compound 41 (38 mg) was added, and the mixture was stirred at room temperature overnight. After adding ethanol (20 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (87.1 mg) as a white solid. The drug introduction rate was 12.3 mol%.

(Example 10) Synthesis of Diclofenac-(N-(Aminoethyl)-2-Hydroxypropanamide)-Alginic Acid Derivative

Scheme 10

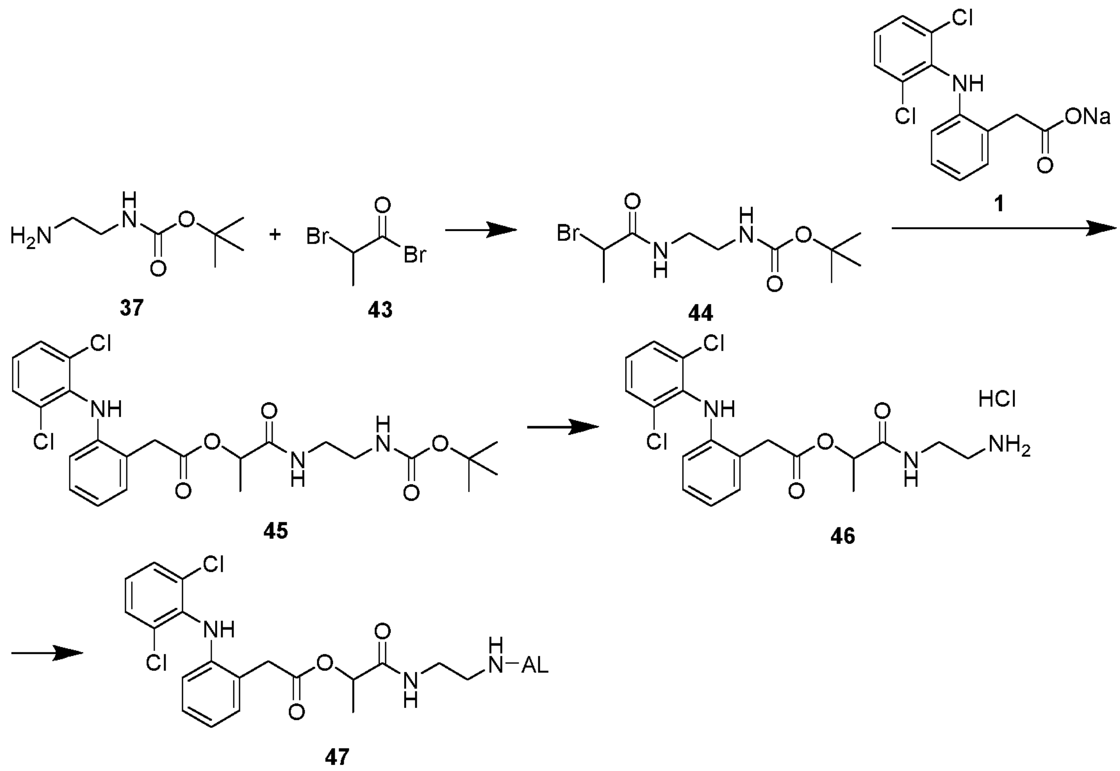

1 37 43 44 45 46 47

<Step 1> Synthesis of Compound 45

To a mixture liquid of ice-cooled commercially available tert-butyl(2-aminoethyl)carbamate (compound 37, 1.6 g), saturated sodium hydrogen carbonate aqueous solution (10 mL), water (20 mL), and 1,2-dimethoxyethane (30 mL), commercially available 2-bromopropanoyl bromide (compound 43, 2.12 mL) was added dropwise, saturated sodium hydrogen carbonate aqueous solution (25 mL) was further added, and the mixture was stirred for 10 minutes. The reaction solution was extracted twice with ethyl acetate (80 mL), the combined extract liquid was dried over anhydrous sodium sulfate, and the solvent was removed under reduced pressure. The residue was washed with heptane-ethyl acetate (3:2, 20 mL) to obtain a crude product of compound 44 (0.39 g).

A mixture of the crude product of compound 44 (0.39 g), commercially available sodium diclofenac (compound 1, 0.84 g), and N-methylpyrrolidone (2.6 mL) was stirred at 50°C for 1 hour and at 60°C for 3 hours. The reaction solution was cooled to room temperature, and then added with ethyl acetate (40 mL) and heptane (20 mL), which was washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (30-100% ethyl acetate/heptane) to obtain compound 45 (0.34 g) as a white solid.
$^1$H-NMR (400 MHz, $CDCl_3$) $\delta$ 7.36 (2H, d, J = 8 Hz), 7.31 (1H, dd, J = 1, 7 Hz), 7.16 (1H, dt, J = 1, 8 Hz), 7.04-6.97 (2H, m), 6.80 (1H, br), 6.68 (1H, br), 6.57 (1H, d, J = 8 Hz), 5.27 (1H, q, J = 7 Hz), 4.81 (1H, br), 4.02-3.88 (2H, m), 3.36-3.09 (4H, m), 1.50 (3H, d, J = 7 Hz), 1.44 (9H, d, J = 9 Hz) ppm.

<Step 2> Synthesis of Compound 46

A mixture of compound 45 (0.34 g) and 4 N hydrochloric acid-ethyl acetate solution (4 mL) was stirred at room temperature for 1 hour. The reaction suspension was filtered, and the solid collected by filtration was washed with ethyl acetate-heptane (2:1) and tert-butyl methyl ether to obtain compound 46 (0.24 g) as a white solid.
$^1$H-NMR (400 MHz, DMSO-$d_6$) $\delta$ 8.36 (1H, t, J = 5 Hz), 7.89 (3H, br), 7.53 (2H, d, J = 8 Hz), 7.25-7.18 (2H, m), 7.06 (1H, dt, J = 1, 8 Hz), 6.99 (1H, s), 6.84 (1H, dt, J = 1, 7 Hz), 6.23 (1H, d, J = 8 Hz), 5.02 (1H, q, J = 7 Hz), 3.91 (2H, s), 3.40-3.22 (2H, m), 2.83 (2H, t, J = 6 Hz), 1.37 (3H, d, J = 7 Hz) ppm.

<Step 3> Synthesis of Diclofenac-(N-(Aminoethyl)-2-Hydroxypropanamide)-Alginic Acid Derivative (Compound 47)

To a 1% (w/w) aqueous solution (10 g) of sodium alginate (manufactured by KIMICA, A2), an ethanol (5 mL) solution of 4-(4,6-dimethoxy-1,3,5-triazin-2-yl)-4-methylmorpholinium chloride (37 mg), 1 M sodium hydrogen carbonate

aqueous solution (0.11 mL), and compound 46 (40 mg) was added, and the mixture was stirred at room temperature overnight. After adding ethanol (20 mL), 0.1 g/mL sodium chloride aqueous solution (1 mL) was added, and the mixture was stirred for 10 minutes. The obtained precipitate was collected by filtration, washed with ethanol, and dried under reduced pressure to obtain the title compound (87.0 mg) as a white solid. The drug introduction rate was 12.5 mol%.

(Example 11) Release Test of Compounds Prepared in Examples 1 to 10

To 1 mg of each of the conjugates prepared in Examples 1 to 10, 20 mM sodium phosphate buffer (pH 5.3 or 7.0) or 1 N sodium hydroxide aqueous solution was added so that the concentration of the conjugate was 0.1% w/w concentration, and the mixture was stirred for 6 hours using a magnetic stirrer (ASONE REMIX RS-6A, 750r.p.m.) under an indoor environment at 20°C (set temperature of air conditioner). After confirming that no gel was formed, this solution was dispensed. Immediately after the dissolution, the amount of free diclofenac present in each solution was measured by LC-MS/MS as an initial state (day 0 of storage). Also, the other dispensed solutions were incubated at 37°C for 1, 3, and 7 days, and then the amount of free diclofenac was measured. At each point of time, the release rate (%) was calculated using the ratio with the amount of diclofenac released by forced decomposition in a 1 N sodium hydroxide aqueous solution.

The LC conditions are as follows.
Temperature: 40°C
Flow rate: 0.7 mL/min
Column: ODS-4: 3 μm (2.1 × 30 mm)
Solvent: (A) 0.1% formic acid aqueous solution, (B) 100% acetonitrile
Gradient:

| Time (min) | %B |
|---|---|
| 0 | 35 |
| 0.5 | 35 |
| 1.5 | 95 |
| 2 | 95 |
| 2 | 35 |
| 3 | 35 |

The MS conditions are as follows.

Ionization mode: ESI-negative
Ion source temperature: 300 degrees
Capillary voltage: -4000 V

Release Rate in Release Test at pH 7.0

Table 3

| Compound Number | 5a | 5b | 5c | 5d |
|---|---|---|---|---|
| Day 1 | 0.2% | 0.1% | 0.3% | 0.2% |
| Day 3 | 2.3% | 2.1% | 2.5% | 2.4% |
| Day 7 | 4.1% | 5.0% | 5.5% | 5.1% |

Release Rate in Release Test at pH 7.0

Table 4

| Compound Number | 9 | 14 | 19 | 24 |
|---|---|---|---|---|
| Day 1 | 0.0% | 0.0% | 0.0% | 0.0% |
| Day 3 | 0.7% | 0.0% | 0.8% | 1.4% |
| Day 7 | 1.9% | 0.0% | 12.3% | 5.4% |

Release Rate in Release Test at pH 7.0

Table 5

| Compound Number | 28 | 32 | 36 | 42 | 47 |
|---|---|---|---|---|---|
| Day 1 | 0.1% | 0.0% | 1.2% | 1.8% | 0.8% |
| Day 3 | 1.2% | 5.7% | 8.8% | 10.2% | 6.1% |
| Day 7 | 4.0% | 15.2% | 12.9% | 21.0% | 10.9% |

Release Rate in Release Test at pH 5.3

Table 6

| Compound Number | 5a | 5b | 5c | 5d |
|---|---|---|---|---|
| Day 1 | 0.0% | 0.0% | 0.0% | 0.0% |
| Day 3 | 0.1% | 0.0% | 0.1% | 0.1% |
| Day 7 | 0.2% | 0.3% | 0.4% | 0.4% |

Release Rate in Release Test at pH 5.3

Table 7

| Compound Number | 9 | 14 | 19 | 24 |
|---|---|---|---|---|
| Day 1 | 0.0% | 0.0% | 0.0% | 0.0% |
| Day 3 | 0.0% | 0.0% | 0.0% | 0.0% |
| Day 7 | 0.1% | 0.0% | 0.0% | 0.0% |

Release Rate in Release Test at pH 5.3

Table 8

| Compound Number | 28 | 32 | 36 | 42 | 47 |
|---|---|---|---|---|---|
| Day 1 | 0.0% | 0.0% | 0.0% | 0.0% | 0.0% |
| Day 3 | 0.1% | 1.5% | 0.5% | 0.5% | 0.1% |
| Day 7 | 0.3% | 7.6% | 1.0% | 3.3% | 0.7% |

(Example 12) Synthesis of Diclofenac-(2-Aminoethoxyethanol)-Alginic Acid Derivative

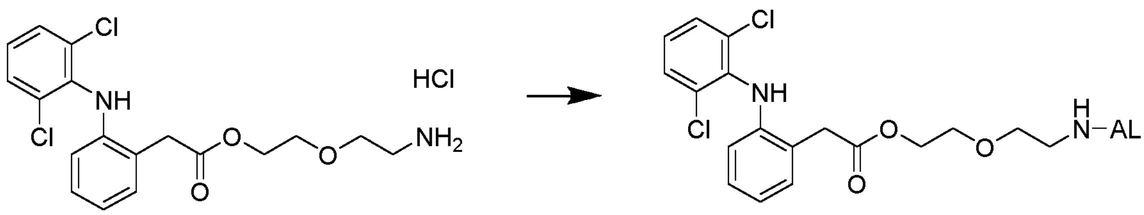

13

14a, 14b

<Step 1-1> Synthesis of Diclofenac-(2-Aminoethoxyethanol)-Alginic Acid Derivative (Compound 14a)

To a solution obtained by dissolving 1 g of sodium alginate (Mochida Pharmaceutical Co., Ltd., A-2) in water

(100 mL) and adding ethanol (30 mL) thereto, 4-(4,6-dimethoxy-1,3,5-triazin-2-yl)-4-methylmorpholinium chloride (456 mg) was added at room temperature. After stirring for 30 minutes, a solution of compound 13 (346 mg) in ethanol (10 mL)-water (5 mL) and a 1 M sodium hydrogen carbonate aqueous solution (0.82 mL) were added dropwise, and the mixture was stirred at room temperature for 4 hours. A 0.1 g/mL sodium chloride aqueous solution (5 mL) and ethanol (100 mL) were added to the reaction liquid, followed by stirring for 10 minutes. The obtained precipitate was collected by filtration, washed with ethanol, and dried under reduced pressure to obtain the title compound (1.06 g) as a white solid. The drug introduction rate was 14.0 mol%.

<Step 1-2> Synthesis of Diclofenac-(2-Aminoethoxyethanol)-Alginic Acid Derivative (Compound 14b)

Using 1 g of sodium alginate (Mochida Pharmaceutical Co., Ltd., A-3), the same operation as in (Example 12) <Step 1-1> was performed to obtain the title compound (1.11 g) as a white solid. The drug introduction rate was 16.4 mol%.

(Example 13) Synthesis of Diclofenac-(2-Amino-N-(2-Hydroxyethyl)Acetamide)-Alginic Acid Derivative

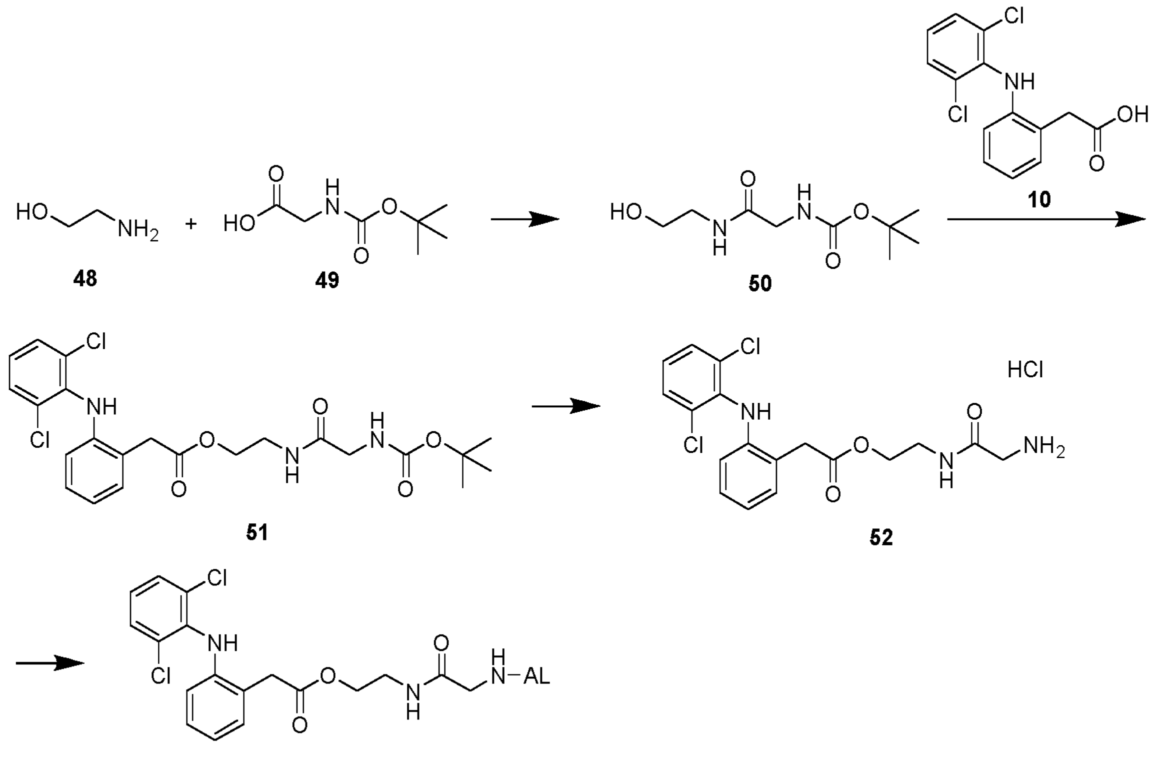

48 49 50 10 51 52

53a, 53b, 53c, 53d

<Step 1> Synthesis of Compound 50

To a mixture liquid of 2-aminoethanol (2.09 g), (tert-butoxycarbonyl)glycine (5.0 g) and ethanol (30 mL), 4-(4,6-dimethoxy-1,3,5-triazin-2-yl)-4-methylmorpholinium chloride (11.4 g) was added at room temperature, and the mixture was stirred for 5 hours. The reaction suspension was filtered using ethanol, and the filtrate was concentrated under reduced pressure. Ethyl acetate was added to the residue, the suspension was filtered, and the filtrate was concentrated under reduced pressure. The residue was purified by silica gel column chromatography (0-20% methanol/ethyl acetate) to obtain compound 50 (7.3 g).
$^1$H-NMR (400 MHz, $CDCl_3$) δ 6.92 (1H, br), 5.50 (1H, br), 4.03 (1H, s), 3.80 (2H, d, J = 6 Hz), 3.73-3.68 (2H, m), 3.46-3.40 (2H, m), 1.45 (9H, s) ppm.

<Step 2> Synthesis of Compound 51

To a mixture of commercially available diclofenac (compound 10, 2.0 g), compound 50 (2.2 g), N,N-dimethyl-4-aminopyridine (0.17 g), and dichloromethane (7 mL), a dichloromethane (7 mL) solution of N,N'-dicyclohexylcarbodiimide (1.39 g) was added dropwise under ice cooling. The reaction liquid was stirred at room temperature for 2 hours. The reaction liquid was filtered using ethyl acetate (60 mL), then washed successively with saturated sodium hydrogen carbonate aqueous solution (20 mL) and water, and dried over anhydrous sodium sulfate, and the solvent wasremoved under reduced pressure. The residue was purified by silica gel column chromatography (10-100% ethyl acetate/heptane) to obtain compound 51 (2.7 g) as a white amorphous.
$^1$H-NMR (400 MHz, $CDCl_3$) δ 7.35 (2H, d, J = 8 Hz), 7.23 (1H, dd, J = 7, 1 Hz), 7.14 (1H, td, J = 8, 1 Hz), 7.02-6.94 (2H, m), 6.83 (1H, s), 6.55 (1H, d, J = 8 Hz), 6.23 (1H, br), 4.96 (1H, br), 4.25 (2H, t, J = 5 Hz), 3.83 (2H, s), 3.69 (2H, d, J = 6 Hz), 3.59-3.53 (2H, m), 1.44 (9H, s) ppm.

<Step 3> Synthesis of Compound 52

A mixture of compound 51 (2.7 g) and 4 N hydrochloric acid-1,4-dioxane solution (27.2 mL) was stirred at room temperature for 30 minutes. The reaction suspension was filtered, and the solid collected by filtration was washed with dimethoxyethane-ethanol (1:1) and dimethoxyethane to obtain compound 52 (1.73 g) as a white solid.
$^1$H-NMR (400 MHz, DMSO-$d_6$) δ 8.66 (1H, t, J = 5 Hz), 8.11 (3H, br), 7.54 (2H, d, J = 8 Hz), 7.24-7.17 (2H, m), 7.09-7.03 (2H, m), 6.85 (1H, td, J = 7, 1 Hz), 6.24 (1H, d, J = 8 Hz), 4.12 (2H, t, J = 6 Hz), 3.83 (2H, s), 3.52 (2H, s), 3.45-3.38 (2H, m) ppm.

<Step 4-1> Synthesis of Diclofenac-(2-Amino-N-(2-Hydroxyethyl)Acetamide)-Alginic Acid Derivative (Compound 53a)

To a solution obtained by dissolving 2 g of sodium alginate (Mochida Pharmaceutical Co., Ltd., A-1) in water (200 mL) and adding ethanol (60 mL) thereto, 4-(4,6-dimethoxy-1,3,5-triazin-2-yl)-4-methylmorpholinium chloride (1.3 g) was added at room temperature. After stirring for 30 minutes, a solution of compound 52 (0.85 g) in ethanol (20 mL)-water (10 mL) and a 1 M sodium hydrogen carbonate aqueous solution (1.97 mL) were added dropwise, and the mixture was stirred at room temperature for 4 hours. A 0.1 g/mL sodium chloride aqueous solution (10 mL) and ethanol (200 mL) were added to the reaction liquid, followed by stirring for 10 minutes. The obtained precipitate was collected by filtration, washed with ethanol, and dried under reduced pressure to obtain the title compound (1.80 g) as a white solid. The drug introduction rate was 19.7 mol%.

<Step 4-2> Synthesis of Diclofenac-(2-Amino-N-(2-Hydroxyethyl)Acetamide)-Alginic Acid Derivative (Compound 53b)

To a solution obtained by dissolving 2 g of sodium alginate (Mochida Pharmaceutical Co., Ltd., A-2) in water (200 mL) and adding ethanol (60 mL) thereto, 4-(4,6-dimethoxy-1,3,5-triazin-2-yl)-4-methylmorpholinium chloride (611 mg) was added at room temperature. After stirring for 30 minutes, a solution of compound 52 (396 mg) in ethanol (20 mL)-water (10 mL) and a 1 M sodium hydrogen carbonate aqueous solution (0.92 mL) were added dropwise, and the mixture was stirred at room temperature for 4 hours. A 0.1 g/mL sodium chloride aqueous solution (10 mL) and ethanol (200 mL) were added to the reaction liquid, followed by stirring for 10 minutes. The obtained precipitate was collected by filtration, washed with ethanol, and dried under reduced pressure to obtain the title compound (2.04 g) as a white solid. The drug introduction rate was 14.6 mol%.

<Step 4-3> Synthesis of Diclofenac-(2-Amino-N-(2-Hydroxyethyl)Acetamide)-Alginic Acid Derivative (Compound 53c)

Using 2 g of sodium alginate (Mochida Pharmaceutical Co., Ltd., A-3), the same operation as in (Example 13) <Step 4-2> was performed to obtain the title compound (1.99 g) as a white solid. The drug introduction rate was 17.0 mol%.

<Step 4-4> Synthesis of Diclofenac-(2-Amino-N-(2-Hydroxyethyl)Acetamide)-Alginic Acid Derivative (Compound 53d)

To a solution obtained by dissolving 100 mg of sodium alginate (Mochida Pharmaceutical Co., Ltd., B-2) in water (10 mL) and adding ethanol (3 mL) thereto, 4-(4,6-dimethoxy-1,3,5-triazin-2-yl)-4-methylmorpholinium chloride (46 mg) was added at room temperature. After stirring for 10 minutes, a solution of compound 52 (40 mg) in ethanol (2 mL)-water (1 mL) and a 1 M sodium hydrogen carbonate aqueous solution (0.09 mL) were added dropwise, and the mixture was stirred at room temperature for 4 hours. A 0.1 g/mL sodium chloride aqueous solution (1 mL) and ethanol (20 mL) were added to the reaction liquid, followed by stirring for 10 minutes. The obtained precipitate was collected by filtration, washed with ethanol, and dried under reduced pressure to obtain the title compound (108 mg) as a white solid. The drug introduction rate was 23.1 mol%.

(Example 14) Synthesis of Diclofenac-(2-Amino-1-(4-(Hydroxymethyl)Piperidin-1-Yl)Ethan-1-One)-Alginic Acid Derivative

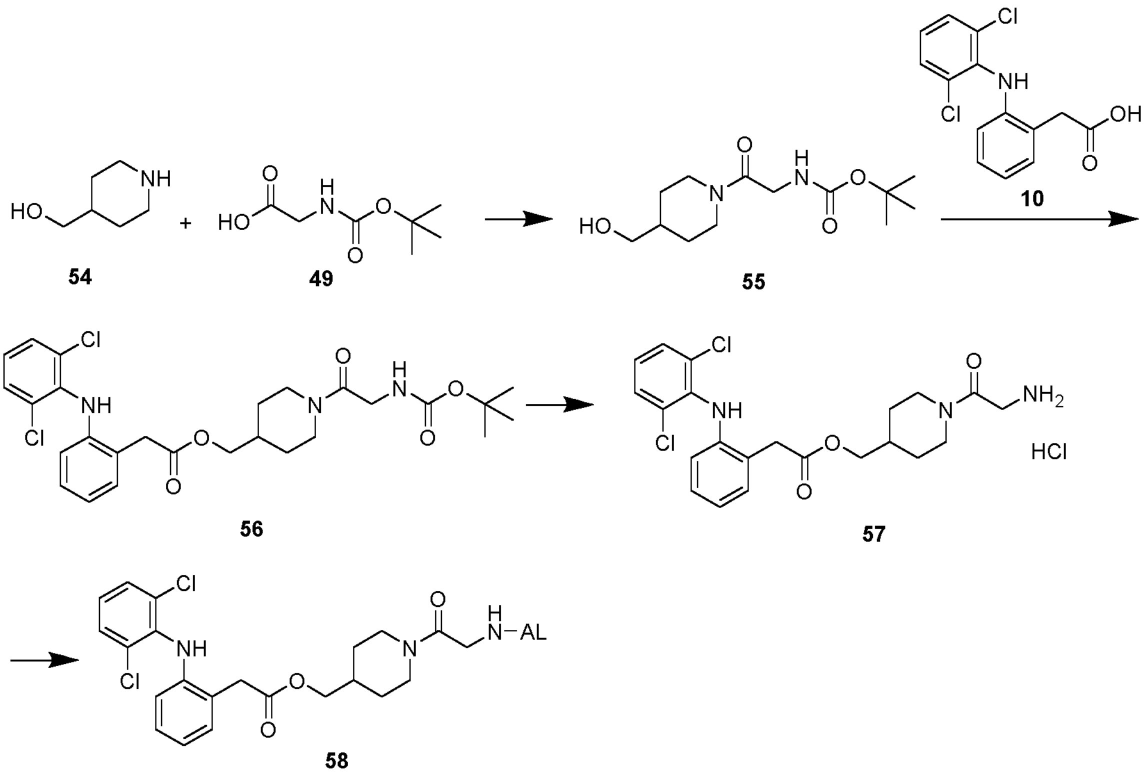

54 49 55 10 56 57 58

<Step 1> Synthesis of Compound 55

To an ethanol (10 mL) mixture liquid of 4-piperidinemethanol (0.79 g) and (tert-butoxycarbonyl)glycine (1 g), 4-(4,6-dimethoxy-1,3,5-triazin-2-yl)-4-methylmorpholinium chloride (1.9 g) was added at room temperature, and the mixture was stirred for 4 hours. Ethyl acetate was added to the reaction suspension, the suspension was filtered, and the filtrate was concentrated under reduced pressure. The residue was purified by silica gel column chromatography (0-20% methanol/ethyl acetate) to obtain compound 55 (1.2 g) as a white amorphous.

$^1$H-NMR (400 MHz, $CDCl_3$) δ 5.57 (1H, br), 4.64-4.56 (1H, m), 4.02-3.89 (2H, m), 3.77-3.68 (1H, m), 3.56-3.46 (2H, m), 3.01 (1H, td, J = 13, 3 Hz), 2.63 (1H, td, J = 13, 3 Hz), 1.89-1.67 (3H, m), 1.46 (9H, s), 1.24-1.09 (2H, m) ppm.

<Step 2> Synthesis of Compound 56

To a mixture of commercially available diclofenac (compound 10, 0.87 g), compound 55 (1.2 g), N,N-dimethyl-4-aminopyridine (0.07 g), and dichloromethane (5 mL), a dichloromethane (5 mL) solution of N,N'-dicyclohexylcarbodiimide (0.61 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (20 mL) and water, and dried over anhydrous sodium sulfate, and the solvent was removed under reduced pressure. The residue was purified by silica gel column chromatography (5-50% ethyl acetate/heptane) to obtain compound 56 (1.4 g) as a white amorphous.

$^1$H-NMR (400 MHz, $CDCl_3$) δ 7.34 (2H, d, J = 8 Hz), 7.22 (1H, dd, J = 7, 2 Hz), 7.12 (1H, td, J = 8, 2 Hz), 7.01-6.93 (2H, m), 6.86 (1H, s), 6.55 (1H, d, J = 8 Hz), 5.53 (1H, br), 4.61-4.54 (1H, m), 4.06-3.96 (2H, m), 3.95-3.91 (2H, m), 3.81 (2H, s), 3.71-3.63 (1H, m), 3.02-2.90 (1H, m), 2.64-2.53 (1H, d, J = 2.7 Hz), 2.00-1.86 (1H, m), 1.80-1.67 (2H, m), 1.45 (9H, m), 1.30-1.04 (2H, m) ppm.

<Step 3> Synthesis of Compound 57

A mixture of compound 56 (1.4 g) and 4 N hydrochloric acid-1,4-dioxane solution (12.7 mL) was stirred at room

temperature for 30 minutes. The reaction liquid was filtered under reduced pressure to obtain compound 57 (1.3 g) as a white amorphous matter.

$^1$H-NMR (400 MHz, DMSO-$d_6$) $\delta$ 8.09 (3H, br), 7.53 (2H, d, J = 8 Hz), 7.23-7.17 (2H, m), 7.08-7.03 (2H, m), 6.85 (1H, td, J = 7, 1 Hz), 6.26 (1H, d, J = 8 Hz), 4.36-4.28 (1H, m), 4.01-3.92 (2H, m), 3.89-3.76 (2H, m), 3.70-3.61 (1H, m), 3.48-3.40 (1H, m), 3.03-2.92 (1H, m), 2.67-2.57 (1H, m), 1.98-1.85 (1H, m), 1.73-1.63 (2H, m), 1.27-0.95 (3H, m) ppm.

<Step 4> Synthesis of Diclofenac-(2-Amino-1-(4-(Hydroxymethyl)Piperidin-1-Yl)Ethan-1-One)-Alginic Acid Derivative (Compound 58)

To a solution obtained by dissolving 100 mg of sodium alginate (Mochida Pharmaceutical Co., Ltd., B-2) in water (20 mL) and adding ethanol (5 mL) thereto, 4-(4,6-dimethoxy-1,3,5-triazin-2-yl)-4-methylmorpholinium chloride (39 mg) was added at room temperature. After stirring for 10 minutes, a solution of compound 57 (45 mg) in ethanol (3 mL) and a 1 M sodium hydrogen carbonate aqueous solution (0.09 mL) were added dropwise, and the mixture was stirred at room temperature for 4 hours. A 0.1 g/mL sodium chloride aqueous solution (1 mL) and ethanol (20 mL) were added to the reaction liquid, followed by stirring for 10 minutes. The obtained precipitate was collected by filtration, washed with ethanol, and dried under reduced pressure to obtain the title compound (108 mg) as a white solid. The drug introduction rate was 18.4 mol%.

(Example 15) Synthesis of Diclofenac-(2-Amino-1-(4-Hydroxypiperidin-1-Yl)Ethan-1-One)-Alginic Acid Derivative

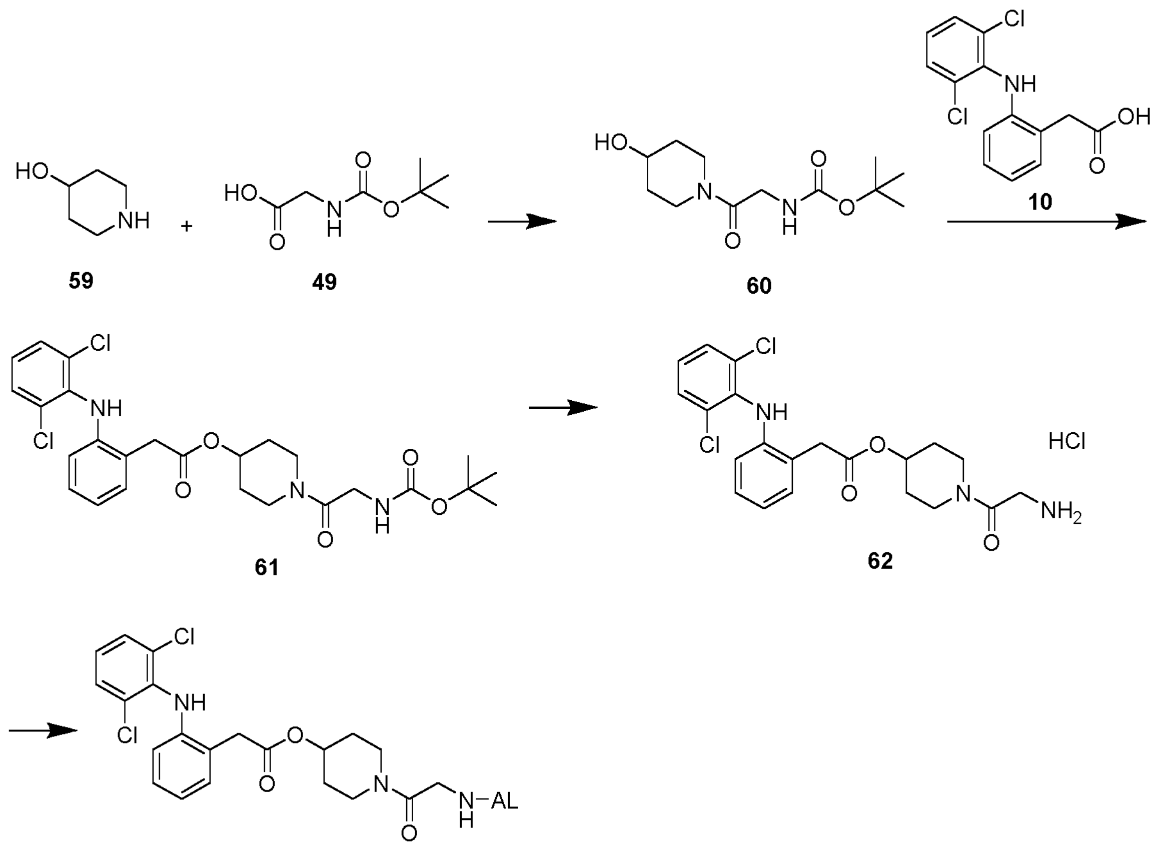

59 49 60 10 61 62

63a, 63b

<Step 1> Synthesis of Compound 60

To an ethanol (10 mL) mixture liquid of 4-hydroxypiperidine (0.69 g) and (tert-butoxycarbonyl)glycine (1.0 g), 4-(4,6-dimethoxy-1,3,5-triazin-2-yl)-4-methylmorpholinium chloride (1.9 g) was added at room temperature, and the mixture was stirred for 4 hours. Ethyl acetate was added to the reaction suspension, the suspension was filtered, and the filtrate was concentrated under reduced pressure. The residue was purified by silica gel column chromatography (0-20% methanol/ethyl acetate) to obtain compound 60 (1.0 g) as a white amorphous.

$^1$H-NMR (400 MHz, $CDCl_3$) δ 5.56 (1H, br), 4.06-3.92 (5H, m), 3.67-3.57 (1H, m), 3.34-3.25 (1H, m), 3.22-3.13 (1H, m), 1.99-1.84 (2H, m), 1.62-1.47 (2H, m), 1.45 (9H, s) ppm.

<Step 2> Synthesis of Compound 61

To a mixture of commercially available diclofenac (compound 10, 0.76 g), compound 60 (0.99 g), N,N-dimethyl-4-aminopyridine (0.06 g), and dichloromethane (5 mL), a dichloromethane (5 mL) solution of N,N'-dicyclohexylcarbodiimide (0.53 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (20 mL) and water, and dried over anhydrous sodium sulfate, and the solvent was removed under reduced pressure. The residue was purified by silica gel column chromatography (5-50% ethyl acetate/heptane) to obtain compound 61 (1.0 g) as a white amorphous.
$^1$H-NMR (400 MHz, $CDCl_3$) δ 7.34 (2H, d, J = 8 Hz), 7.21 (1H, dd, J = 8, 2 Hz), 7.13 (1H, td, J = 8, 2 Hz), 7.01-6.92 (2H, m), 6.81 (1H, s), 6.55 (1H, d, J = 8 Hz), 5.50 (1H, br), 5.08-5.01 (1H, m), 3.95 (2H, d, J = 4 Hz), 3.88-3.79 (3H, m), 3.56-3.42 (2H, m), 3.31-3.21 (1H, m), 1.97-1.84 (2H, m), 1.75-1.64 (2H, m), 1.45 (9H, s) ppm.

<Step 3> Synthesis of Compound 62

A mixture of compound 61 (1.0 g) and 4 N hydrochloric acid-1,4-dioxane solution (9.3 mL) was stirred at room temperature for 30 minutes. The reaction liquid was filtered under reduced pressure to obtain compound 62 (0.91 g) as a white amorphous.
$^1$H-NMR (400 MHz, DMSO-$d_6$) δ 8.14 (3H, br), 7.53 (2H, d, J = 8 Hz), 7.24-7.17 (2H, m), 7.08-7.02 (2H, m), 6.84 (1H, td, J = 7, 1 Hz), 6.24 (1H, d, J = 8 Hz), 5.03-4.94 (1H, m), 3.93-3.79 (4H, m), 3.77-3.63 (1H, m), 3.54-3.24 (3H, m), 1.94-1.45 (4H, m) ppm.

<Step 4-1> Synthesis of Diclofenac-(2-Amino-1-(4-(Hydroxymethyl)Piperidin-1-Yl)Ethan-1-One)-Alginic Acid Derivative (Compound 63a)

To a solution obtained by dissolving 1 g of sodium alginate (Mochida Pharmaceutical Co., Ltd., A-2) in water (100 mL) and adding ethanol (30 mL) thereto, 4-(4,6-dimethoxy-1,3,5-triazin-2-yl)-4-methylmorpholinium chloride (370 mg) was added at room temperature. After stirring for 10 minutes, a solution of compound 62 (350 mg) in ethanol (10 mL) and a 1 M sodium hydrogen carbonate aqueous solution (0.74 mL) were added dropwise, and the mixture was stirred at room temperature for 4 hours. A 0.1 g/mL sodium chloride aqueous solution (10 mL) and ethanol (100 mL) were added to the reaction liquid, followed by stirring for 10 minutes. The obtained precipitate was collected by filtration, washed with ethanol, and dried under reduced pressure to obtain the title compound (1.07 g) as a white solid. The drug introduction rate was 11.5 mol%.

<Step 4-2> Synthesis of

Diclofenac-(2-Amino-1-(4-(Hydroxymethyl)Piperidin-1-Yl)Ethan-1-One)-Alginic Acid Derivative (Compound 63b)

Using 1 g of sodium alginate (Mochida Pharmaceutical Co., Ltd., A-3), the same operation as in (Example 15) <Step 4-1> was performed to obtain the title compound (1.09 g) as a white solid. The drug introduction rate was 12.6 mol%.

(Example 16) Synthesis of

Diclofenac-(2-Amino-N-(6-Hydroxyhexyl)Acetamide)-Alginic Acid Derivative

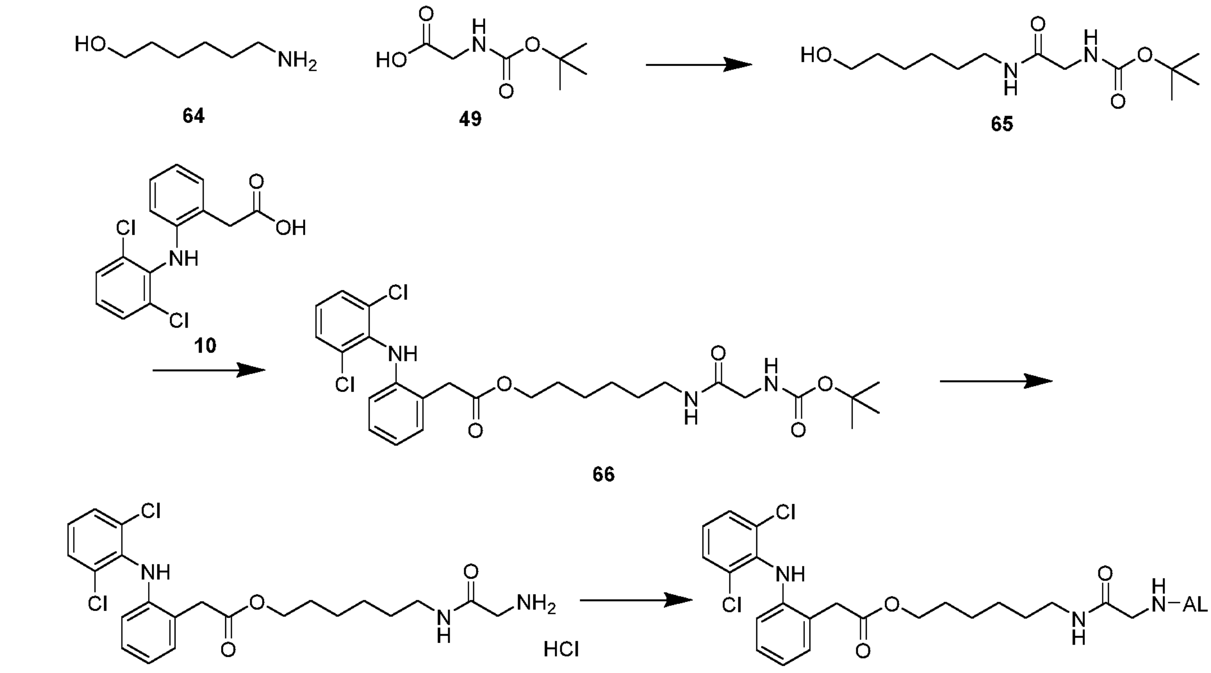

64 49 65

10

66

67 68

<Step 1> Synthesis of Compound 65

To an ethanol (10 mL) mixture liquid of 6-amino-1-hexanol (0.8 g) and (tert-butoxycarbonyl)glycine (1 g), 4-(4,6-dimethoxy-1,3,5-triazin-2-yl)-4-methylmorpholinium chloride (1.9 g) was added at room temperature, and the mixture was stirred for 4 hours. Ethyl acetate was added to the reaction suspension, the suspension was filtered, and the filtrate was concentrated under reduced pressure. The residue was purified by silica gel column chromatography (0-20% methanol/ethyl acetate) to obtain compound 65 (1.6 g) as a white amorphous.
$^1$H-NMR (400 MHz, $CDCl_3$) δ 6.42 (1H, br), 5.38 (1H, br), 3.77 (2H, d, J = 6 Hz), 3.63 (2H, t, J = 6 Hz), 3.31-3.22 (2H, m), 1.62-1.47 (4H, m), 1.45 (9H, s), 1.43-1.28 (4H, m) ppm.

<Step 2> Synthesis of Compound 66

To a mixture of commercially available diclofenac (compound 10, 1.0 g), compound 65 (1.39 g), N,N-dimethyl-4-aminopyridine (0.08 g), and dichloromethane (5 mL), a dichloromethane (5 mL) solution of N,N'-dicyclohexylcarbodiimide (0.7 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (20 mL) and water, and dried over anhydrous sodium sulfate, and the solvent was removed under reduced pressure. The residue was purified by silica gel column chromatography (10-100% ethyl acetate/heptane) to obtain compound 66 (1.5 g) as a white amorphous.
$^1$H-NMR (400 MHz, $CDCl_3$) δ 7.34 (2H, d, J = 8 Hz), 7.22 (1H, dd, J = 8, 2 Hz), 7.12 (1H, td, J = 8, 2 Hz), 7.00-6.90 (3H, m), 6.54 (1H, d, J = 8 Hz), 6.12 (1H, br), 5.16 (1H, br), 4.13 (2H, t, J = 7 Hz), 3.80 (2H, s), 3.76 (2H, d, J = 6 Hz), 3.27-3.19 (2H, m), 1.69-1.59 (2H, m), 1.52-1.40 (11H, m), 1.38-1.25 (4H, m) ppm.

<Step 3> Synthesis of Compound 67

A mixture of compound 66 (1.5 g) and 4 N hydrochloric acid-1,4-dioxane solution (13.6 mL) was stirred at room temperature for 30 minutes. The reaction liquid was filtered under reduced pressure to obtain compound 67 (1.3 g) as a white amorphous.
$^1$H-NMR (400 MHz, DMSO-$d_6$) δ 8.34 (1H, br), 8.03 (3H, br), 7.53 (2H, d, J = 8 Hz), 7.23-7.16 (2H, m), 7.08-7.03 (2H, m), 6.85 (1H, td, J = 8, 1 Hz), 6.26 (1H, d, J = 8 Hz), 4.07 (2H, t, J = 7 Hz), 3.79 (2H, s), 3.49 (2H, s), 3.12-3.04 (2H, m), 1.63-1.51 (2H, m), 1.41-1.33 (2H, m), 1.32-1.21 (4H, m) ppm.

<Step 4> Synthesis of Diclofenac-(2-Amino-N-(6-Hydroxyhexyl)Acetamide)-Alginic Acid Derivative (Compound 68)

To a solution obtained by dissolving 100 mg of sodium alginate (Mochida Pharmaceutical Co., Ltd., B-2) in water (20 mL) and adding ethanol (5 mL) thereto, 4-(4,6-dimethoxy-1,3,5-triazin-2-yl)-4-methylmorpholinium chloride (39 mg) was added at room temperature. After stirring for 10 minutes, a solution of compound 67 (45 mg) in ethanol (3 mL) and a 1 M sodium hydrogen carbonate aqueous solution (0.09 mL) were added dropwise, and the mixture was stirred at room temperature for 4 hours. A 0.1 g/mL sodium chloride aqueous solution (1 mL) and ethanol (20 mL) were added to the reaction liquid, followed by stirring for 10 minutes. The obtained precipitate was collected by filtration, washed with ethanol, and dried under reduced pressure to obtain the title compound (106 mg) as a white solid. The drug introduction rate was 18.0 mol%.

(Example 17) Synthesis of Diclofenac-(2-Amino-N-(2-Hydroxypropyl)Acetamide)-Alginic Acid Derivative

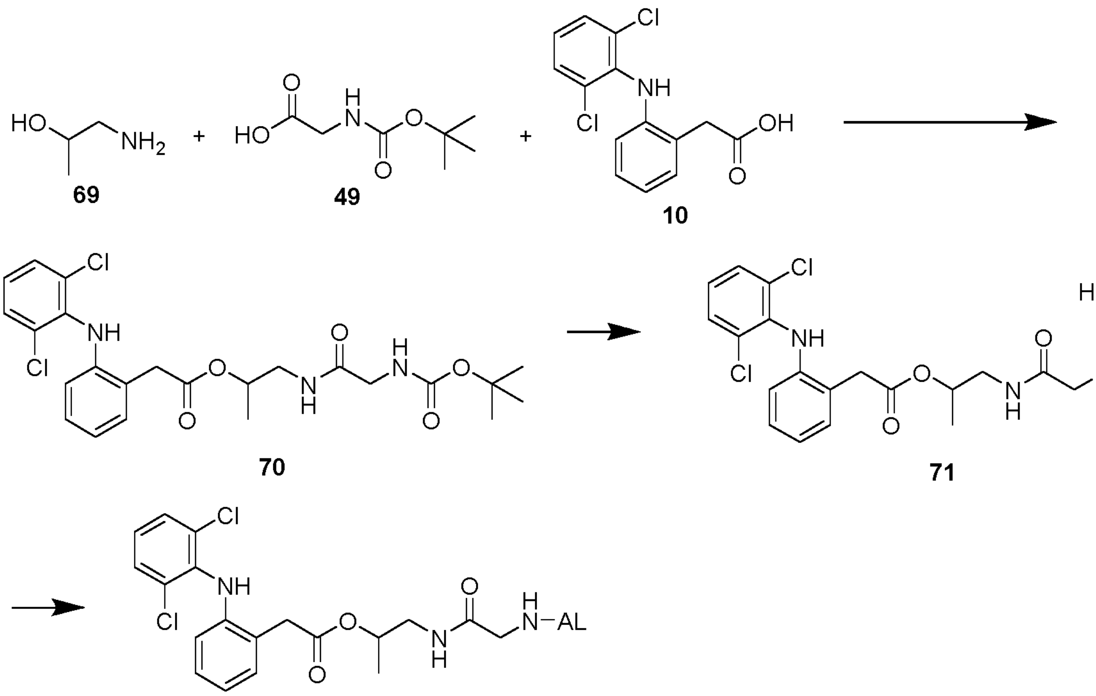

69 49 10

70 71

72a, 72b

<Step 1> Synthesis of Compound 70

To a mixture of 1-amino-2-propanol (0.38 g), (tert-butoxycarbonyl)glycine (0.88 g), N,N-dimethyl-4-aminopyridine (0.12 g), and dichloromethane (20 mL), a dichloromethane (5 mL) solution of N,N'-dicyclohexylcarbodiimide (1.03 g) was added dropwise under ice cooling. The reaction liquid was stirred at room temperature overnight. To the reaction liquid, a dichloromethane (5 mL) solution of commercially available diclofenac (compound 10, 1.48 g) and N,N'-dicyclohexylcarbodiimide (1.03 g) was added dropwise. The reaction liquid was stirred at room temperature overnight. The reaction liquid was filtered, and then the filtrate was removed under reduced pressure to remove the solvent. The residue was purified by silica gel column chromatography (5-100% ethyl acetate/heptane) to obtain compound 70 (0.74 g) as a white amorphous.
$^1$H-NMR (400 MHz, $CDCl_3$) δ 7.34 (2H, d, J = 8 Hz), 7.24 (1H, dd, J = 8, 1 Hz), 7.14 (1H, td, J = 8, 1 Hz), 7.02-6.95 (2H, m), 6.87 (1H, s), 6.56 (1H, d, J = 8 Hz), 6.08 (1H, br), 5.12-5.02 (1H, m), 4.84 (1H, br), 3.84-3.76 (2H, m), 3.67-3.34 (4H, m), 1.43 (9H, s), 1.27 (3H, d, J = 6 Hz) ppm.

<Step 2> Synthesis of Compound 71

A mixture of compound 70 (0.74 g) and 4 N hydrochloric acid-1,4-dioxane solution (8 mL) was stirred at room temperature for 30 minutes. The reaction liquid was filtered under reduced pressure to obtain compound 71 (0.7 g) as

a white amorphous.
$^1$H-NMR (400 MHz, DMSO-$d_6$) $\delta$ 8.62 (1H, t, J = 6 Hz), 8.12 (3H, br), 7.53 (2H, d, J = 8 Hz), 7.24-7.17 (2H, m), 7.09-7.02 (2H, m), 6.85 (1H, td, J = 7, 1 Hz), 6.25 (1H, d, J = 8 Hz), 4.95-4.84 (1H, m), 3.80 (2H, s), 3.59-3.21 (4H, m), 1.18 (3H, d, J = 6 Hz) ppm.

<Step 3-1> Synthesis of Diclofenac-(2-Amino-N-(2-Hydroxypropyl)Acetamide)-Alginic Acid Derivative (Compound 72a)

To a solution obtained by dissolving 1 g of sodium alginate (Mochida Pharmaceutical Co., Ltd., A-2) in water (100 mL) and adding ethanol (30 mL) thereto, 4-(4,6-dimethoxy-1,3,5-triazin-2-yl)-4-methylmorpholinium chloride (305 mg) was added at room temperature. After stirring for 30 minutes, a solution of compound 71 (205 mg) in ethanol (10 mL) and a 1 M sodium hydrogen carbonate aqueous solution (0.46 mL) were added dropwise, and the mixture was stirred at room temperature for 4 hours. A 0.1 g/mL sodium chloride aqueous solution (5 mL) and ethanol (100 mL) were added to the reaction liquid, followed by stirring for 10 minutes. The obtained precipitate was collected by filtration, washed with ethanol, and dried under reduced pressure to obtain the title compound (1.04 g) as a white solid. The drug introduction rate was 13.1 mol%.

<Step 3-2> Synthesis of Diclofenac-(2-Amino-N-(2-Hydroxypropyl)Acetamide)-Alginic Acid Derivative (Compound 72b)

To a solution obtained by dissolving 200 mg of sodium alginate (Mochida Pharmaceutical Co., Ltd., A-3) and adding ethanol (6 mL) thereto, 4-(4,6-dimethoxy-1,3,5-triazin-2-yl)-4-methylmorpholinium chloride (62 mg) was added at room temperature. After stirring for 10 minutes, a solution of compound 71 (41 mg) in ethanol (2 mL) and a 1 M sodium hydrogen carbonate aqueous solution (0.09 mL) were added dropwise, and the mixture was stirred at room temperature for 4 hours. A 0.1 g/mL sodium chloride aqueous solution (1 mL) and ethanol (40 mL) were added to the reaction liquid, followed by stirring for 10 minutes. The obtained precipitate was collected by filtration, washed with ethanol, and dried under reduced pressure to obtain the title compound (199 mg) as a white solid. The drug introduction rate was 14.5 mol%.

(Example 18) Synthesis of Diclofenac-(2-Amino-N-(3-Hydroxypropyl)Acetamide)-Alginic Acid Derivative

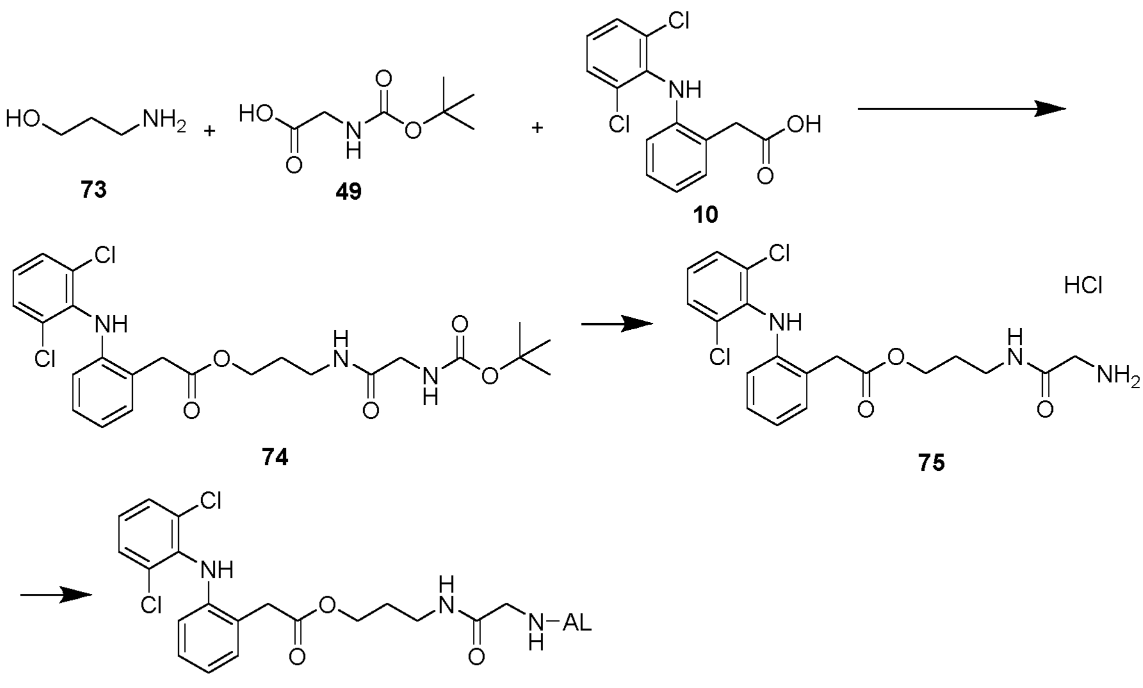

73 49 10

74 75

76a, 76b

<Step 1> Synthesis of Compound 74

To a mixture of 3-amino-1-propanol (0.38 g), (tert-butoxycarbonyl)glycine (0.88 g), N,N-dimethyl-4-aminopyridine (0.12 g), and dichloromethane (20 mL), a dichloromethane (5 mL) solution of N,N'-dicyclohexylcarbodiimide (1.03

g) was added dropwise under ice cooling. The reaction liquid was stirred at room temperature overnight. To the reaction liquid, a dichloromethane (5 mL) solution of commercially available diclofenac (compound 10, 1.48 g) and N,N'-dicyclohexylcarbodiimide (1.03 g) was added dropwise. The reaction liquid was stirred at room temperature overnight. The reaction liquid was filtered, and the filtrate was removed under reduced pressure to remove the solvent. The residue was purified by silica gel column chromatography (5-100% ethyl acetate/heptane) to obtain compound 74 (1.4 g) as a white amorphous.

$^1$H-NMR (400 MHz, $CDCl_3$) δ 7.34 (2H, d, J = 8 Hz), 7.22 (1H, dd, J = 8, 1 Hz), 7.12 (1H, td, J = 8, 1 Hz), 7.02-6.92 (2H, m), 6.86 (1H, s), 6.54 (1H, d, J = 8 Hz), 6.40 (1H, br), 5.14 (1H, br), 4.21 (2H, t, J = 6 Hz), 3.82 (2H, s), 3.74 (2H, d, J = 6 Hz), 3.30 (2H, q, J = 6 Hz), 1.91-1.82 (2H, m), 1.44 (9H, s) ppm.

<Step 2> Synthesis of Compound 75

A mixture of compound 74 (1.4 g) and 4 N hydrochloric acid-1,4-dioxane solution (14 mL) was stirred at room temperature for 30 minutes. The reaction liquid was filtered under reduced pressure to obtain compound 75 (1.3 g) as a white amorphous.

$^1$H-NMR (400 MHz, DMSO-$d_6$) δ 8.46 (1H, t, J = 5 Hz), 8.07 (3H, br), 7.53 (2H, d, J = 8 Hz), 7.23-7.17 (2H, m), 7.10-7.03 (2H, m), 6.85 (1H, td, J = 7, 1 Hz), 6.25 (1H, d, J = 8 Hz), 4.10 (2H, t, J = 6 Hz), 3.81 (2H, s), 3.51 (2H, s), 3.22-3.15 (2H, m), 1.82-1.72 (2H, m) ppm.

<Step 3-1> Synthesis of Diclofenac-(2-Amino-N-(3-Hydroxypropyl)Acetamide)-Alginic Acid Derivative (Compound 76a)

To a solution obtained by dissolving 1 g of sodium alginate (Mochida Pharmaceutical Co., Ltd., A-2) in water (100 mL) and adding ethanol (30 mL) thereto, 4-(4,6-dimethoxy-1,3,5-triazin-2-yl)-4-methylmorpholinium chloride (305 mg) was added at room temperature. After stirring for 30 minutes, a solution of compound 75 (205 mg) in ethanol (10 mL) and a 1 M sodium hydrogen carbonate aqueous solution (0.46 mL) were added dropwise, and the mixture was stirred at room temperature for 4 hours. A 0.1 g/mL sodium chloride aqueous solution (5 mL) and ethanol (100 mL) were added to the reaction liquid, followed by stirring for 10 minutes. The obtained precipitate was collected by filtration, washed with ethanol, and dried under reduced pressure to obtain the title compound (1.03 g) as a white solid. The drug introduction rate was 13.7 mol%.

<Step 3-2> Synthesis of Diclofenac-(2-Amino-N-(3-Hydroxypropyl)Acetamide)-Alginic Acid Derivative (Compound 76b)

Using 1 g of sodium alginate (Mochida Pharmaceutical Co., Ltd., A-3), the same operation as in (Example 18) <Step 3-1> was performed to obtain the title compound (0.99 g) as a white solid. The drug introduction rate was 14.4 mol%.

(Example 19) Synthesis of Diclofenac-(2-Amino-N-(1-Hydroxypropan-2-Yl)Acetamide)-Alginic Acid Derivative

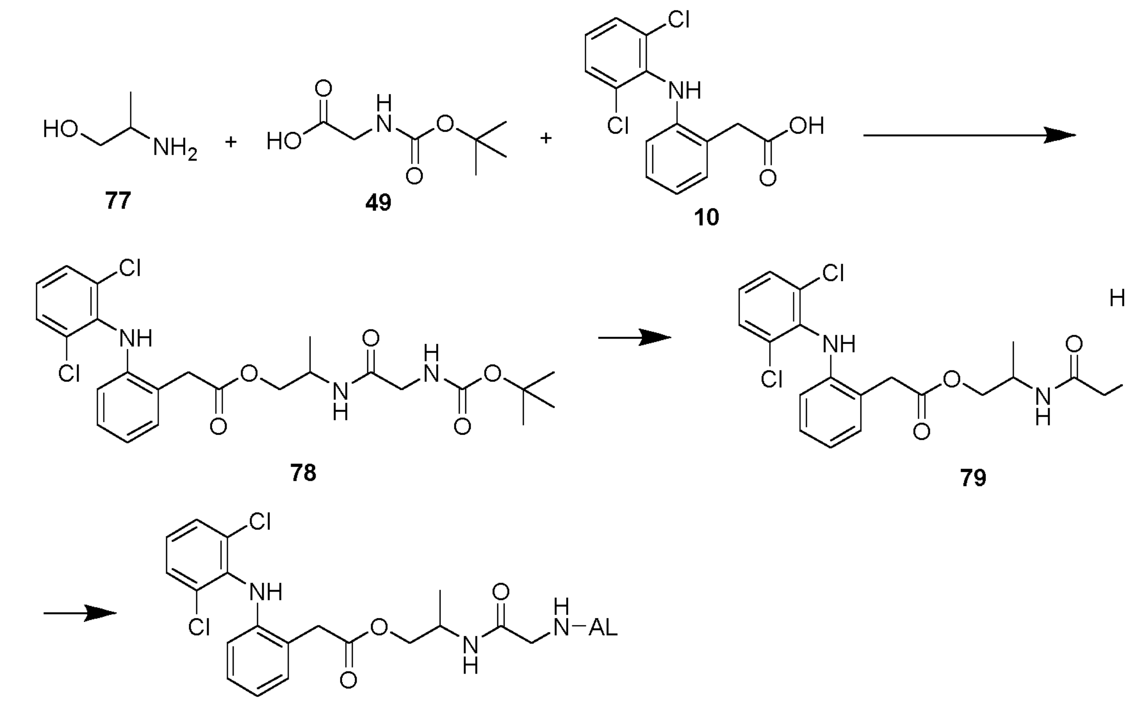

77 49 10

78 79

80a, 80b

<Step 1> Synthesis of Compound 78

To a mixture of 3-amino-1-propanol (0.38 g), (tert-butoxycarbonyl)glycine (0.88 g), N,N-dimethyl-4-aminopyridine (0.12 g), and dichloromethane (20 mL), a dichloromethane (5 mL) solution of N,N'-dicyclohexylcarbodiimide (1.03 g) was added dropwise under ice cooling. The reaction liquid was stirred at room temperature overnight. To the reaction liquid, a dichloromethane (5 mL) solution of commercially available diclofenac (compound 10, 1.48 g) and N,N'-dicyclohexylcarbodiimide (1.03 g) was added dropwise. The reaction liquid was stirred at room temperature overnight. The reaction liquid was filtered, and the filtrate was removed under reduced pressure to remove the solvent. The residue was purified by silica gel column chromatography (5-100% ethyl acetate/heptane) to obtain compound 78 (1.4 g) as a white amorphous.
$^1$H-NMR (400 MHz, $CDCl_3$) $\delta$ 7.34 (2H, d, J = 8 Hz), 7.23 (1H, dd, J = 8, 1 Hz), 7.13 (1H, td, J = 8, 1 Hz), 7.02-6.93 (2H, m), 6.86 (1H, s), 6.55 (1H, d, J = 8 Hz), 6.03 (1H, d, J = 8 Hz), 4.94 (1H, br), 4.37-4.25 (1H, m), 4.22-4.07 (2H, m), 3.89-3.77 (2H, m), 3.72-3.52 (2H, m), 1.44 (9H, s), 1.16 (3H, d, J = 7 Hz) ppm.

<Step 2> Synthesis of Compound 79

A mixture of compound 78 (1.4 g) and 4 N hydrochloric acid-1,4-dioxane solution (14 mL) was stirred at room temperature for 30 minutes. The reaction liquid was filtered under reduced pressure to obtain compound 79 (1.3 g) as a white amorphous.
$^1$H-NMR (400 MHz, DMSO-$d_6$) $\delta$ 8.53 (1H, d, J = 8 Hz), 8.13 (3H, br), 7.53 (2H, d, J = 8 Hz), 7.21 (2H, t, J = 8 Hz), 7.09-7.03 (2H, m), 6.85 (1H, td, J = 7, 1 Hz), 6.25 (1H, d, J = 8 Hz), 4.14-3.99 (3H, m), 3.83 (2H, s), 3.56-3.44 (2H, m), 1.09 (3H, d, J = 7 Hz) ppm.

<Step 3-1> Synthesis of Diclofenac-(2-Amino-N-(1-Hydroxypropan-2-Yl)Acetamide)-Alginic Acid Derivative (Compound 80a)

To a solution obtained by dissolving 1 g of sodium alginate (Mochida Pharmaceutical Co., Ltd., A-2) in water (100 mL) and adding ethanol (30 mL) thereto, 4-(4,6-dimethoxy-1,3,5-triazin-2-yl)-4-methylmorpholinium chloride (305 mg) was added at room temperature. After stirring for 30 minutes, a solution of compound 79 (205 mg) in ethanol (10 mL) and a 1 M sodium hydrogen carbonate aqueous solution (0.46 mL) were added dropwise, and the mixture was stirred at room temperature for 4 hours. A 0.1 g/mL sodium chloride aqueous solution (5 mL) and ethanol (100 mL) were added to the reaction liquid, followed by stirring for 10 minutes. The obtained precipitate was collected by filtration, washed with ethanol, and dried under reduced pressure to obtain the title compound (0.97 g) as a white solid. The drug

introduction rate was 12.2 mol%.

<Step 3-2> Synthesis of Diclofenac-(2-Amino-N-(1-Hydroxypropan-2-Yl)Acetamide)-Alginic Acid Derivative (Compound 80b)

Using 1 g of sodium alginate (Mochida Pharmaceutical Co., Ltd., A-3), the same operation as in (Example 19) <Step 3-1> was performed to obtain the title compound (0.99 g) as a white solid. The drug introduction rate was 14.4 mol%.

(Example 20) Synthesis of Felbinac-(2-Amino-N-(2-Hydroxyethyl)Acetamide)-Alginic Acid Derivative

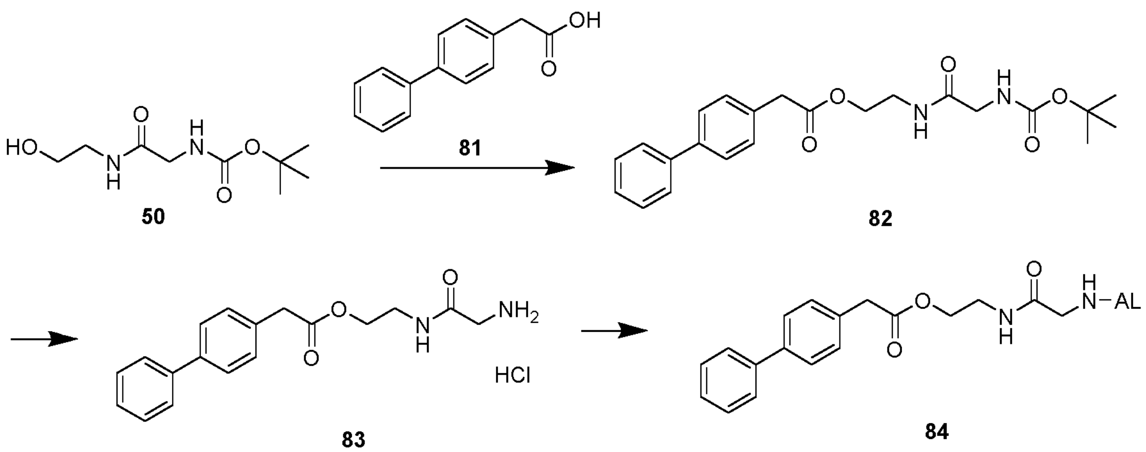

50 81 82 83 84

<Step 1> Synthesis of Compound 82

To a mixture of commercially available felbinac (compound 81, 1.0 g), compound 50 (1.13 g), N,N-dimethyl-4-aminopyridine (0.12 g), and dichloromethane (6 mL), a dichloromethane (4 mL) solution of N,N'-dicyclohexylcarbodiimide (1.07 g) was added dropwise under ice cooling. The reaction liquid was stirred at room temperature for 2 hours. The reaction liquid was filtered using ethyl acetate (50 mL), then washed successively with saturated sodium hydrogen carbonate aqueous solution (20 mL) and water (20 mL), and dried over anhydrous sodium sulfate, and the solvent was removed under reduced pressure. The residue was triturated with ethanol to obtain compound 82 (0.80g) as a white solid.
$^1$H-NMR (400 MHz, $CDCl_3$) δ 7.60-7.54 (4H, m), 7.46-7.40 (2H, m), 7.38-7.31 (3H, m), 6.27 (1H, br), 4.97 (1H, br), 4.21 (2H, t, J = 5 Hz), 3.72 (2H, d, J = 6 Hz), 3.68 (2H, s), 3.58-3.52 (2H, m), 1.45 (9H, s) ppm.

<Step 2> Synthesis of Compound 83

A mixture of compound 82 (0.80 g) and 4 N hydrochloric acid-1,4-dioxane solution (10 mL) was stirred at room temperature for 30 minutes. The reaction suspension was filtered, and the solid collected by filtration was triturated with dimethoxyethane to obtain compound 83 (0.56 g) as a white solid.
$^1$H-NMR (400 MHz, DMSO-$d_6$) δ 8.68 (1H, br), 8.14 (3H, br), 7.67-7.60 (4H, m), 7.49-7.43 (2H, m), 7.39-7.33 (3H, m), 4.10 (2H, t, J = 6 Hz), 3.74 (2H, s), 3.55 (2H, s), 3.46-3.37 (2H, m) ppm.

<Step 3> Synthesis of Felbinac-(2-Amino-N-(2-Hydroxyethyl)Acetamide)-Alginic Acid Derivative (Compound 84)

To a solution obtained by dissolving 200 mg of sodium alginate (Mochida Pharmaceutical Co., Ltd., A-2) in water (20 mL) and adding ethanol (8 mL) thereto, 4-(4,6-dimethoxy-1,3,5-triazin-2-yl)-4-methylmorpholinium chloride (46 mg) was added at room temperature. After stirring for 10 minutes, a solution of compound 83 (32 mg) in ethanol (2 mL)-water (1 mL) and a 1 M sodium hydrogen carbonate aqueous solution (0.09 mL) were added dropwise, and the mixture was stirred at room temperature for 4 hours. A 0.1 g/mL sodium chloride aqueous solution (2 mL) and ethanol (40 mL) were added to the reaction liquid, followed by stirring for 10 minutes. The obtained precipitate was collected by filtration, washed with ethanol, and dried under reduced pressure to obtain the title compound (193 mg) as a white solid. The drug introduction rate was 10.8 mol%.

(Example 21) Synthesis of Ketoprofen-(2-Amino-N-(2-Hydroxyethyl)Acetamide)-Alginic Acid Derivative

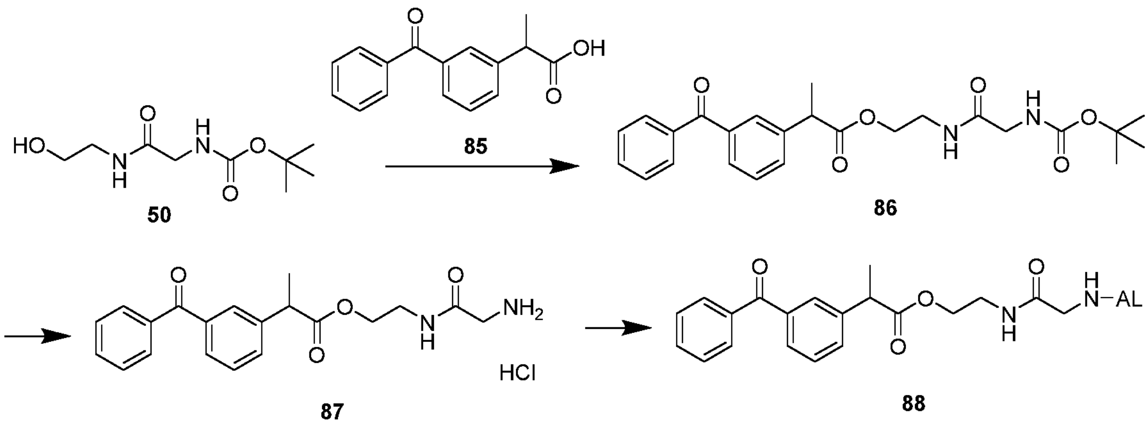

<Step 1> Synthesis of Compound 86

To a mixture of commercially available ketoprofen (compound 85, 1.0 g), compound 50 (0.94 g), N,N-dimethyl-4-aminopyridine (0.1 g), and dichloromethane (10 mL), 1-(3-dimethylaminopropyl)-3-ethylcarbodiimide hydrochloride (0.83 g) was added at room temperature. The reaction liquid was stirred at room temperature for 3 hours. The reaction liquid was diluted with ethyl acetate (50 mL), washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (5-100% ethyl acetate/heptane) to obtain compound 86 (1.38 g) as a colorless gum-like matter.
$^1$H-NMR (400 MHz, $CDCl_3$) $\delta$ 7.84-7.77 (3H, m), 7.65-7.58 (2H, m), 7.55-7.42 (4H, m), 6.50 (1H, br), 5.28 (1H, br), 4.27-4.13 (2H, m), 3.74 (2H, d, J = 6 Hz), 3.58-3.41 (3H, m), 1.56 (3H, d, J = 7 Hz), 1.43 (9H, s) ppm.

<Step 2> Synthesis of Compound 87

A mixture of compound 86 (1.38 g) and 4 N hydrochloric acid-1,4-dioxane solution (15 mL) was stirred at room temperature for 30 minutes. The solvent was removed from the reaction liquid under reduced pressure to obtain compound 87 (1.27 g) as a white amorphous.
$^1$H-NMR (400 MHz, DMSO-$d_6$) $\delta$ 8.67 (1H, t, J = 6 Hz), 8.19 (3H, br), 7.76-7.51 (9H, m), 4.17-4.10 (1H, m), 4.04-3.92 (2H, m), 3.51 (2H, s), 3.44-3.27 (2H, m), 1.45 (3H, d, J = 7 Hz) ppm.

<Step 3> Synthesis of Ketoprofen-(2-Amino-N-(2-Hydroxyethyl)Acetamide)-Alginic Acid Derivative (Compound 88)

To a solution obtained by dissolving 200 mg of sodium alginate (Mochida Pharmaceutical Co., Ltd., A-2) in water (20 mL) and adding ethanol (6 mL) thereto, 4-(4,6-dimethoxy-1,3,5-triazin-2-yl)-4-methylmorpholinium chloride (46 mg) was added at room temperature. After stirring for 10 minutes, a solution of compound 87 (36 mg) in ethanol (2 mL) and a 1 M sodium hydrogen carbonate aqueous solution (0.09 mL) were added dropwise, and the mixture was stirred at room temperature for 4 hours. A 0.1 g/mL sodium chloride aqueous solution (2 mL) and ethanol (40 mL) were added to the reaction liquid, followed by stirring for 10 minutes. The obtained precipitate was collected by filtration, washed with ethanol, and dried under reduced pressure to obtain the title compound (201 mg) as a white solid. The drug introduction rate was 11.9 mol%.

(Example 22) Synthesis of Naproxen-(2-Amino-N-(2-Hydroxyethyl)Acetamide)-Alginic Acid Derivative

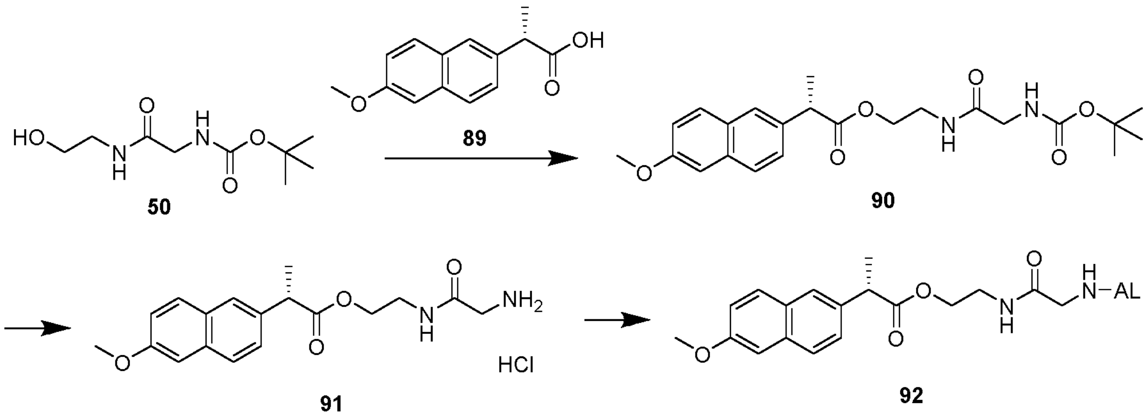

50 89 90 91 92

<Step 1> Synthesis of Compound 90

To a mixture of commercially available naproxen (compound 89, 1.0 g), compound 50 (1.04 g), N,N-dimethyl-4-aminopyridine (0.11 g), and dichloromethane (10 mL), 1-(3-dimethylaminopropyl)-3-ethylcarbodiimide hydrochloride (0.92 g) was added at room temperature. The reaction liquid was stirred at room temperature for 3 hours. The reaction liquid was diluted with ethyl acetate (50 mL), washed successively with saturated sodium hydrogen carbonate aqueous solution (20 mL) and water (20 mL), and dried over anhydrous sodium sulfate, and the solvent was removed under reduced pressure. The residue was purified by silica gel column chromatography (5-100% ethyl acetate/heptane) to obtain compound 90 (1.39 g) as a colorless gum-like matter.
$^1$H-NMR (400 MHz, $CDCl_3$) δ 7.72 (2H, dd, J = 8, 4 Hz), 7.66 (1H, d, J = 1 Hz), 7.39 (1H, dd, J = 8, 2 Hz), 7.17-7.10 (2H, m), 5.97 (1H, br), 4.79 (1H, br), 4.23-4.08 (2H, m), 3.92 (3H, s), 3.86 (1H, q, J = 7 Hz), 3.56-3.37 (4H, m), 1.58 (3H, d, J = 7 Hz), 1.45 (9H, s) ppm.

<Step 2> Synthesis of Compound 91

A mixture of compound 90 (1.39 g) and 4 N hydrochloric acid-1,4-dioxane solution (15 mL) was stirred at room temperature for 30 minutes. The solvent was removed from the reaction liquid under reduced pressure to obtain compound 91 (1.28 g) as a white amorphous.
$^1$H-NMR (400 MHz, DMSO-$d_6$) δ 8.61 (1H, t, J = 5 Hz), 8.13 (3H, br), 7.79 (2H, t, J = 9 Hz), 7.72 (1H, d, J = 2 Hz), 7.40 (1H, dd, J = 9, 2 Hz), 7.29 (1H, d, J = 2 Hz), 7.15 (1H, dd, J = 8, 2 Hz), 4.17-4.09 (1H, m), 4.02-3.88 (2H, m), 3.86 (3H, s), 3.50 (2H, s), 3.43-3.26 (2H, m), 1.49 (3H, d, J = 7 Hz) ppm.

<Step 3> Synthesis of Ketoprofen-(2-Amino-N-(2-Hydroxyethyl)Acetamide)-Alginic Acid Derivative (Compound 92)

To a solution obtained by dissolving 200 mg of sodium alginate (Mochida Pharmaceutical Co., Ltd., A-2) in water (20 mL) and adding ethanol (6 mL) thereto, 4-(4,6-dimethoxy-1,3,5-triazin-2-yl)-4-methylmorpholinium chloride (46 mg) was added at room temperature. After stirring for 10 minutes, a solution of compound 91 (34 mg) in ethanol (2 mL) and a 1 M sodium hydrogen carbonate aqueous solution (0.09 mL) were added dropwise, and the mixture was stirred at room temperature for 4 hours. A 0.1 g/mL sodium chloride aqueous solution (2 mL) and ethanol (40 mL) were added to the reaction liquid, followed by stirring for 10 minutes. The obtained precipitate was collected by filtration, washed with ethanol, and dried under reduced pressure to obtain the title compound (196 mg) as a white solid. The drug introduction rate was 12.9 mol%.

(Example 23) Diclofenac Release Test Using Compounds Prepared in Example 12 to Example 19, Felbinac Release Test Using Compound Prepared in Example 20, Ketoprofen Release Test Using Compound Prepared in Example 21, and Naproxen Release Test Using Compound Prepared in Example 22

Similar to the method of (Example 10), the release rate was calculated by measuring the amount of free drug of diclofenac, felbinac, ketoprofen, and naproxen in a sodium phosphate buffer at pH = 5.3 or pH = 7.0.

The LC conditions are as follows.
Temperature: 40°C
Flow rate: 0.7 mL/min
Column: ODS-4: 3 μm (2.1 × 30 mm)
Solvent: (A) 0.1% formic acid aqueous solution, (B) 100% acetonitrile
Gradient:

Table 9

| Time (min) | %B |
|---|---|
| 0 | 45 |
| 1.5 | 98 |
| 2.5 | 98 |
| 2.5 | 45 |
| 3.3 | 45 |

The MS conditions are as follows.

Ionization mode: ESI-negative

Ion source temperature: 300 degrees

Capillary voltage: -4000 V

Release Rate in Release Test at pH 7.0

Table 10

| Compound Number | 14a | 14a | 53a | 53b | 53c | 53d |
|---|---|---|---|---|---|---|
| dayl | 0.6 | 0.6 | 0.9 | 0.9 | 0.9 | 0.7 |
| day3 | 1.9 | 1.9 | 3.7 | 2.3 | 2.5 | 3.0 |
| day7 | 4.2 | 4.2 | 10.0 | 6.4 | 7.4 | 9.3 |

Release Rate in Release Test at pH 7.0

Table 11

| Compound Number | 58 | 63a | 63b | 68 | 72a | 72b |
|---|---|---|---|---|---|---|
| dayl | 0.3 | 0.3 | 0.7 | 0.3 | 0.3 | 0.4 |
| day3 | 1.3 | 0.8 | 1.9 | 0.9 | 1.0 | 1.5 |
| day7 | 3.1 | 2.2 | 2.5 | 2.1 | 2.2 | 3.7 |

Release Rate in Release Test at pH 7.0

Table 12

| Compound Number | 76a | 76b | 80a | 80b | 84 | 88 | 92 |
|---|---|---|---|---|---|---|---|
| dayl | 0.5 | 0.4 | 0.8 | 0.7 | 0.9 | 0.4 | 0.2 |
| day3 | 1.3 | 1.4 | 2.4 | 2.3 | 3.3 | 1.4 | 0.8 |
| day7 | 3.5 | 3.6 | 5.3 | 5.6 | 7.3 | 3.4 | 1.7 |

Release Rate in Release Test at pH 5.3

Table 13

| Compound Number | 14a | 14b | 53a | 53b | 53c | 53d |
|---|---|---|---|---|---|---|
| dayl | 0.1 | 0.1 | 0.1 | 0.6 | 0.5 | 0.1 |

(continued)

| Compound Number | 14a | 14b | 53a | 53b | 53c | 53d |
|---|---|---|---|---|---|---|
| day3 | 0.2 | 0.2 | 0.4 | 0.8 | 0.9 | 0.5 |
| day7 | 0.4 | 0.5 | 0.8 | 1.0 | 1.0 | 0.6 |

Release Rate in Release Test at pH 5.3

Table 14

| Compound Number | 58 | 63a | 63b | 68 | 72a | 72b |
|---|---|---|---|---|---|---|
| dayl | 0.0 | 0.0 | 0.1 | 0.0 | 0.0 | 0.0 |
| day3 | 0.2 | 0.2 | 0.1 | 0.2 | 0.1 | 0.1 |
| day7 | 0.3 | 0.2 | 0.2 | 0.3 | 0.2 | 0.2 |

Release Rate in Release Test at pH 5.3

Table 15

| Compound Number | 76a | 76b | 80a | 80b | 84 | 88 | 92 |
|---|---|---|---|---|---|---|---|
| dayl | 0.1 | 0.3 | 0.2 | 0.2 | 0.2 | 0.1 | 0.2 |
| day3 | 0.2 | 0.5 | 0.4 | 0.3 | 0.3 | 0.2 | 0.1 |
| day7 | 0.4 | 0.6 | 0.6 | 0.7 | 0.5 | 0.3 | 0.2 |

(Example 24)

Effect of Compound 53b Obtained in (Example 13) by Intra-Articular Administration on Rat 1% Silver Nitrate-Induced Pain Model

(1) Administration of Pain-Inducing Substance

Inhalation anesthesia of isoflurane was used as a general anesthetic.
Rats (Crj: SD system (SPF), male, 6 weeks of age) were anesthetized, and a 1% silver nitrate solution was administered into the left hindlimb knee joint cavity at a dose of 50 μL/joint.

(2) Administration of Test Substances

The following test substances were prepared.

- Vehicle (VH): 5% glucose solution using 10 mM phosphate buffer (PB) as a solvent
- 0.9 mg/mL Diclofenac Na in Vehicle (DF)
- 0.9% Na alginate (Mochida Pharmaceutical Co., Ltd., A-2) in Vehicle (ALG)
- 0.9 mg/mL diclofenac Na + 0.9% Na alginate (Mochida Pharmaceutical Co., Ltd., A-2) in Vehicle (DF & ALG)
- 0.9% solution of compound 53b obtained in Example 13 (DF-ALG) (conjugate)

The gait state of the rats on the day after the model was created was evaluated by scoring. The rats were divided into groups based on the obtained scores, and under inhalation anesthesia with isoflurane, each test substance was administered at 0.1 mL/kg into the rat left hindlimb knee joint (n = 8).

(3) Evaluation Method

The gait score was given once a day for 5 days from the day on which test substance was administrated. Note

that, as the score on the first day, the score before administration (grouping) was used. Under the blind, the gait state of each group was visually observed using the following pain score table based on the gait state. Table 16 presents the results.

The results are shown by the average pain score on Table 16.

0: Normal
1: mild claudication with lifting the foot
2: severe claudication with completely closing the toe
3: walking on three legs

From Table 16, the degree of pain relief (recovery from pain) was faster in the DF-ALG (compound 53b) administration group than in the diclofenac administration group, the alginic acid administration group, the diclofenac + alginic acid administration group, and the like.

Table 16

| | Day 1 | Day 2 | Day 3 | Day 4 | Day 5 |
|---|---|---|---|---|---|
| VH | 2.4 | 2.5 | 1.9 | 1.4 | 0.8 |
| DF | 2.5 | 1.9 | 1.9 | 1.0 | 0.8 |
| ALG | 2.5 | 1.9 | 1.5 | 0.9 | 0.5 |
| DF&ALG | 2.5 | 2.0 | 1.4 | 1.0 | 0.4 |
| DF-ALG | 2.5 | 1.0 | 0.9 | 0.5 | 0.4 |

(Example 25) Study on Sustained Release of Diclofenac-Introduced Alginic Acid in Rabbit Knee Joint

(1) Method of Administering Test Substances

The following test substances were prepared.

- 0.9% solution of compound 53b obtained in Example 13 (solvent: 10 mM phosphate buffer containing 5% glucose) (DF-ALG-53b)
- 0.9% solution of compound 63a obtained in Example 15 (solvent: 10 mM phosphate buffer containing 5% glucose and 3% HP-β-CD) (DF-ALG-63a)

For each test substance, 4 rabbits were used, the whole body was fixed with a towel under no anesthesia, the area around the left knee joint was wiped with alcohol, and then 0.1 mL/kg of each of the above test substances was administered into the joint cavity from the outside of the rabbit knee using a Terumo 1 mL syringe equipped with a 26 G injection needle (manufactured by Terumo). Necropsy was performed 3 days, 7 days, 14 days, 28 days, 56 days, and 84 days after administration of the test substance.

(2) Method of Measuring Amount of Free Diclofenac (DF) in Synovial Fluid

Rabbits were exsanguinated and killed under combined anesthesia by intramuscular administration of ketamine hydrochloride and xylazine. The joint capsule was incised just below the patella to expose the knee joint cavity, and the inside of the joint cavity was washed with 2 mL of physiological saline using a catheter with a Surflo indwelling needle 20G, and the synovial fluid mixed with physiological saline was collected. The amount of DF in the recovered synovial fluid was measured by the following procedure.

To the synovial fluid (0.05 mL), 1 N HCl (0.02 mL) was added, and the mixture was sufficiently stirred and then incubated at room temperature for 30 minutes or more. Moreover, 0.12 mL of 0.1% formic acid aqueous solution and 0.01 mL of internal standard solution (methanol solution having a Celecoxib concentration of 0.1 μg/mL) were added and sufficiently stirred, followed by centrifugation at 450 G for 5 minutes. The whole amount of this supernatant was added to Oasis (registered trademark) PRiME HLB μ-Elution Plate (extraction column) equilibrated with 0.2 mL of methanol and 0.2 mL of purified water, and the added sample was sucked with an air pump. The extraction column was washed with 0.02 mL of a 5% methanol aqueous solution, and the drug was eluted from the extraction column with 0.025 mL of acetonitrile (this elution operation was repeated twice). To the collected eluate, 0.05 μL of 0.1% formic acid

aqueous solution was added, and the amount of free diclofenac was measured by LC-MS/MS (the amount of free diclofenac in the synovial fluid).

(3) Method of Measuring Diclofenac (DF) Concentration in Synovium

The synovial tissue was separated and collected from the knee joint after collecting the synovial fluid in (2) above. The collected synovial tissue was washed with physiological saline to remove the attached synovial fluid. After removing the patella, the synovial tissue was placed in a tube, and the tissue was cut into small pieces with scissors. About 50 mg of synovial tissue was taken in a tube containing stainless beads, 19 times the amount of purified water was added, and the mixture was homogenized using a bead type homogenizer. To the homogenate (0.1 mL), 1 N HCl (0.02 mL) was added, and the mixture was sufficiently stirred and then incubated at room temperature for 30 minutes or more. Moreover, 0.07 mL of 0.1% formic acid aqueous solution and 0.01 mL of internal standard solution (methanol solution having a Celecoxib concentration of 0.1 μg/mL) were added and sufficiently stirred, followed by centrifugation at 450 G for 5 minutes. The whole amount of this supernatant was added to Oasis (registered trademark) PRiME HLB μ-Elution Plate (extraction column) equilibrated with 0.2 mL of methanol and 0.2 mL of purified water, and the added sample was sucked with an air pump. The same extraction operation as in (2) above was performed, and the amount of free diclofenac was measured using LC-MS/MS. Table 17 presents the results.

In the case of administering the substances of the present invention (DF-ALG-53b) and (DF-ALG-63a) (conjugates) as test substances, the presence of free diclofenac was observed in the synovial tissues 56 to 84 days after the administration, and it was speculated that diclofenac was continuously present at the administration site, exhibiting a sustained effect (Table 17: DF concentration in synovial tissue (ng/g)).

It is speculated that the pain occurring in the joint is caused by synovitis, and it is considered that, when NSAIDs are administered into the joint cavity, the NSAIDs are rapidly transferred to the synovium and exhibit an analgesic·anti-inflammatory action. Therefore, it is considered that the maintenance of NSAIDs concentration in the synovium due to the sustained release effect is greatly related to the sustained effect of pain suppression.

Table 17

| Compound Administered | Day 3 | Day 7 | Day 14 | Day 28 | Day 56 | Day 84 |
|---|---|---|---|---|---|---|
| DF-ALG-53b | 132 | 84 | 106 | 123 | 23 | 21 |
| DF-ALG-63a | 33 | 20 | 25 | 36 | 14 | 21 |

From Table 17 above, in the case of administering (DF-ALG-53b) and (DF-ALG-63a) being DF-ALG (conjugates), the DF was retained in the synovial tissue continuously until 84 days after administration, suggesting that it is effective as a sustained-release formulation of DF.

In particular, as compared with the DF concentration in synovium (about 10 ng/g (day 28), < 5 ng/g (day 35)) (see Fig. 7a in the literature) upon administration of DF-HA (conjugate) described in the related art (BMC Musculoskeletal Disorders (2018) 19: P 157 and later), the concentration of diclofenac in the synovium upon administration of DF-ALG (conjugate) is high even 84 days after administration, and it is expected that the pain suppressing effect for a long period (for example, 2 to 3 months) will continue.

<<Molecular Weight Measurement Results>>

Results of Molecular Weight Measurement of Alginic Acid Derivatives

Table 18

| Example Number | Weight Average Molecular Weight (Da) |
|---|---|
| Example 1 (Compound 5a) | 1.08 Million |
| Example 1 (Compound 5b) | 1.72 Million |
| Example 1 (Compound 5c) | 1.81 Million |
| Example 1 (Compound 5d) | 1.61 Million |
| Example 2 (Compound 9) | 1.52 Million |

(continued)

| Example Number | Weight Average Molecular Weight (Da) |
|---|---|
| Example 3 (Compound 14) | 1.72 Million |
| Example 4 (Compound 19) | 1.57 Million |
| Example 5 (Compound 24) | 1.63 Million |
| Example 6 (Compound 28) | 1.37 Million |
| Example 7 (Compound 32) | 1.62 Million |
| Example 8 (Compound 36) | 1.38 Million |
| Example 9 (Compound 42) | 1.75 Million |
| Example 10 (Compound 47) | 1.70 Million |
| Example 12 (Compound 14a) | 1.57 Million |
| Example 12 (Compound 14b) | 1.71 Million |
| Example 13 (Compound 53a) | 0.86 Million |
| Example 13 (Compound 53b) | 1.53 Million |
| Example 13 (Compound 53c) | 1.76 Million |
| Example 13 (Compound 53d) | 1.17 Million |
| Example 14 (Compound 58) | 1.74 Million |
| Example 15 (Compound 63a) | 1.50 Million |
| Example 15 (Compound 63b) | 1.71 Million |
| Example 16 (Compound 68) | 1.44 Million |
| Example 17 (Compound 72a) | 1.55 Million |
| Example 17 (Compound 72b) | 1.75 Million |
| Example 18 (Compound 76a) | 1.48 Million |
| Example 18 (Compound 76b) | 1.74 Million |
| Example 19 (Compound 80a) | 1.54 Million |
| Example 19 (Compound 80b) | 1.74 Million |
| Example 20 (Compound 84) | 1.57 Million |
| Example 21 (Compound 88) | 1.63 Million |
| Example 22 (Compound 92) | 1.59 Million |

Results of Molecular Weight Measurement of Raw Material Alginic Acid

Table 19

| Sample | Weight Average Molecular Weight (Da) |
|---|---|
| A1 | 0.855 Million |
| A2 | 1.69 Million |
| A3 | 1.78 Million |
| A4 | 1.64 Million |

From the results of the above release tests (in vitro tests) and animal experiments (in vivo tests), it has been found that the sustained release rate can be adjusted by the structure of the linker, and the derivatives of the present invention can be expected to have a long-term sustainable analgesic action and anti-inflammatory action by adjusting

the type of the linker and the drug introduction rate.

Specifically, in the release test at pH 7.0, the confirmed release rate of compound 5a obtained in Example 1 was 2.3% on day 3 and 4.1% on day 7. Similarly, compound 5b, compound 5c, compound 5d, compound 42, and compound 47 were also stably released. Moreover, in the release test at pH 7.0, the confirmed release rate of compound 53b obtained in Example 13 was 3.7% on day 3 and 10.0% on day 7. In addition, in the release test at pH 7.0, the confirmed release rate of compound 63a obtained in Example 15 was 0.8% on day 3 and 2.2% on day 7. In any of the compounds, the day counts and the release rate are almost proportional to each other, and therefore stable and sustained release can be expected.

In addition, the inflammatory site is generally acidic, and the pH may vary between neutral and weakly acidic, so that the sustained release rate may vary accordingly. However, by adjusting the structure of the linker, it is possible to form an analgesic·anti-inflammatory agent having a stable sustained release action even when the pH varies.

Moreover, from the results of the amount of diclofenac in the rabbit knee joint synovium of the compounds 53b and 63a was measured, the released diclofenac in the synovium at day 28, day 56, and day 84 were observed as presented in Table 17 above.

It is known that the pain suppressing effect depends on the amount of diclofenac in the synovium, and at day 28, day 56, and day 84, both compounds 53b and 63a were able to be maintained above the minimum amount (5 ng/g) for exhibiting the pain suppressing effect. From this, it can be expected that the nonsteroidal anti-inflammatory compound-bound alginic acid derivative of the present invention, especially the diclofenac-bound alginic acid derivative, will continue to suppress pain for a long period of time (for example, 2 to 3 months).

Note that, from the results of the above release tests (in vitro tests) and animal experiments (in vivo tests), it can be expected that the nonsteroidal anti-inflammatory compound-bound alginic acid derivative will continuously suppress pain for 2 to 3 months, if the release rate on day 7 in the release test is preferably about 2%.

## Claims

1. A water-soluble alginic acid derivative, comprising a structure in which alginic acid or a salt thereof and a nonsteroidal anti-inflammatory compound are covalently bonded through a linker.

2. The water-soluble alginic acid derivative according to claim 1, which comprises a structure represented by the following formula (1):

$$\text{(D)-L-(A)} \qquad (1)$$

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound; and
L is a linker having a functional group capable of binding to (A) via an amide bond and having a functional group capable of binding to (D) via an ester bond.

3. The water-soluble alginic acid derivative according to claim 1, which comprises a structure represented by the following formula (2):

$$\text{(A)-NH-}(CH_2)_{n1}\text{-}[X^1]_{n2}\text{-}(CR^1R^2)_{n3}\text{-}[Y]_{n4}\text{-}(CH_2)_{n5}\text{-}(CR^3R^4)_{n6}\text{-}[X^2]_{n7}\text{-}(CH_2)_{n8}\text{-[Z]-(D)} \qquad (2)$$

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound;
$X^1$ and $X^2$ represent hetero atoms;
$R^1$, $R^2$, $R^3$, and $R^4$ each independently represent hydrogen, a halogen atom, a $C_{1-10}$ alkyl group, a $C_{1-10}$ alkoxy group, or a $C_{1-10}$ alkoxycarbonyl group; or $R^1$ and $R^2$ or $R^3$ and $R^4$ together form =O;
Y represents a cycloalkane ring, an aromatic ring, or a heterocycle, wherein the cycloalkane ring, the aromatic ring, or the heterocycle may be substituted with halogen atom(s) or $C_{1-10}$ alkyl group(s);
Z represents O or C(=O) for forming an ester bond with (D); and

n1 represents any integer of 0 to 10; and n2 to n8 independently represent any integer of 0 to 3, but not all of n1 to n8 are 0.

**4.** The water-soluble alginic acid derivative according to claim 1, which is represented by the following formula (2a):

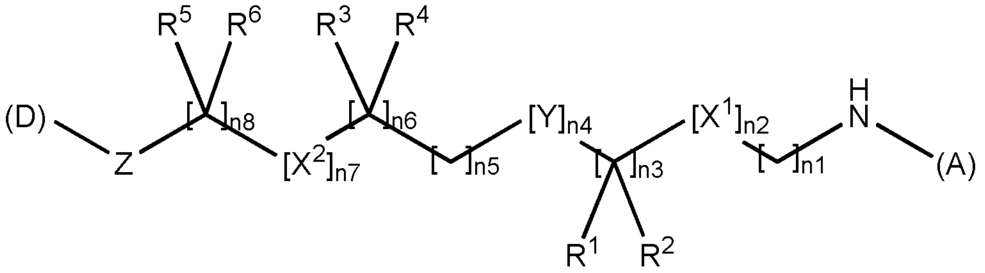

(2a)

wherein

(A) represents one residue derived from alginic acid or the salt thereof which has a C(=O)- group in a monosaccharide of either L-guluronic acid or D-mannuronic acid constituting alginic acid;
(D) represents one residue of the nonsteroidal anti-inflammatory compound;
$X^1$ and $X^2$ are hetero atoms;
$R^1$, $R^2$, $R^3$, $R^4$, $R^5$, and $R^6$ are each independently a group selected from a hydrogen atom, a halogen atom, a $C_{1-6}$ alkyl group, a $C_{1-6}$ alkoxy group, or a $C_{1-6}$ alkoxycarbonyl group ($R^1$ and $R^2$, $R^3$ and $R^4$, or $R^5$ and $R^6$ can together form an oxo group (=O));
Y is a $C_{3-8}$ cycloalkyl ring, a $C_{6-10}$ aryl ring, or a heterocycle, wherein the $C_{3-8}$ cycloalkyl ring, the $C_{6-10}$ aryl ring, or the heterocycle may be substituted with 1 to 3 halogen atoms or $C_{1-6}$ alkyl groups;
Z is an oxygen atom or a carbonyl group;
n1 or n8 is any integer of 0 to 10;
n3, n5, or n6 are independently any integer of 0, 1, 2, or 3;
n2, n4, or n7 are independently any integer of 0 or 1; and
not all of nl to n8 are 0.

**5.** The water-soluble alginic acid derivative according to any one of claims 1 to 3, wherein the nonsteroidal anti-inflammatory compound has a carboxyl group, and the carboxyl group is bonded to a linker.

**6.** The water-soluble alginic acid derivative according to claim 1, 2, or 4, wherein the nonsteroidal anti-inflammatory compound has a carboxyl group, and the carboxyl group is bonded to a linker, wherein the linker is represented by the following formula (LKA-1) [excluding a left side of a broken line in the formula]:

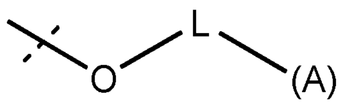

(LKA-1)

wherein the definitions of -L- and (A) are the same as those defined in claim 2; or represented by a formula (LKA-2) [excluding a left side of a broken line in the formula] :

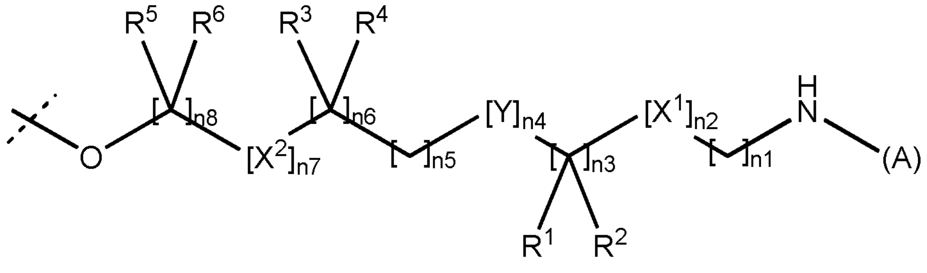

(LKA-2)

wherein the definitions of (A), $R^1$, $R^2$, $R^3$, $R^4$, $R^5$, $R^6$, $X^1$, $X^2$, Y, n1, n2, n3, n4, n5, n6, n7, and n8 are the same as

those defined in claim 4.

7. The water-soluble alginic acid derivative according to any one of claims 1 to 6, wherein the nonsteroidal anti-inflammatory compound is a salicylic acid-based, propionic acid-based, or phenylacetic acid-based nonsteroidal anti-inflammatory drug (NSAID), and a carboxyl group of the NSAID is bonded to the linker represented by formula (LKA-1) or formula (LKA-2) according to claim 4.

8. The water-soluble alginic acid derivative according to claim 7, wherein the nonsteroidal anti-inflammatory compound is a phenylacetic acid-based nonsteroidal anti-inflammatory drug (NSAID) or a propionic acid-based nonsteroidal anti-inflammatory drug (NSAID).

9. The water-soluble alginic acid derivative according to claim 8, wherein the nonsteroidal anti-inflammatory compound is selected from diclofenac, felbinac, ketoprofen, and naproxen.

10. The water-soluble alginic acid derivative according to any one of claims 1 to 9, wherein the introduction rate (mol%) of the nonsteroidal anti-inflammatory compound is at least 1.0 mol% or more.

11. A water-soluble alginic acid derivative gel obtained by cross-linking the water-soluble alginic acid derivative according to any one of claims 1 to 10.

12. A sustained-release pharmaceutical composition, comprising the water-soluble alginic acid derivative according to any one of claims 1 to 10 or the water-soluble alginic acid derivative gel according to claim 11.

13. The sustained-release pharmaceutical composition according to claim 12, as an arthritis therapeutic agent.

14. Use of the water-soluble alginic acid derivative according to any one of claims 1 to 10 or the water-soluble alginic acid derivative gel according to claim 11, for sustained release of a nonsteroidal anti-inflammatory compound.

15. A water-soluble alginic acid derivatives listed below, wherein (A) is one residue derived from alginic acid or the salt thereof which has a C(=O)- group in a monosaccharide of either L-guluronic acid or D-mannuronic acid constituting alginic acid:

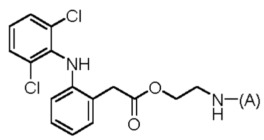
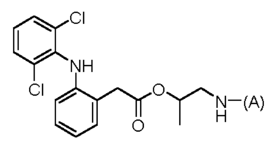
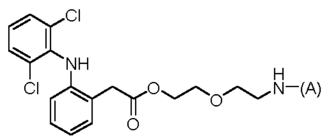

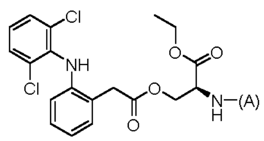
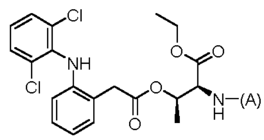
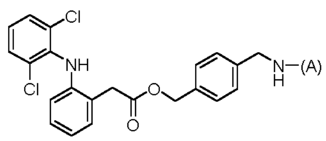

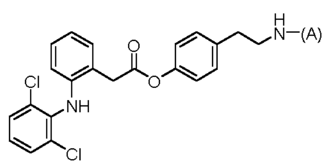
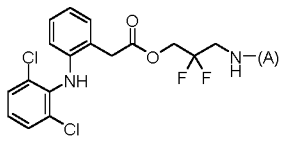
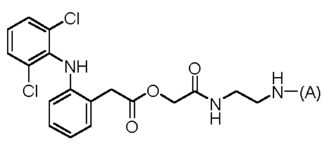

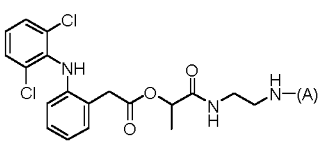
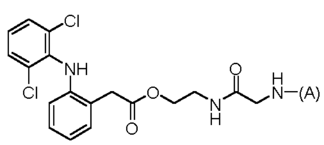
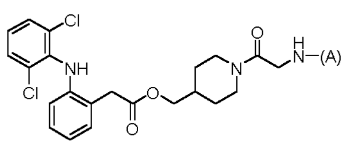

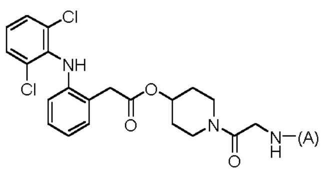
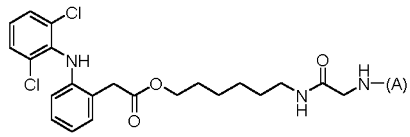
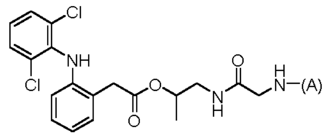

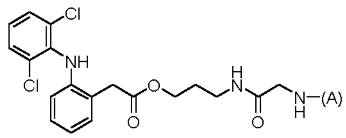
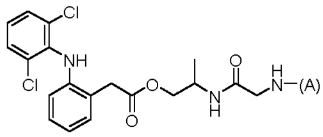
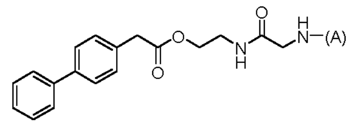

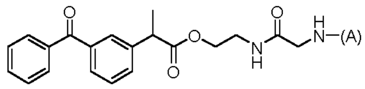
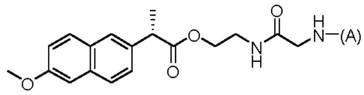

**16.** An amino compound represented by the following formula (AM), a salt thereof, or a solvate thereof:

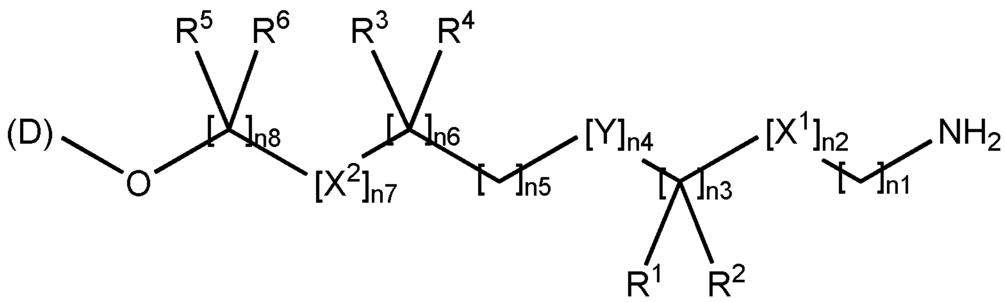
(AM)

wherein the definitions of (D), $X^1$, $X^2$, $R^1$, $R^2$, $R^3$, $R^4$, $R^5$, $R^6$, Y, Z, n1, n2, n3, n4, n5, n6, n7, and n8 are the same as those defined in claim 4.

## INTERNATIONAL SEARCH REPORT

International application No.
PCT/JP2019/011715

A. CLASSIFICATION OF SUBJECT MATTER
Int.Cl. C08B37/04(2006.01)i, A61K9/08(2006.01)i, A61K31/196(2006.01)i, A61K45/00(2006.01)i, A61K47/36(2006.01)i, A61K47/54(2017.01)i, A61K47/61(2017.01)i, A61P19/02(2006.01)i, A61P29/00(2006.01)i
According to International Patent Classification (IPC) or to both national classification and IPC

B. FIELDS SEARCHED
Minimum documentation searched (classification system followed by classification symbols)
Int.Cl. C08B37/04, A61K9/08, A61K31/196, A61K45/00, A61K47/36, A61K47/54, A61K47/61, A61P19/02, A61P29/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2005/026214 A1 (SEIKAGAKU CORPORATION) 24 March 2005, entire text (in particular, preparation example 1) & JP 2011-102405 A & US 2007/0009579 A1, synthesis example 1 & US 2012/0055780 A1 & EP 1666503 A1 & CN 1849341 A | 1-14<br>15 |
| Y<br>A | WO 2007/004675 A1 (SEIKAGAKU CORPORATION) 11 January 2007, entire text (in particular, examples 2-6, 8) & US 2009/0118348 A1, examples 2-6, 8 & EP 1905456 A1 & CN 101217982 A | 1-14<br>15 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 June 2019 (14.06.2019) | 25 June 2019 (25.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/011715

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2005/066214 A1 (SEIKAGAKU CORPORATION) 21 July 2005, entire text, (in particular, examples 1-41) & US 2008/0221062 A1, examples 1-41 & US 2011/0083991 A1 & EP 1710257 A1 & EP 2497785 A2 & EP 3363463 A2 & EP 2754454 A2 & CN 1930190 A & CN 101921347 A & KR 10-1142583 B1 | 1-14<br>15 |
| Y<br>A | JP 04-505334 A (HARBOE, Elin) 17 September 1992, entire text (in particular, claim 1) & WO 1989/008119 A1, claim 1 & EP 0331471 A1 | 1-14<br>15 |
| Y<br>A | WERMUTH C. G., 最新創薬化学下巻, 株式会社テクノミック, 25 September 1999, pp. 321-338, ("The Practice of Medicinal Chemistry last volume", TECHNOMICS, INC.) | 1-14<br>15 |
| A | WO 2013/155375 A1 (ACADEMIA SINICA) 17 October 2013, entire text (in particular, intermediate compound illustrated in fig. 19, 20) & JP 2015-516394 A & US 2013/0274229 A1 & EP 2841066 A1 & KR 10-2015-0013527 A & CN 104640540 A | 1-15 |
| A | JP 2006-111867 A (SEIKAGAKU CORPORATION) 27 April 2006, entire text & US 2006/0057098 A1 & WO 2006/030965 A1 & EP 1794192 A1 & EP 2287205 A2 & KR 10-2007-0060114 A & CN 101018812 A | 1-15 |
| A | WO 2014/080730 A1 (GLYTECH, INC.) 30 May 2014, entire text & US 2015/0299337 A1 & EP 2924053 A1 & CN 104918962 A & KR 10-2015-0102003 A | 1-15 |

Form PCT/ISA/210 (continuation of second sheet) (January 2015)

## INTERNATIONAL SEARCH REPORT

International application No.
PCT/JP2019/011715

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-15

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

INTERNATIONAL SEARCH REPORT

International application No.
PCT/JP2019/011715

<Continuation of Box No. III>

Claim 1 is considered to be an invention pertaining to a compound specified by the matter of a "water-soluble alginic acid derivative having a structure in which alginic acid or a salt thereof and a nonsteroidal anti-inflammatory compound are covalently bonded via a linker."

Meanwhile, claim 16 is considered to be an invention pertaining to a compound specified by a structural formula represented by formula (AM).

Comparing the invention in claim 1 with the invention in claim 16, said "alginic acid" is not a technical feature shared between claim 1 and claim 16 in that "alginic acid" is not specified as an essential structure in the invention in claim 16.

In addition, the invention in claim 1 mentions only the wording "linker," whereas the invention in claim 16 specifies the structural portion corresponding to the "linker" of the invention in claim 1 by a specific chemical structural formula. In this regard, the "linker" portion is also not a technical feature in common between claim 1 and claim 16.

Thus, the technical feature in common between the invention in claim 1 and the invention in claim 16 is considered to be only the structural portion specified by the matter of a "nonsteroidal anti-inflammatory compound."

Document 1 (paragraph [0072]) lists a group of a plurality of materials corresponding to the "nonsteroidal anti-inflammatory compound" that is the technical feature shared by the invention in claim 1 and the invention in claim 16.

Document 2 (paragraph [0033]) lists a group of a plurality of materials corresponding to the aforementioned "nonsteroidal anti-inflammatory compound." In addition, the compounds prepared in examples 2-7 are understood to be compounds corresponding to the invention in claim 16.

Document 3 (paragraph [0056]) lists a group of a plurality of materials corresponding to the aforementioned "nonsteroidal anti-inflammatory compound." In addition, the compounds prepared in examples 1, 4, 6, 8, 10, 12, 14, 16, 18, 20, 24, 27, 29, 31, 32, 34, and 38 are understood to be compounds corresponding to the invention in claim 16.

The compounds mentioned in FIGS. 19 and 20 of document 4 are understood to be compounds corresponding to the invention in claim 16.

As disclosed in documents 1-3, the "nonsteroidal anti-inflammatory compound" that is the technical feature in common between claim 1 and claim 16 is a well-known substance to a person skilled in the art, and is not a special technical feature. In addition, the invention in claim 16 lacks novelty as disclosed in document 2, document 3, or document 4, and is not an invention pertaining to a group of compounds sharing a special technical feature.

Thus, this application is considered to have at least two inventions, i.e., a group of inventions consisting of the invention in claim 1 and the invention in claims 2-15 dependent on claim 1, and the invention in claim 16 that does not share a special technical feature with said group of inventions.

Claim 1 is an invention of a substance pertaining to a "water-soluble alginic acid derivative," and is specified as "having a structure in which alginic acid or a salt thereof and a nonsteroidal anti-inflammatory compound are covalently bonded via a linker."

However, the "nonsteroidal anti-inflammatory compound" only specifies the structure of a substance according to the present invention in terms of nature, but the structure of the substance specified by the matter cannot be clearly understood.

Form PCT/ISA/210 (extra sheet) (January 2015)

**INTERNATIONAL SEARCH REPORT**

International application No.
PCT/JP2019/011715

In addition, there is no disclosure in which the concrete structure of the "linker" is specified, and the positions at which and the manner in which the "water-soluble alginic acid derivative" and the "linker," and the "linker" and the "non-steroidal anti-inflammatory compound" are linked, are also not specified.

Due to the reasons explained above, the whole structure of the "water-soluble alginic acid derivative" of this invention cannot be understood, and the inventions in claims 1-14 do not comply with the requirements for clarity under PCT Article 6.

Considering the disclosure of claim 2, the "water-soluble alginic acid derivative" according to the present invention is considered to include an aspect in which the "nonsteroidal anti-inflammatory compound" and the "linker" are linked by an ester bond.

Meanwhile, the substance referred to as the "nonsteroidal anti-inflammatory compound" includes a compound (e.g., nimesulide, antipyrine) which does not have a hydroxyl group or carboxyl group in the molecular structure thereof.

In addition, even among the group of substances listed in this description (paragraph [0084]) as the "nonsteroidal anti-inflammatory compound," there exist substances (indometacin farnesil and nabumetone) that do not have a hydroxyl group or carboxyl group in the molecule thereof and that do not seem to be physically able to form an ester bond with other substances, or a substance (proglumetacin maleate) that has a carboxylic group not existing in the drug molecule itself but existing only in addition salts thereof and thus does not seem to be physically able to form an ester bond with the drug molecule.

Upon considering the molecular structures of the compounds belonging to the aforementioned "nonsteroidal anti-inflammatory compound," although considering the disclosure of this description, it is impossible to understand the "water-soluble alginic acid derivative" according to the present invention to be able to be created with any compound belonging to the "non-steroidal anti-inflammatory compound," and there is no disclosure of an example of synthesis of a supporting compound.

Thus, the present description is not disclosed clearly or sufficiently enough for a person skilled in the art to carry out the invention in claims 1-8 and 10-14 including any "nonsteroidal inflammatory compound" as a subject matter, and the claims 1-8 and 10-14 do not comply with the requirements of PCT Article 5.

In addition, the invention in claims 1-8 and 10-14 including any "nonsteroidal inflammatory compound" as a subject matter is beyond the scope disclosed in the description, and the claims 1-8 and 10-14 do not comply with the requirements of PCT Article 6.

Due to the reasons explained above, the international search for this invention was carried out by limiting the "nonsteroidal anti-inflammatory compound" set forth in claim 1 to the compounds below the concrete names of which are listed in paragraph [0084] of this description and which have a carboxylic group or hydroxyl group in the molecules of said substances.

"Salicylic acid, sasapyrine, aspirin, diflunisal, salicylamide, ibuprofen, flurbiprofen, ketoprofen, naproxen, pranoprofen, fenoprofen, tiaprofenic acid, oxaprozin, loxoprofen sodium, alminoprofen, zaltoprofen, tiaprofenic acid, felbinac, diclofenac, tolmetin sodium, sulindac, fenbufen, indometacin, acemetacin, amfenac sodium, mofezolac, etodolac, and alclofenac"

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007075425 A
- WO 2008102855 A
- WO 200954181 A
- WO 2017163603 A
- WO 200566214 A
- WO 20155458 A
- WO 20074675 A
- JP HEI0824325 B
- JP HEI08502053 PCT


### Non-patent literature cited in the description


- *BMC Musculoskelet Disord.,* 2018, vol. 19, 157
- *Journal of Young Pharmacists,* 2009, vol. 1 (4), 301-304
- *Pharmaceutica Acta Helvetiae,* 1997, vol. 72 (3), 159-164
- **CHIKAKO YOMOTA.** *Bull. Natl. Health Sci.,* 1999, vol. 117, 135-139
- **CHIKAKO YOMOTA.** *Bull. Natl. Inst. Health Sci.,* 2003, vol. 121, 30-33
- Size Exclusion Chromatography with Multiple Angle Laser Light Scattering Detection. ASTM F2064-00. ASTM International, 2006
- *CHEMICAL ABSTRACTS,* 1644429-26-4
- *CHEMICAL ABSTRACTS,* 1644429-25-3
- *CHEMICAL ABSTRACTS,* 1644429-24-2
- *CHEMICAL ABSTRACTS,* 1644429-23-1
- *CHEMICAL ABSTRACTS,* 1644429-21-9
- *CHEMICAL ABSTRACTS,* 1384127-03-0
- *CHEMICAL ABSTRACTS,* 918636-69-8
- *CHEMICAL ABSTRACTS,* 918636-66-5
- *CHEMICAL ABSTRACTS,* 918636-63-2
- *CHEMICAL ABSTRACTS,* 918636-60-9
- *CHEMICAL ABSTRACTS,* 918636-57-4
- **STAHL ; WERMUTH.** Handbook of Pharmaceutical Salts: Properties, Selection, and Use. Wiley-VCH, 2002
- *BMC Musculoskeletal Disorders,* 2018, vol. 19, 157